# NEW EUROPEAN PATENT SPECIFICATION

(11) **EP 0 272 703 B2**
(45) Date of publication and mention of the opposition decision: **15.10.1997**
(45) Mention of the grant of the patent: 28.07.1993
(21) Application number: 87119157.3
(22) Date of filing: 23.12.1987
(51) Int. Cl.: C12N 15/70, C07K 14/475, C12P 21/00

(54) **Novel polypeptide**
Neues Polypeptid
Nouveau polypeptide

(30) Priority: 23.12.1986 JP 306799/86
(43) Date of publication of application: 29.06.1988
(73) Proprietor: KYOWA HAKKO KOGYO CO., LTD., Chiyoda-ku, Tokyo-to (JP)
(72) Inventor: Kuga, Tetsuro, Hofu-shi Yamaguchi (JP); Komatsu, Yoshinori, Machida-shi Tokyo (JP); Miyaji, Horomasa, Machida-shi Tokyo (JP); Sato, Moriyuki, Machida-shi Tokyo (JP); Okabe, Masami, Suntogun Shizuoka (JP); Morimoto, Makoto, Sunto-gun Shizuoka (JP); Itoh, Seiga, Sagamihara-shi Kanagawa (JP); Yamasaki, Motoo, Machida-shi Tokyo (JP); Yokoo, Yoshiharu, Sagamihara-shi Kanagawa (JP); Yamaguchi, Kazuo, Sagamihara-shi Kanagawa (JP)
(74) Representative: Kinzebach, Werner, Dr.

(56) References cited:
- EP-A- 0 169 566
- EP-A- 0 220 520
- WO-A-86/04605
- WO-A-87/01132
- JP-A-61 306 799
- NATURE, vol. 319, 30 January 1986, New York-London; S.NAGATA et al., pp. 415-418
- SCIENCE, vol. 232, no. 4746, 04 April 1986, Washington, DC (US); L.M.SOUZA et al., pp. 61-65
- Proc. Natl. Acad. Sci. USA, vol. 83, pages 7633-7637; Tsuchiya et al
- Biochem. Biophys. Res. Comm., vol. 159, 1989, pages 103-111; Kuga et al
- Blood, vol. 75, 1990, pages 1788-1793; Okabe et al
- Blood, vol. 79, 1992, pages 536-537; Tanaka and Kaneko
- Blood, vol. 79, 1992, pages 537-539; Kuwabara et al
- The Clinical Report, vol. 13, 1993, pages 61-76; Tanaka et al
- Experimental Hematology, 24th Annual Meeting of the Intern. Society for Experimental Hematology, August 27-31, 1995, Düsseldorf, Germany, Abstract 499; Shibuya et al
- Jpn. J. Cancer Res. (Gann), vol. 78, November 1987, pages 1179-1181; Komatsu et al
- Bioscience, Biotechnology, and Biochemistry, vol. 60, Jan. 1996, Published by Japan Society for Bioscience, Biotechnology and Agrochemistry, pages 1-8; Ito et al
- Science, vol. 229, July 1985, pages 16-22; D. Metcalf

## Description

### FIELD OF THE INVENTION

The present invention relates to novel human granulocyte colony stimulating factor (hG-CSF) polypeptide derivatives, recombinant plasmids with a DNA coding for any of said polypeptide derivatives being inserted therein, microorganisms each carrying any of said plasmids, and a method of producing said novel hG-CSF polypeptide derivatives.

### BACKGROUND OF THE INVENTION

The human granulocyte colony stimulating factor (hG-CSF) is a kind of polypeptide which is essential in the formation of various blood cells as a result of proliferation and differentiation of hematopoietic stem cells. Its major effect is to promote the increase in number of granulocytes, in particular neutrophils. Neutrophils play an important part in the protection of the living body from infection.

However, their life spans are short and, therefore, constant supplementation is required by proliferation and differentiation of precursor cells. The therapies widely employed in recent years for proliferative tumors simultaneously inhibit the growth of neutrophil precursors, hence cause a severe side effect, namely a reduction in neutrophilic protection in cancer-bearing patients making them more susceptible to infection. hG-CSF is expected to be effective in alleviating this undesirable side effect through promotion of the increase in the number of neutrophils on one band and, on the other, in preventing and treating infectious diseases. Furthermore, hG-CSF is active in causing differentiation of leukemic cell lines in vitro and therefore may possibly be useful as a therapeutic agent for leukemia. The hG-CSF polypeptide derivatives according to the invention are superior in hG-CSF activity to the known hG-CSF and are expected to be useful as drugs.

With the recent rapid progress in recombinant DNA technology, genes for proteins involved in the proliferation and differentiation of blood cells have been isolated in succession. Such factors are in production by genetic engineering techniques using microorganisms or animal cells.

A cDNA for hG-CSF was isolated from the human squamous cell carcinoma cell line CHU-II, its base sequence was determined and its expression in COS cells was reported by Nagata et al. [Nagata et al.: Nature, 319, 415 (1986)]. Souza et al. also isolated a cDNA from the human bladder cancer cell line 5637, determined its base sequence and reported its expression in Escherichia coli (E. coli) [Souza et al.: Science, 232, 61 (1986)].

The amino acid sequence of the protein encoded by the above two cDNAs is in agreement with the amino acid sequence (Table 1) of the protein encoded by the cDNA isolated from normal human peripheral blood macrophages by the present inventors.

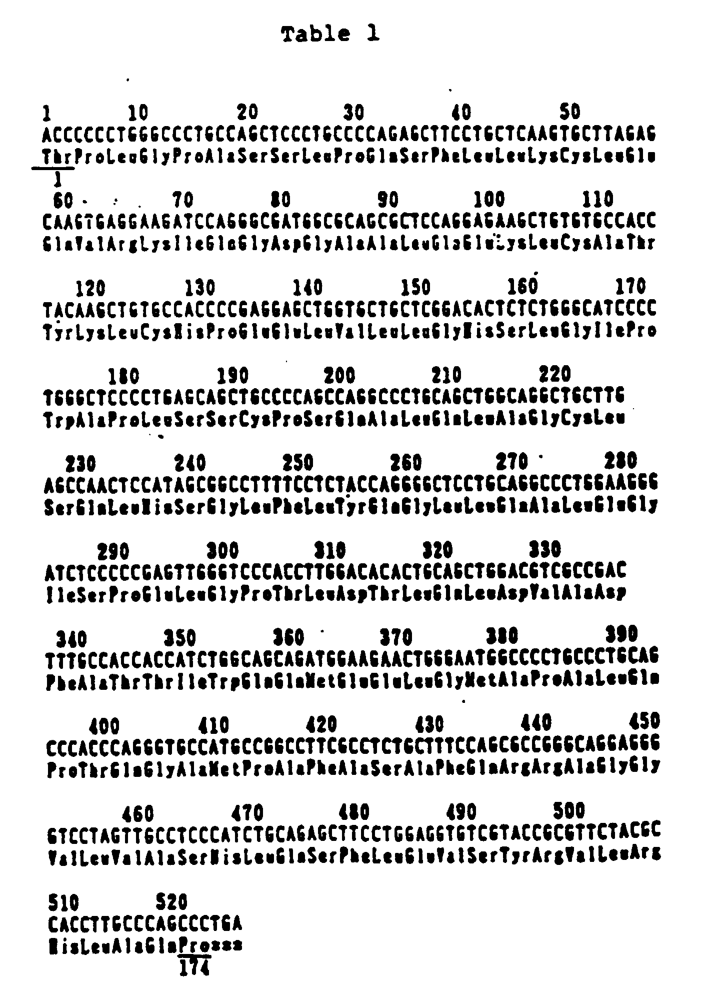

WO 87/01132 which is a document according to Article 54(3) EPC discloses novel polypeptides possessing part or all of the primary structural conformation and one or more of the biological properties of hpG-CSF. For example the substitution analogs [Ala¹]hpG-CSF and [Ser¹⁷]hpG-CSF are disclosed.

EP-A-0 256 843 which is also a document according to Article 54(3) EPC discloses recombinant analogs of hG-CSF. For example substitution analogs are mentioned wherein [Thr¹] of hG-CSF is replaced by Ala, Gly or Pro. Moreover the deletion analog ∇₄-G-CSF, missing the four N-terminal amino acids of hG-CSF is also disclosed.

### SUMMARY OF THE INVENTION

It is an object of the invention to provide a means of producing, at low cost and in large quantities, hG-CSF polypeptide derivatives having high specific activity and high stability in blood.

The present inventors found that hG-CSF polypeptide derivatives having high specific activity can be produced by modifying the cDNA for hG-CSF shown in Table 1 and cultivating a strain of E. coli that harbors a plasmid with the modified cDNA inserted therein or by limited polypeptide decomposition using a protease, and they have now completed the present invention.

### BRIEF DESCRIPTION OF THE DRAWINGS

Fig. 1 shows a construction scheme for the plasmid pCfTA1.

Fig. 2 shows a construction scheme for the plasmid pCfTB20.

Fig. 3 shows construction schemes for the plasmids pCfTL23, 38, 35 and 41.

Fig. 4 shows construction schemes for the plasmids pCfTM14, 17 and 113.

Fig. 5 shows a construction scheme for the plasmid pCfWD1.

Fig. 6 shows construction schemes for the plasmids pCfT95K19, pCfAA1 and pCfAB5.

Fig. 7 shows construction schemes for the plasmids pCfBA8, pCfBB101, pCfBC52, 59, 42B1, 45, 76, 77, 93, 95, 97, pCfBD28, 56 and 82.

Fig. 8 shows construction schemes for the plasmids pCfCB101, pCfCC52, 59, pCfCD28 and 56.

Fig. 9 (1) and (2) schematically show the processes involved in the Okayama-Berg method for cDNA synthesis and construction of a recombinant plasmid containing the DNA synthesized.

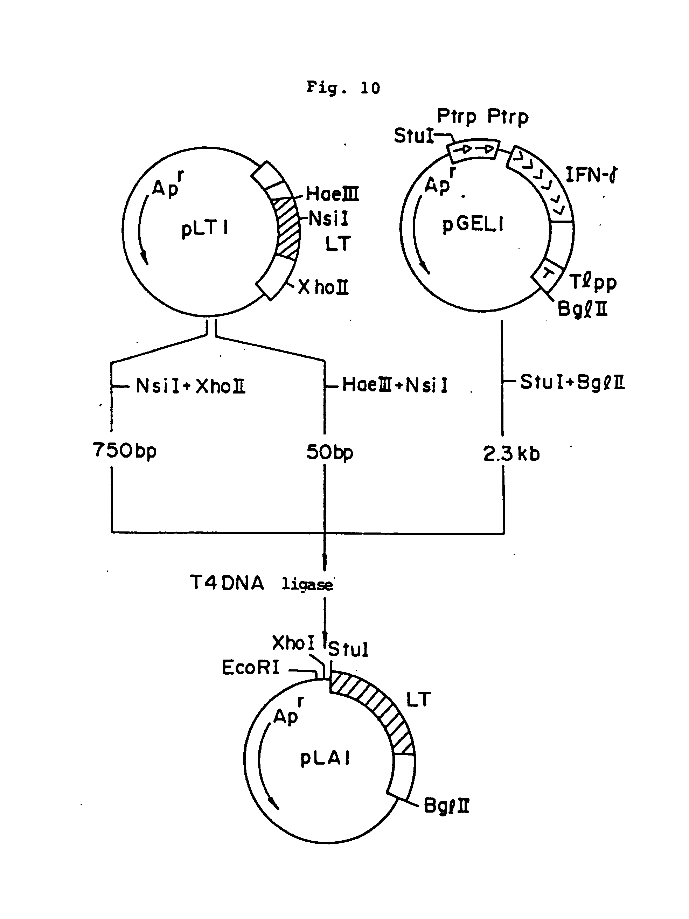

Fig. 10 shows a construction scheme for the plasmid pLA1.

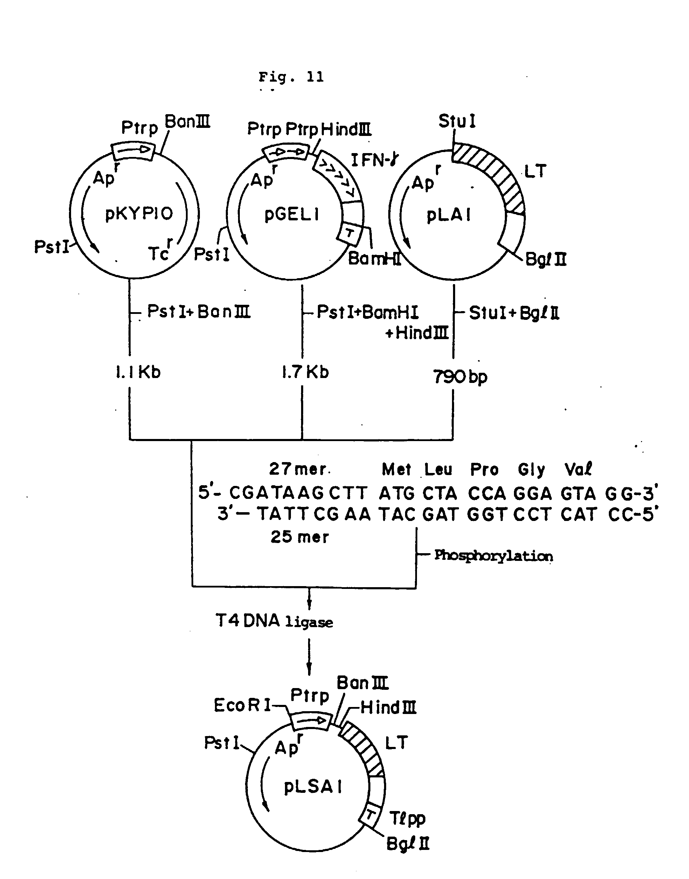

Fig. 11 shows a construction scheme for the plasmid pLSA1.

Fig. 12 shows a construction scheme for the plasmid pCfTNS501.

Fig. 13 shows a construction scheme for the plasmid pTrS20.

Fig. 14 shows construction schemes for the plasmids pCfTNS7 and pCfTAArg4S.

Fig. 15 shows construction schemes for the plasmids pCfTN205 and pCfTAArg4.

Fig, 16 shows construction schemes for the plasmids pCfTNS301 and pCfTNS401.

Fig. 17 (1) and (2) show construction schemes for the plasmids pCfBD28A17 and pCfBD28T17.

### DETAILED DESCRIPTION OF THE INVENTION

The hG-CSF polypeptide derivatives according to the invention differ in part of the amino acid sequence from the hG-CSF polypeptide having the amino acid sequence shown in Table 1 as a result of substitution and/or deletion. The amino acid or amino acids to be substituted are those amino acids that are located at or in the neighborhood of the N terminus. Similarly, the amino acids to be deleted are those amino acids at or in the neighborhood of the N terminus.

The recombinant plasmids according to the invention are obtained by inserting a DNA fragment coding for any of the above-mentioned hG-CSF polypeptide derivatives into an appropriate plasmid having a DNA expression function.

Preferred as the DNA fragments coding for the above-mentioned hG-CSF polypeptide derivatives of the invention are those resulting from substitution of at least one base selected from among the 1st to 51st bases of the base sequence shown in Table 1 of the DNA coding for hG-CSF.

In more detail the present invention concerns: novel polypeptides having an amino acid sequence as derived from the amino acid sequence of the human granulocyte colony stimulating factor (hG-CSF) polypeptide by amino acid substitution, which are characterized in that at least one amino acid out of the 1st to 6th amino acids on the N terminus side and optionally the 17th amino acid thereof differs from the amino acid in the corresponding position of the hG-CSF polypeptide, with the proviso that more than one amino acid of hG-CSF is substituted if the amino acid Thr⁺¹ of hG-CSF is replaced by Ala, Gly or Pro; and with the further proviso that the amino acids in position +1 and +2 are not simultaneously substituted as well as a DNA coding for those polypeptides and recombinant plasmids comprising a plasmid DNA and, as an insert therein, a DNA fragment coding for a polypeptide as defined above.

Preferably those plasmids are concerned wherein the plasmid DNA is a tryptophan promoter-containing plasmid DNA, with said DNA fragment inserted in said plasmid DNA at a site downstream from the tryptophan promoter.

Preferably the plasmids are selected from the group consisting of pCfTL38, pCfTL41, pCfTL35 and pCfTAArg4 comprising an insert which codes for a hG-CSF polypeptide being modified as follows:

| Plasmid | Position of amino acid substitution (substituted amino acid of hG-CSF) | |
|---|---|---|
| | 1st (Thr) | 4th (Gly) |
| pCfTL38 | Ser | *) |
| pCfTL41 | Arg | |
| pCfTL35 | Cys | |
| pCfTAArg4 | | Arg |

| | | |
|---|---|---|
| *) No substitution | | |

or selected from the group consisting of pCfTM14, pCfTM17, pCfTM113 and pCfTAArg4S comprising an insert which codes for a hG-CSF polypeptide being modified as follows:

| Plasmid | Position of amino acid substitution (substituted amino acid of hG-CSF) | | | |
|---|---|---|---|---|
| | 1st (Thr) | 3rd(Leu) | 4th (Gly) | 17th (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |

| | | | | |
|---|---|---|---|---|
| *) No substitution | | | | |

or selected from the group consisting of pCfBC42B1, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 and pCfBD28T17 comprising an insert which codes for a hG-CSF polypeptide being modified as follows:

| Plasmid | Position of amino acid substitution (amino acid of hG-CSF) | | | | | |
|---|---|---|---|---|---|---|
| | 1st (Thr) | 3rd (Leu) | 4th (Gly) | 5th (Pro) | 17th (Cys) | 6th (Ala) |
| pCfBC42B1 | -* | Glu | Lys | Ser | Ser | -* |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -* | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |

| | | | | | | |
|---|---|---|---|---|---|---|
| -* No substitution | | | | | | |

Another embodiment of the present invention relates to a microorganism harboring at least one of the above-mentioned plasmids. Preferably said microorganism belongs to the species Escherichia coli.

Another embodiment of the present invention relates to a method of producing the above-mentioned substitution analogs, which method comprises (a) cultivating in a medium a microorganism harboring a recombinant plasmid coding for said polypeptide to thereby cause formation and accumulation of said polypeptide in the culture, and (b) recovering said polypeptide from said culture.

Preferably one of the above-mentioned recombinant plasmids is applied which is harbored in a microorganism belonging to the species Escherichia coli.

Another embodiment of the present invention relates to polypeptides derived from the hG-CSF polypeptide by deletion, wherein a peptide comprising the 1st to 4th, 1st to 5th, 1st to 6th, 1st to 7th or 1st to 11th amino acids on the N terminus portion of the hG-CSF polypeptide is missing, and optionally in addition to amino acid deletion, the 17th amino acid cysteine (Cys⁺¹⁷) of the hG-CSF polypeptide has been replaced by serine (Ser), with the proviso that the 1st to 4th amino acids of hG-CSF are only then deleted if the Cys⁺¹⁷ of hG-CSF is replaced by Ser, as well as to DNA molecules coding for these polypeptides and to recombinant plasmids which comprise a plasmid DNA and, as an insert therein, a DNA fragment coding for a polypeptide as defined above.

Preferably the recombinant plasmid comprises a plasmid DNA and, as an insert therein, a DNA fragment coding for a polypeptide as derived from the hG-CSF polypeptide by deletion of a peptide comprising the 1st to 11th amino acids on the N terminus portion thereof. Preferred are plasmids wherein a tryptophan promoter-containing plasmid DNA is used as said plasmid DNA, with said DNA fragment inserted in the plasmid DNA at a site downstream from the tryptophan promoter.

More preferred are plasmids selected from the group consisting of pCfTNS7, pCfTNS301, pCfTNS401 and pCfTNS501 comprising an insert which codes for a hG-CSF polypeptide being modified as follows:

| Plasmid | type of sequence modification | |
|---|---|---|
| | Deletion¹⁾ | Substitution 17th (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |

| | | |
|---|---|---|
| ¹⁾position of deleted amino acids of hG-CSF | | |
| ²⁾substituted amino acid hG-CSF | | |

Another embodiment of the present invention relates to a method of producing the above-mentioned deletion analogs of hG-CSF, which method comprises cultivating in a medium a microorganism harboring a recombinant plasmid comprising a plasmid DNA and, as an insert therein, a DNA fragment coding for said polypeptide to thereby cause formation and accumulation of said polypeptide in the culture, and recovering said polypeptide from said culture. This method is applied for the production of a novel polypeptide having an amino acid sequence as derived from the amino acid sequence of the hG-CSF polypeptide by substitution of the 17th amino acid (cysteine) by serine and deletion of 4-7 amino acids on the N terminus side thereof, which method comprises subjecting a polypeptide as derived from the hG-CSF polypeptide by substitution of the 17th amino acid (cysteine) by serine and of at least one amino acid out of the 1st to 6th amino acids on the N terminus side thereof by an amino acid different therefrom to the action of a protease in an aqueous medium to thereby cause formation of said novel polypeptide in the reaction mixture, and recovering said novel polypeptide from said reaction mixture.

Preferably the protease is selected from the group consisting of subtilisin A, subtilisin BPN', subtilisin Carlsberg, subtilisin novo, proteinase K, nagase, thermolysin, endoproteinase Arg-C, trypsin and α-chymotrypsin.

A cDNA (hG-CSF cDNA) obtained by reverse transcription of an hG-CSF-encoding messenger RNA by recombinant DNA technology or an hG-CSF-encoding DNA obtained from chromosomal DNA, for instance, can be used as the hG-CSF-encoding DNA shown in Table 1.

Any hG-CSF cDNA may be used provided that it codes for hG-CSF. As an specific example, pCSF1-2, a plasmid produced by the present inventors, can be used. The process for the production of pCSF1-2 is described in Reference Example 1.

The hG-CSF cDNA contained in pCSF1-2 has the base sequence shown in Table 1 as determined by the dideoxy sequencing method using M13 phage [J. Messing et al.: Gene, 19, 269 (1982)].

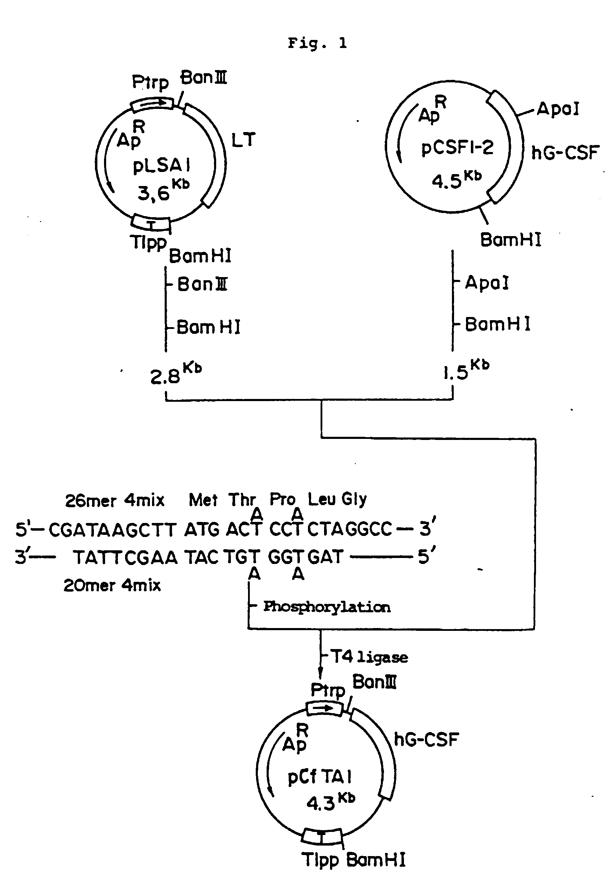

pCSF1-2 is a plasmid having the restriction enzyme map shown in Fig. 1 and on E. coli strain containing it, E. coli ECSF 1-2, has been deposited with the Fermentation Research Institute, Agency of Industrial Science and Technology (FRI) since November 27, 1986 under the deposit number FERM BP-1220 in accordance with the Budapest treaty.

Any plasmid may be used for the insertion of an hG-CSF polypeptide derivative-encoding DNA thereinto provided that said DNA con be expressed in E. coli.

A plasmid can be used with advantage which allows foreign DNA insertion thereinto at a site downstream from an appropriate promoter, for example, a trp or lac promoter, and has the distance between the Shine-Dalgarno sequence (hereinafter SD sequence) and the initiation codon (ATG) adjusted to an appropriate distance, for example, 6-18 base pairs.

As suitable examples of such plasmids there may be mentioned pKYP10 (US Patent 4,686,191) pLSA1 (Reference Example 3), pGEL1 [Sekine et al.: Proc. Natl. Acad. Sci. USA, 82, 4306 (1985)], pKYP26 [Japanese Patent Application (OPI) No 48 699/87 (the term "OPI" means an unexamined published application) and pBR322 (Bolivar et al.: Gene, 2, 95 (1977)].

Recombination between a DNA Coding for the hG-CSF polypeptide or a derivative thereof and a vector DNA can be effected by recombinant DNA techniques in general use which comprise digesting both DNA with a restriction enzyme or enzymes and the subsequent ligation using T4 DNA ligase. For ligation, the DNA fragment termini resulting from restriction enzyme digestion may be processed, when appropriate, by making use of the filling-in reaction using DNA polymerase I Klenow fragment or the trimming reaction using T4 DNA polymerase; the DNA linker technique is also applicable.

Examples of the construction of recombinant plasmids containing an hG-CSF polypeptide derivative-encoding DNA inserted therein by using pCSF1-2 as the hG-CSF cDNA, pGEL1, pKYP10, pKYP26, pBR322 or pLSA1 as the plasmid for incorporation of the DNA thereinto and, as necessary, a chemically synthesized DNA linker or a technique of site-directed mutagenesis are given in the following

### DETAILED DESCRIPTION OF THE DRAWINGS

### (Recombination Procedures)

As shown in Fig. 1, pCSF1-2 [about 4.5 kilobases (hereinafter kb)] is cleaved with ApaI and BamHI and a DNA fragment of about 1.5 kb is purified by low gelling temperature agarose gel electrophoresis (LGT method) [L. Wieslander: Analytical Biochemistry, 98, 305 (1979)].

Then, pLSA1 is cleaved with BanIII and BamHI, and a DNA fragment of about 2.8 kb is purified by the LGT method. Both the fragments thus obtained and the synthetic DNA shown in Fig. 1 are ligated together using T4 DNA ligase to give pCfTA1.

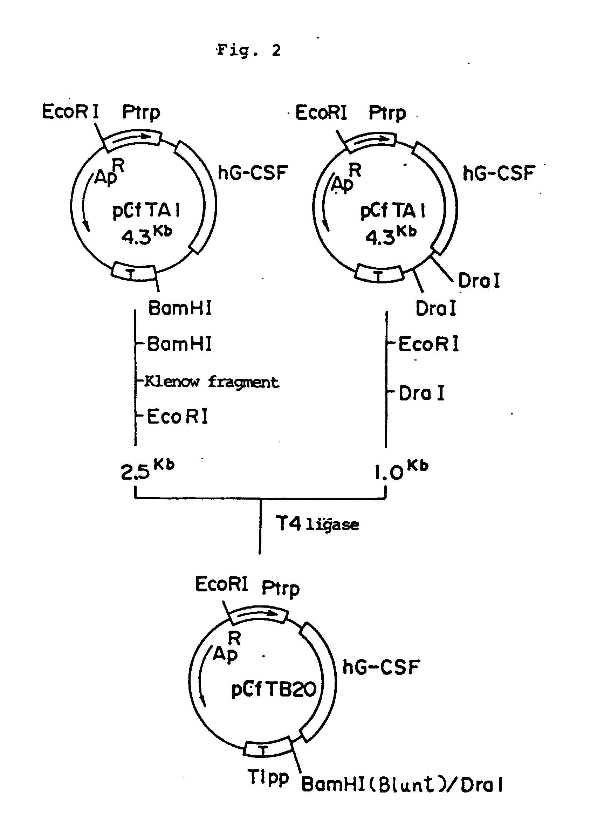

Then, as shown in Fig. 2, pCfTA1 is cleaved with BamHI, the protruding ends are converted to blunt ends by treatment with the Klenow fragment and, after further cleavage with EcoRI, a DNA fragment of about 2.5 kb is purified by the LGT method. Separately, pCfTA1 is cleaved with EcoRI and DraI and a DNA fragment of about 1.0 kb is purified by the LGT method. The DNA fragments thus obtained are ligated together using T4 DNA ligase to give pCfTB20.

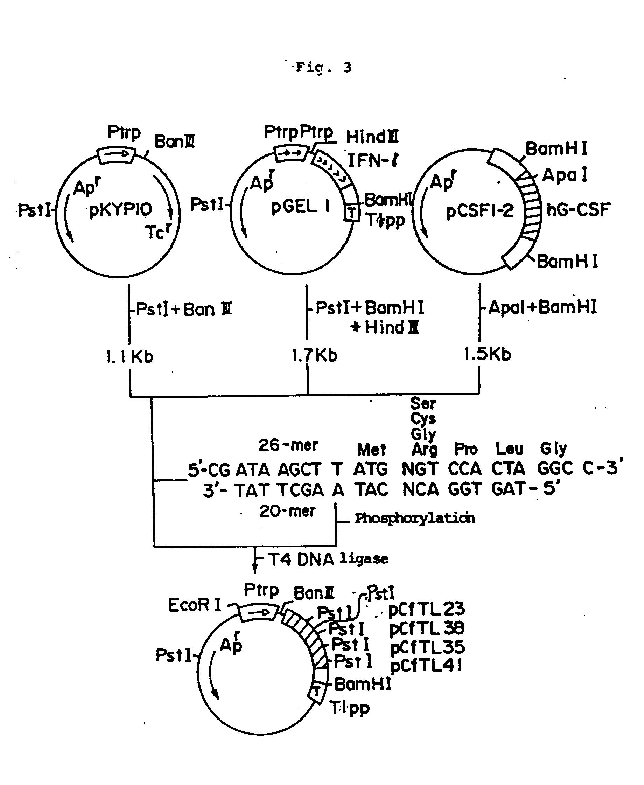

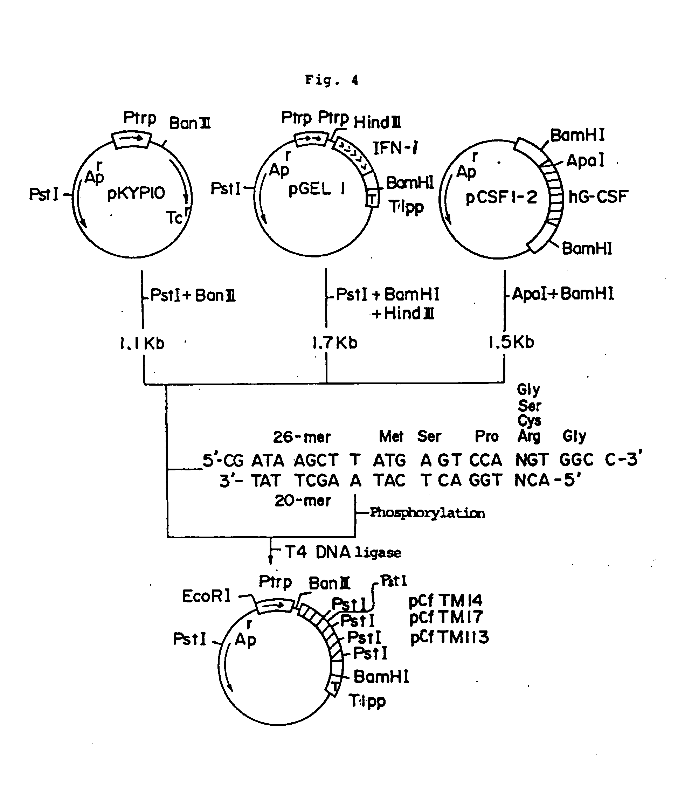

Further, as shown in Fig. 3, pCSF1-2 is cleaved with ApaI and BamHI and a DNA fragment of about 1.5 kb is purified by the LGT method. Separately, pGEL1 is cleaved with HindIII, BamHI and PstI and a DNA fragment of about 1.7 kb is purified by the LGT method. Furthermore, pKYP10 is cleaved with PstI and BanIII and a DNA fragment of about 1.1 kb is purified by the LGT method. Ligation of these three DNA fragments and the synthetic DNA shown in Fig. 3 gives pCfTL23, pCfTL38, pCfTL35 and pCfTL41 whereas ligation of these three DNA fragments and the synthetic DNA shown in Fig. 4 gives pCfTM14, pCfTM17 and pCfTM113.

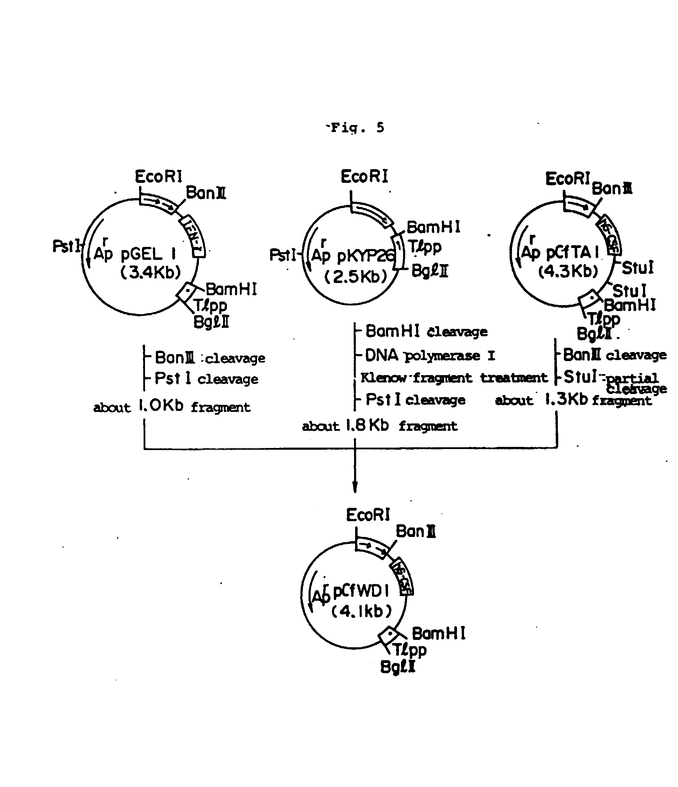

Furthermore, as shown in Fig. 5, pCfTA1 is cleaved with BanIII and StuI and an hG-CSF cDNA-containing DNA fragment of about 1.3 kb is purified by the LGT method, Separately, pKYP26 is cleaved with BamHI, the protruding ends are converted to blunt ends by treatment with DNA polymerase I Klenow fragment and, after further cleavage with PstI, a DNA of about 1.8 kb is purified by the LGT method. Further, separately, pGEL1 is cleaved with BanIII and PstI and a DNA fragment of about 1.0 kb is purified by the LGT method. The three DNA fragments thus obtained are ligated together using T4 DNA ligase to give pCfWD1.

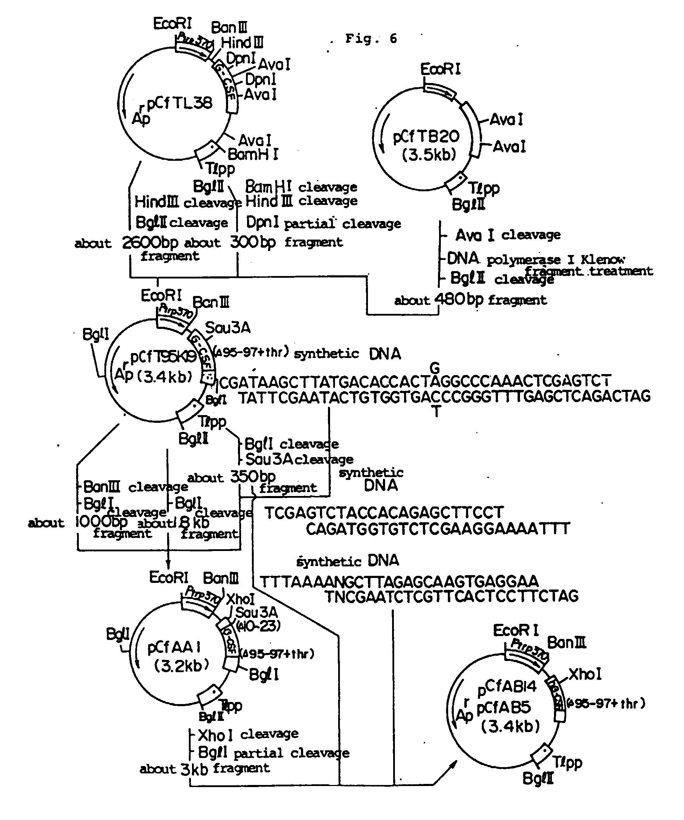

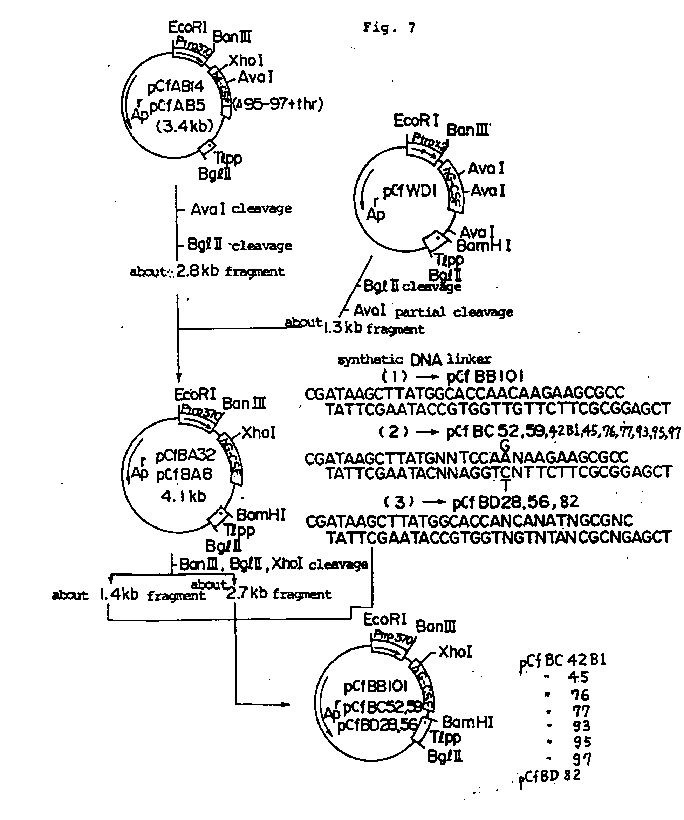

Further, as shown in Fig. 6, pCfTL38 is cleaved with HindIII and BgIII and a DNA fragment of about 2.6 kb is purified by the LGT method. Separately, pCfTL38 is cleaved with HindIII, BamHI and DpnI and a DNA fragment of about 300 bp (base pairs) is purified by the LGT method. Further, separately, pCfTB20 is cleaved with AvaI, the protruding ends are pared off by treatment with the Klenow fragment and, after further cleavage with BglII, a DNA fragment of about 480 bp is purified by the LGT method. The three DNA fragments thus obtained are ligated together using T4 DNA ligase to give pCfT95K19. Further, as also shown in Fig. 6, pCfT95K19 is cleaved with BanIII and BgII and a DNA of about 1.0 kb is purified by the LGT method and, separately, pCfT95K19 is cleaved with BglI alone and a DNA fragment of about 1.8 kb is purified by said method. Further, separately, pCfT95K19 is cleaved with BglI and Sau3A and a DNA fragment of about 350 bp is purified by the LGT method. The three DNA fragments thus obtained and the synthetic DNA shown in Fig. 6 in the middle thereof (i.e. halfway down) are ligated together to give pCfAA1. Then, as also shown in Fig. 6, pCfAA1 is cleaved with XhoI and BglI and a DNA fragment of about 3.0 kb is purified by the LGT method. This fragment, the above-mentioned BglI-Sau3A fragment (about 350 bp) of pCfT95K19 and the synthetic DNA shown in Fig. 6 at the bottom thereof are ligated together using T4 DNA ligase to give pCfAB5 and pCfAB14. Further, as shown in Fig. 7, pCfAB5 is cleaved with AvaI and BglII and a DNA fragment of about 2.8 kb is purified by the LGT method. Separately, pCfWD1 is cleaved with BglII and AvaI and the DNA fragment of about 1.3 kb is purified by the LGT method. The two fragments thus obtained are ligated together using T4 DNA ligase to give pCfBA8. On the other hand, pCfAB14 is cleaved with AvaI and BglII and a DNA fragment of about 2.8 kb is purified by the LGT method, and this fragment is ligated with the above-mentioned 1.3 kb DNA fragment derived from pCfWD1 by cleavage with BglII and AvaI using T4 DNA ligase, to give pCfBA32. Further, as also shown in Fig. 7, pCfBA8 is cleaved with BanIII, BglII and XhoI and a DNA fragment of about 1.4 kb and a DNA fragment of about 2.7 kb are purified by the LGT method. Ligation of the two DNA fragments thus obtained and the synthetic DNA linker shown in Fig. 7 using T4 DNA ligase gives pCfBB101, pCfBC52, pCfBC59, pCfBD28, pCfBD56, pCfBC42B1, pCfBC45, pCfBC76, pCfBC77, pCfBC93, pCfBC95, pCfBC97 and pCfBD82.

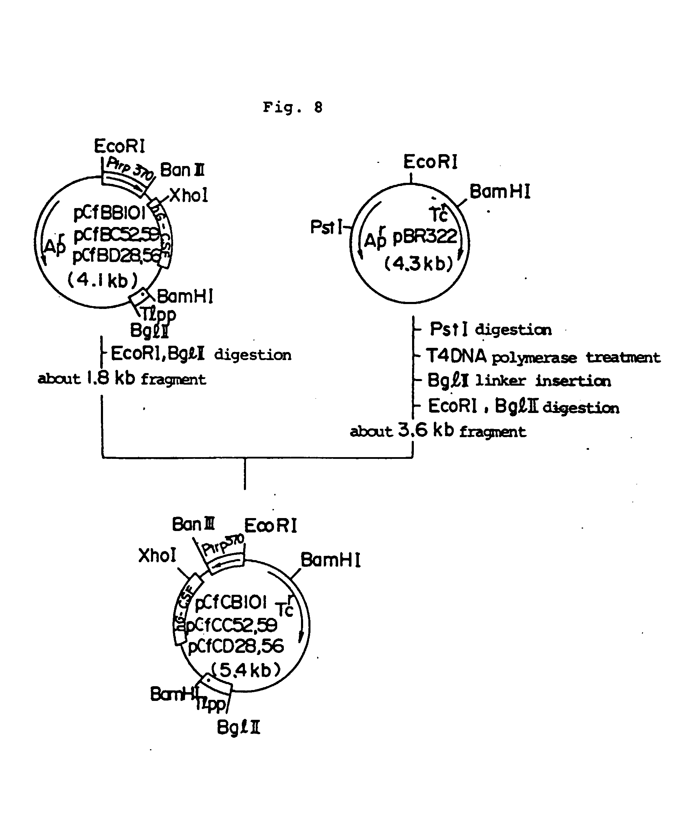

As shown in Fig. 8, pBR322 is cleaved with PstI, the protruding ends are pared off with T4 DNA polymerase, the BglII linker is inserted using T4 DNA ligase and, after further cleavage with EcoRI and BglII, a DNA fragment of about 3.6 kb is purified by the LGT method.

The plasmids pCfBB101, pCfBC52, pCfBC59, pCfBD28 and pCfBD56 obtained in the above manner are each cleaved with EcoRI and BglII and a DNA fragment of about 1.8 kb is purified by the LGT method. Each 1.8 kb DNA fragment is ligated with the pBR322-derived 3.6 kb DNA fragment using T4 DNA ligase. There are thus obtained pCfCB101, pCfCC52, pCfCC59, pCfCD28 and pCfCD56 corresponding to the respective plasmids mentioned above.

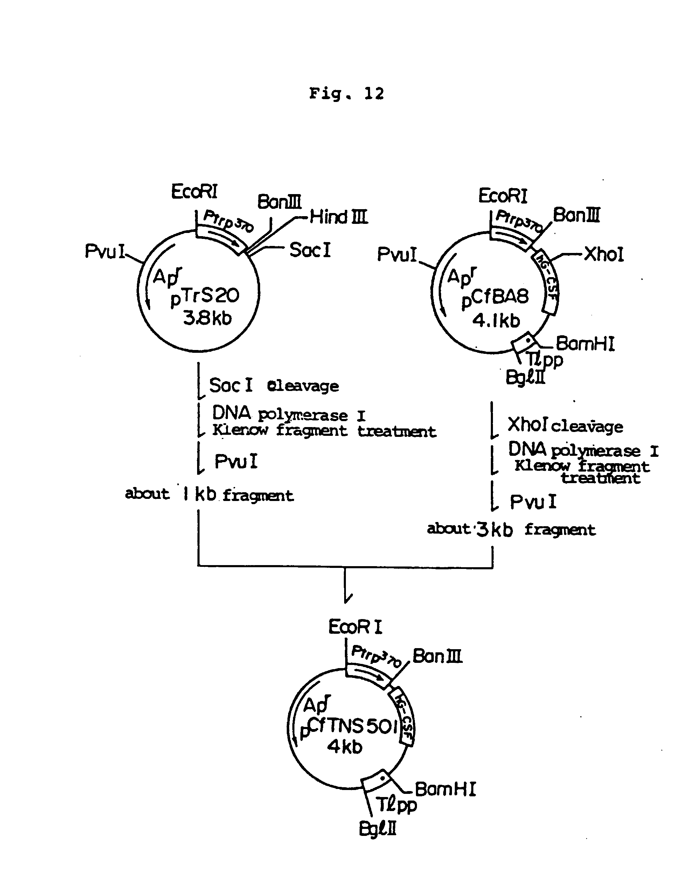

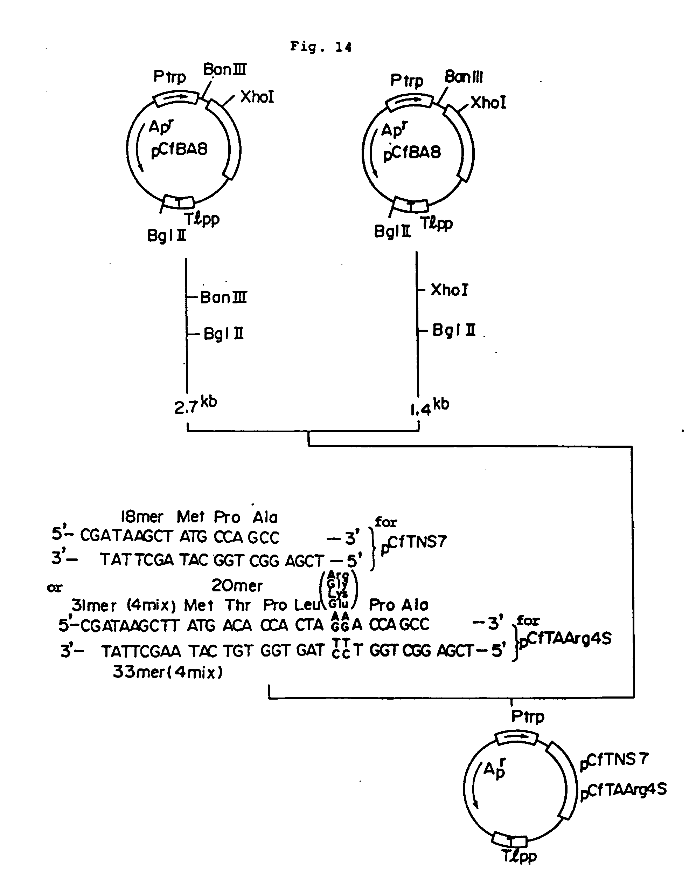

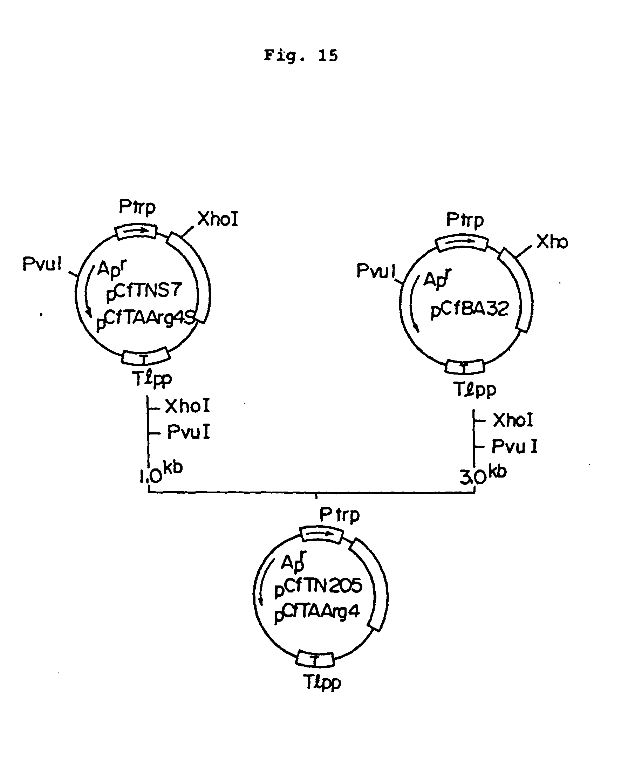

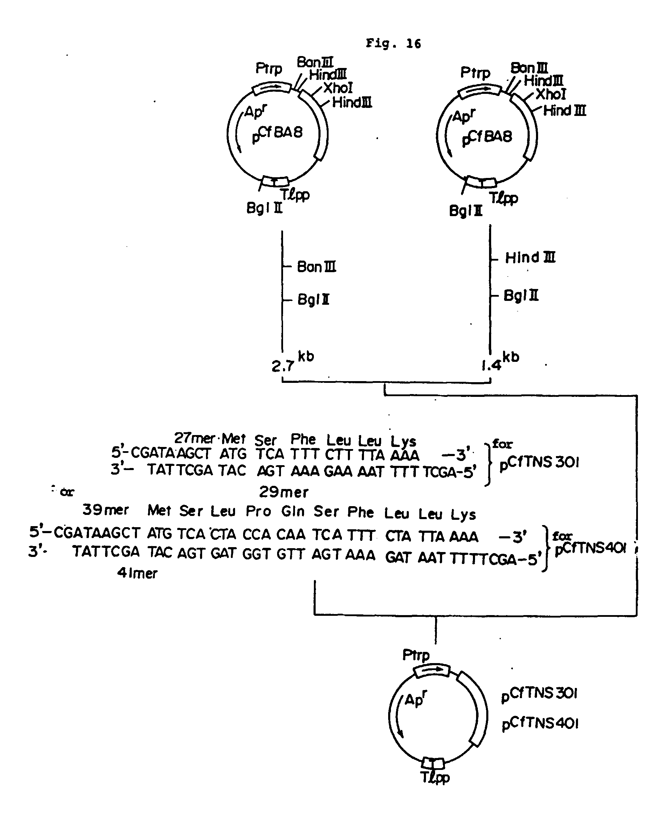

On the other hand, pCfBA8 is cleaved with BanIII and BglII and a DNA fragment of about 2.7 kb is purified by the LGT method. Separately, pCfBA8 is cleaved with XhoI and BglII and a DNA fragment of about 1.4 kb is purified by the LGT method. Ligation of the two fragments thus obtained and the synthetic DNA linker shown in Fig. 14 gives pCfTNS7 and pCfTAArg4S. In addition, as shown in Fig. 15, pCfTNS7 is cleaved with PvuI and XhoI and a DNA fragment of about 1 kb is purified by the LGT method. Separately, pCfBA32 is cleaved with PvuI and XhoI and a DNA fragment of about 3 kb is purified by the LGT method. The two fragments thus obtained are ligated together using T4 DNA ligase to give pCfTN205. Similarly, pCfTAArg4S is cleaved with PvuI and XhoI, a fragment of about 1 kb is purified by the LGT method and this fragment is ligated with a DNA fragment of about 3 kb derived from the above-mentioned plasmid pCfBA32 by cleavage with PvuI and XhoI, using T4 DNA ligase to give pCfTAArg4. Further, pCfBA8 is cleaved with BanIII and BglII and a DNA fragment of about 2.7 kb is purified by the LGT method. Separately, pCfBA8 is cleaved with HindIII and BglII and a DNA fragment of about 1.4 kb is purified by the LGT method. The two fragments thus-obtained and the synthetic linker shown in Fig. 16 are ligated together using T4 DNA ligase to give pCfTNS301 and pCfTNS401. Furthermore, as shown in Fig. 12, pCfBA8 is cleaved with XhoI, the protruding ends are converted to blunt ends by Klenow fragment treatment and, after further cleavage with PvuI, a DNA fragment of about 3 kb is purified by the LGT method. Separately, the ATG vector pTrS20 (reference Example 4) is cleaved with SacI, followed by conversion of the protruding ends to blunt ends by Klenow fragment treatment. After further cleavage with PvuI, a DNA fragment of about 1 kb is purified by the LGT method. The thus-obtained two fragments are ligated together using T4 DNA ligase to give pCfTNS501.

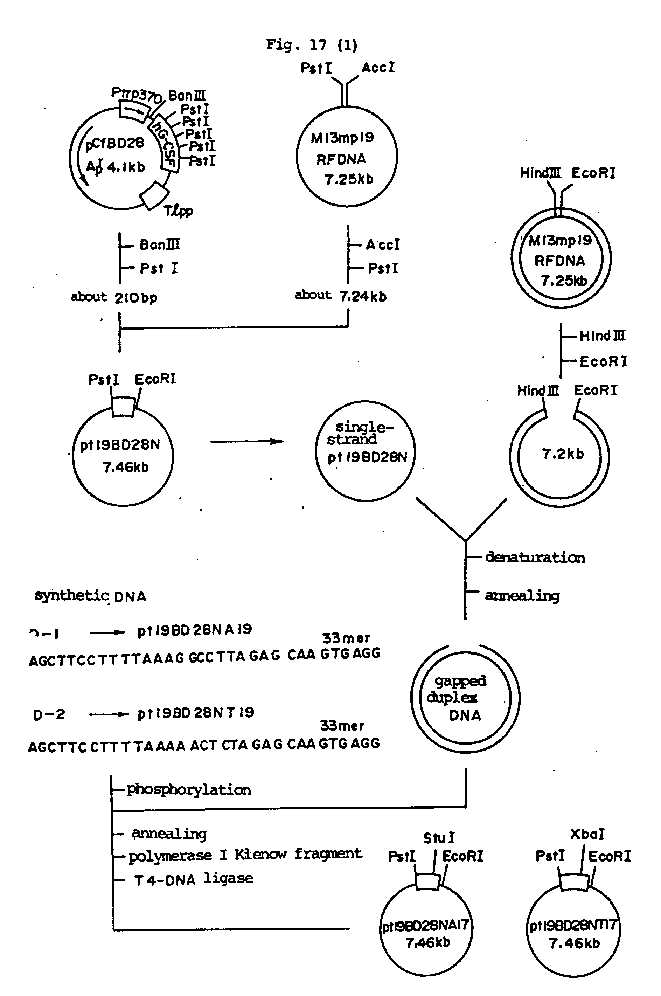

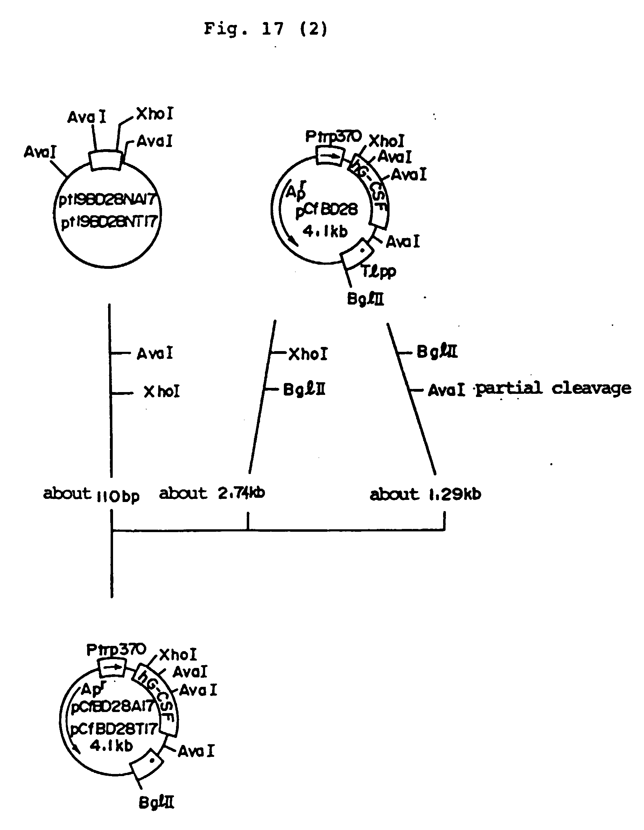

In an example where site-directed mutagenesis is utilized, pCfBD28 is cleaved with BanIII and PstI, as shown in Fig. 17, and a DNA fragment of about 210 bp is purified by the LGT method. Separately, the M13 phage vector M13mp19RF DNA is cleaved with AccI and PstI and a DNA fragment of about 7.24 kb is purified by the LGT method. The thus-obtained two DNA fragments are ligated together using T4 DNA ligase to give pt19BD28N. Then, this pt19BD28N is used to transfect E. coli JM105, and single-stranded pt19BD28N is recovered from the phage obtained. Similarly, as also shown in Fig. 17, the M13mp19RF DNA is cleaved with HindIII and EcoRI and a DNA fragment of about 7.2 kb is purified by the LGT method. After this 7.2 kb DNA fragment is mixed with the single-stranded pt19BD28N obtained in the above manner, gapped duplex DNA formation is caused by denaturation treatment followed by annealing and the resultant gapped duplex DNA is purified by the LGT method. Then, this DNA is annealed with the synthetic DNA shown in Fig. 17 and thereafter circularized using the Klenow fragment and T4 DNA ligase. This circularized DNA is used to transfect E. coli JM105, whereby pt19BD28NA17 and pt19BD28NT17 with site-directed mutagenesis introduced therein are obtained. Further, as also shown in Fig. 17, pt19BD28NA17 and pt19BD28NT17 are cleaved with AvaI and XhoI and each DNA fragment of about 110 bp is purified by the LGT method. Separately, pCfBD28 is cleaved with XhoI and BglII and a DNA fragment of about 2.74 kb is purified by the LGT method. Further, separately, pCfBD28 is cleaved with BglII and AvaI and a DNA fragment of about 1.29 kb is purified by the LGT method. Ligation of the thus-obtained DNA fragments of about 110 bp, about 2.74 kb and about 1.29 kb using T4 DNA ligase gives pCfBD28A17 and pCfBD28T17, respectivey.

The reaction conditions of the above recombinant DNA technology is generally as follows:

The DNA digestion reaction in the presence of a restriction enzyme or enzymes is generally carried out using 0.1-20 µg of DNA in a reaction mixture containing 2-200 mM (preferably 10-40 mM) Tris-HCl (pH 6.0-9.5, preferably 7.0-8.0), 0-200 mM NaCI and 2-20 mM (preferably 5-10 mM) MgCl₂ at 20-70 ° C (the optimal temperature varying depending on the restriction enzyme(s) used) for 15 minutes to 24 hours. The restriction enzymes are each used in an amount of 0.1-100 units (preferably 1-3 units) per microgram of DNA. Termination of the reaction is generally effected by heating at 55-75°C for 5-30 minutes. It is also possible to inactivate the restriction enzymes with a reagent such as phenol or diethyl pyrocarbonate.

The DNA fragments formed by the restriction enzyme digestion or the gapped duplex DNAs can be purified by the LGT method or by polyacrylamide gel electrophoresis [A. M. Maxam et al.: Proc. Natl. Acad. Sci. USA, 74, 560 (1977)], among others.

The DNA fragment ligation reaction is carried out in a reaction mixture containing 2-200 mM (preferably 10-40 mM) Tris-HCI (pH 6.1-9.5, preferably 7.8-8.0), 2-20 mM (preferably 5-10 mM) MgCl₂, 0.1-10 mM (preferably 0.5-2.0 mM) ATP and 1-50 mM (preferably 5-10 mM) dithiothreitol at 1-37°C (preferably 3-20°C) for 15 minutes to 72 hours (preferably 2-20 hours), using 0.3-10 units of T4 DNA ligase.

The recombinant plasmid DNA obtained by ligation reaction may be introduced into E. coli according to the transformation method of Cohen el al [S.N. Cohen et al: Proc. Natl. Acad. Sci. USA, 69, 2110 (1972)], if desired.

The recombinant M13 phage RF DNAs formed by the ligation reaction are introduced into E. coli JM105 [J. Messing et al.: Gene, 33, 103 (1985)], using the known method of transfection [Yoshiyuki Kuchino et al.: Tanpakushitsu, Kakusan, Koso (Protein, Nucleic Acid and Enzyme), 29, 294 (1984)], as necessary.

The recombinant plasmid DNAs and recombinant M13 phage RF DNAs can be isolated from the respective E. coli transformants by the method of Birnboim et al. [H.C. Birnboim et al.: Nucleic Acids Res., 7, 1513 (1979)], for example.

The isolation of the single-stranded DNA from the recombinant M13 phage is carried out by the known method [Yoshiyuki Kuchino et al.: Tanpakushitsu, Kakusan, Koso, 29, 294 (1984)].

The plasmid DNAs are examined for cleavage sites by agarose gel electrophoresis or polyacrylamide gel electrophoresis following cleavage with 1-10 restriction enzymes. Further DNA base sequence determination is performed, if necessary, by the dideoxy sequencing method using M13 phage [J. Messing et al.: Gene, 19, 269 (1982)].

The desired recombinant plasmid DNAs can be produced under the conditions such as mentioned above.

The hG-CSF polypeptide derivatives of the invention can be produced in the following manner.

Thus, E. coli K-12 HB101 is transformed with a suitable plasmid (e.g. pCfBD28), and a plasmid (e.g. pCfBD28)-carrying transformant of E. coli is selected from among ampicillin resistant (hereinafter, Ap^{r}) colonies. Growing the plasmid (e.g. pCfBD28)-bearing strain of E. coli in a medium can lend to formation of an hG-CSF polypeptide derivative in the culture.

Any medium, whether synthetic or natural, may be used provided that it is suited for the growth of E. coli and for the production of the hG-CSF polypeptide derivative.

Usable carbon sources include glucose, fructose, lactose, glycerol, mannitol and sorbitol, among others, and usable nitrogen sources are NH₄Cl, (NH₄)₂SO₄, casamino acids, yeast extract, polypeptone, meat extract, Bactotryptone, corn steep liquor, etc. K₂HPO₄, KH₂PO₄, NaCI, MgSO₄, vitamin B₁, MgCl₂ and so forth may be used as other nutrient sources. The cultivation is carried out with aeration and stirring at a pH of 5.5-8.5 and a temperature of 18-40°C. Cultivation for 5-90 hours leads to accumulation of an hG-CSF polypeptide derivative in cultured cells. The cells are then harvested from the culture and disrupted by ultra sonication. Centrifugation gives cell residues. The hG-CSF polypeptide derivative is extracted from the cell residues, purified, solubilized and renatured by the method of Marston et al. [F. A. O. Marston et al.: BIO/TECHNOLOGY, 2, 800 (1984)]. Mouse bone marrow cells are treated with said derivative, and the hG-CSF polypeptide derivative is assayed by the method using the number of colonies formed in soft agar as an index.

In the practice of the invention, the hG-CSF activity is determined in the following manner. Bone marrow cells are aseptically collected from the femur of male C3H/He mice of 8-12 weeks of age (Shizuoka Laboratory Animal Center) and suspended in α-Minimum Essential Medium (Flow Laboratories; hereinafter referred to as α-MEM) supplemented with 10% of fetal bovine serum (FBS). Nylon wool (0.3 g; Wako Pure Chemical Industries' Nylon Fiber 146-04231) packed in a column is impregnated with 1.5 ml of the above cell suspension (about 5 x 10⁷ cells), and the reaction is allowed to proceed in a 5% CO₂ incubator at 37°C for 90 minutes. Then, α-MEM warmed to 37°C in advance is passed through the column, and bone marrow cells unadsorbed on the nylon wool are collected as an effluent fraction. The cells are washed once with α-MEM and the cell concentration is adjusted to a predetermined one.

Thereafter, the myelopoietic stem cell colony-forming ability is determined by the method of Okabe at al. [T. Okabe, et al.: Cancer Research, 44, 4503-4506 (1986)]. Thus, 0.2 ml of the bone marrow cell suspension (2 x 10⁶ cells/ml) prepared in the above manner is admixed with a mixture of 0.2 ml of α-MEM, 0.4 ml of FBS and 0.2 ml of each 2-fold diluted sample. An equal volume (1.0 ml) of 0.6% agar (Difco, Agar purified 0506-01) solution maintained at 42°C is admixed with the above mixture and the resulting mixture is distributed in 0.5-ml portions onto a 24-well microtiter plate [Nunc's Multidish #143982) (5 x 10⁴ cells/well, n=3). After 7 days of incubation at 37°C in a 5% CO₂ incubator, colonies comprising not less than 40 cells are counted under a microscope (Olympus X40). After counting, each colony is transferred onto a slide glass with care, fixed there with an acetone-formalin mixed solution for 30 seconds and subjected to esterase double stain by the method of Kubota et al. [K. Kubota, et al.: Exp. Hematology, 8, 339-344 (1980)] for identification of the colony.

The potency of each sample is calculated based on the result of counting in the colony formation test for the 2-fold dilution as follows. The activity giving half of the maximum colony formation value obtained with intact G-CSF used as a standard is defined as 50 units. The potency (in units) is calculated according to this definition and using the factor 20 for multiplication for conversion to the activity per mililiter also in view of the dilution factor for the sample. The specific activity is expressed in terms of potency (units/mg) per unit weight (mg) of protein.

The hG-CSF polypeptide derivatives lacking one or more amino acids on the N-terminal side of the hG-CSF polypeptide can also be produced by enzymatic degradation.

The derivatives can, of course, be produced by enzymatic degradation of natural hG-CSF as the starting material. However, since natural hG-CSF is low in reactivity with the enzyme (protease), the use of a modified hG-CSF having increased reactivity against protease is preferable for producing such derivatives having high activity in good yields.

Preferably used as such starting materials are the modified hG-CSFs (a), (b), (c) and (d) shown in Table 2 as resulting from substitution of one or more amino acids on the N-terminal side of the hG-CSF polypeptide. Modifications (a), (b), (c) and (d) can be obtained by cultivating bacterial strains harboring the plasmids having the corresponding base sequences, namely pCfBC59 (NC59), pCfBD28 (ND28), pCfBC95 (NC95) and pCfTAArg4S (Arg 4S), respectively, followed by isolation and purification by known methods.

Suitably used as the enzymes are endoproteases such as serine protease and thiol protease. More specifically, there may be mentioned, for example, subtilisin A, subtilisin BPN', subtilisin Carlsberg, subtilisin novo, proteinase K, nagase, thermolysin, endoproteinase Arg-C, trypsin and α-chymotrypsin. The enzyme is used is an amount of 3.4 x 10⁻⁶ to 8.5 x 10⁻³ units per milligram of the starting material.

**Table 2**

| Examples of N-terminally protease-susceptible hG-CSF derivatives | | |
|---|---|---|
| Modified hG-CSF | Substituent amino acids | Plasmid* |
| a | Tyr¹, Ile³, Arg⁴, Ser⁵ ...... Ser¹⁷ | NC59 |
| b | Ala¹, Thr³, Tyr⁴, Arg⁵ ...... Ser¹⁷ | ND28 |
| c | Ile¹, Thr³, Arg⁴, Ser⁵ ...... Ser¹⁷ | NC95 |
| d | Arg⁴, ......Ser¹⁷ | Arg4S |

| | | |
|---|---|---|
| * Amino acids after substitution. The superscripts indicate the position numbers of the relevant amino acids from the N terminus. | | |

Following dissolution of the starting material in an aqueous solution such as Tris hydrochloride buffer or phosphate buffer and addition of an enzyme, the enzymatic reaction is carried out at 10-37°C for 30 minutes to 3 days.

The total protein quantity and the protein quantity are determined by the following methods:

The total protein determination is performed by the method of M. M. Bradford [M. M. Bradford: Anal. Biochem., 72, 248 (1976)].

The protein quantity is determined by SDS-polyacrylamide gel electrophoresis by the method of Laemmli [U. K. Laemmli: Nature, 227, 680 (1970)] followed by measurement on a chromatoscanner (Shimadzu CS-930).

The N-terminal amino acid sequence of the peptide obtained after enzymatic cleavage is determined using an automatic amino acid sequencer "Gas-Phase Protein Sequencer Model 470A" (Applied Biosystems) in combination with a Spectra Physics high-performance liquid chromatograph.

### DESCRIPTION OF THE PREFERRED EMBODIMENTS

The following examples are illustrative of the invention.

### Example 1

### Construction of the hG-CSF expression plasmid pCfTA1 (cf. Fig. 1)

A 2-µg portion of the pCSF1-2 DNA obtained in Reference Example 1 was dissolved in a total amount of 20 µl of a solution (hereinafter referred to as "Y-100 buffer") containing 10 mM Tris-HCI (pH 7.5), 7 mM MgCl₂, 6 mM 2-mercaptoethanol and 100 mM NaCI. 10 units each of the restriction enzymes ApaI (Boehringer Mannheim) and BamHI (Takara Shuzo; hereinafter, unless otherwise specified, all the restriction enzymes used were obtained from Takara Shuzo) were added, and the reaction was carried out at 37°C for 4 hours. From the reaction mixture, there was purified and recovered 0.4 µg of a 1.5 kb DNA fragment by the LGT method.

Separately, 2 µg of the plasmid pLSA1 prepared by the method of Reference Example 3 was dissolved in 20 µl of Y-100 buffer, 10 units each of the restriction enzymes BanIII (Toyobo) and BamHI were added, and the reaction was carried out at 37°C for 4 hours. From this reaction mixture, there was purified and recovered 0.8 µg of a 2.8 kb DNA fragment by the LGT method.

On the other hand, the following DNA linker was synthesized to provide the codons coding for the first to fifth N-terminal amino acids of the mature hG-CSF polypeptide [threonine¹ (ACA or ACT), proline² (CCA or CCT), leucine³ (CTA), glycine⁴ (GGC) and proline⁵ (CCC)] and the initiation codon (ATG) required for the expression and for adjusting the distance between the SD sequence and ATG downstream from the tryptophan promoter (Ptrp) to an appropriate length between 6-18 bp:

BanIII HindIII

Met Thr¹ Pro² Leu³ Gly⁴ Pro⁵

5'-CGATAAGCTT ATG AC(A/T) CC(A/T) CTA GGC C -3' 26 mer (4mix)

3'- TATTCGAA TAC TG(T/A) GG(T/A) GAT -5' 20 mer (4mix)

First, the 26-mer and 20-mer single-stranded DNAs were synthesized by the phosphotriester method [R. Crea et al.: Proc. Natl. Acad. Sci. USA, 75, 5765 (1978)]. The 26-mer and 20-mer (each 2 µg) were dissolved in 40 µl of a buffer (hereinafter referred to as "T4 kinase buffer") containing 50 mM Tris-HCI (pH 7.5), 10 mM MgCl₂, 5 mM dithiothreitol, 0.1 mM EDTA and 1 mM ATP. 30 units of T4 polynucleotide kinase (Takara Shuzo; hereinafter the same shall apply) was added, and the phosphorylation reaction was carried out at 37°C for 60 minutes.

In 25 µl of a buffer (hereinafter referred to as "T4 ligase buffer") containing 20 mM Tris-HCI (pH 7.6), 10 mM MgCl₂, 10 mM dithiothreitol and 1 mM ATP, there were dissolved 0.4 µg of the pCSF1-2-derived ApaI-BamHI fragment (1.5 kb) obtained in the above manner and 0.2 µg of the pLSA1-derived BanIII-BamHI fragment (2.8 kb) obtained in the above manner; 0.1 µg of the above-mentioned DNA linker was added to the mixture. To this mixed solution, there was further added 6 units of T4 DNA ligase (obtained from Takara Shuzo; hereinafter the same shall apply), and the ligation reaction was carried out at 4°C for 18 hours.

The thus-obtained recombinant plasmid mixture was used to transform E. coli HB101 [Bolivar et al.: Gene, 2, 75 (1977)] by the method of Cohen at al. [S. N. Cohen et al.: Proc. Natl. Acad. Aci. USA, 69, 2110 (1972)] (hereinafter, this method was used for transforming E. coli), and an Ap^{r} colony was obtained. The plasmid DNA was recovered from the cultured cells of this colony by the known method [H. C. Birnboim et al.: Nucleic Acids Res., 7, 1513 (1979)] (hereinafter, this method was used for plasmid DNA separation). The structure of the plasmid obtained was confirmed by cleavage with BanIII, RsaI, PstI HindIII and BglII followed by agarose gel electrophoresis. This plasmid is called pCfTA1. The base sequence of pCfTA1 in the neighborhood of the BanIII and HindIII sites was confirmed to be as follows by the dideoxy sequencing method using M13 phage:

BanIII HindIII
Met Thr Pro Leu Gly Pro
CGAT AAGCTT ATG ACT CCT CTA GGC CCT

### Example 2

### Construction of the plasmid pCfTB20 lacking in part of the 3'-untranslated region of the hG-CSF cDNA (cf. Fig. 2)

In 20 µl of Y-100 buffer, there was dissolved 2 µg of the hG-CSF expression plasmid pCfTA1 (4.3 kb) obtained in Example 1, 4 units of the restriction enzyme BamHI was added, and the digestion reaction was carried out at 37°C for 4 hours. After extraction with a mixture of an equal volume of phenol and chloroform (hereinafter referred to as phenol-chloroform extraction), 1.8 µg of a DNA fragment was recovered by precipitation with ethanol. This DNA fragment was dissolved in 20 µl of a buffer (hereinafter referred to as "Klenow buffer") containing 50 mM Tris-HCI (pH 7.8), 7 mM MgCl₂ and 6 mM mercaptoethanol, then dATP, dTTP, dCTP and dGTP were added each to a concentration of 1 mM and, after further addition of 4 units of DNA polymerase I Klenow fragment (obtained from Takara Shuzo; hereinafter the same shall apply), and the reaction was carried out at room temperature for 1 hour to thereby convert the protruding ends to blunt ends. After phenol-chloroform extraction, 1.6 µg of a DNA fragment was recovered by ethanol precipitation. This DNA fragment was dissolved in 20 µl of Y-100 buffer, 10 units of EcoRI was added, and the cleavage reaction was carried out at 37°C for 4 hours. From the reaction mixture, there was obtained 1 µg of a 2.5 kb DNA fragment [BamHI(blunt)-EcoRI fragment] by the LGT method.

Separately, 2 µg of pCfTAl was dissolved in 20 µl of Y-100 buffer, 10 units of EcoRI was added, and the cleavage reaction was carried out at 37°C for 4 hours. Thereafter, NaCI was added to an NaCI concentration of 150 mM, then 10 units of Dral was added, and the cleavage reaction was carried out at 37°C for 4 hours. After confirmation of complete cleavage by agarose gel electrophoresis, 0.2 µg of an hG-CSF cDNA-containing 1.0 kb DNA fragment (EcoRI-DraI fragment) was purified and recovered by the LGT method.

In 25 µl of T4 ligase buffer, there were dissolved 0.2 µg of the BamHI (blunt)-EcoRI fragment (2.5 kb) and 0.2 µg of the EcoRI-DraI fragment (1.0 kb) each obtained in the above manner, 6 units of T4 DNA ligase was added to the resultant mixture, and the ligation reaction was carried out at 4°C for 18 hours.

The thus-obtained recombinant plasmid mixture was used to transform E. coli HB101, and an Ap^{r} colony was obtained. From cultured cells derived from this colony, a plasmid DNA was recovered. The structure of the plasmid obtained was confirmed by agarose gel electrophoresis following cleavage with HindIII and PstI. This plasmid is called pCfTB20.

### Example 3

### Construction of the plasmids coding for polypeptides resulting from substitution of the N-terminal amino acid of hG-CSF, namely pCfTL23, pCfTL38, pCfTL35 and pCfTL41 (cf. Fig. 3)

In 60 µl of Y-100 buffer, there was dissolved 3 µg of pCSF1-2 (4.5 kb) obtained by the method of Reference Example 1, 8 units each of the restriction enzymes ApaI (Boehringer Mannheim) and BamHI were added, and the cleavage reaction was carried out at 37°C for 3 hours. From this reaction mixture, there was obtained about 0.4 µg of a DNA fragment of about 1.5 kb (ApaI-BamHI fragment) containing most of the hG-CSF gene.

Separately, 2 µg of pGEL1 [Sekine et al.: Proc. Natl. Acad. Sci. USA, 82, 4306 (1985)] (obtained from a culture of E. coli IGEL1 FERM BP-629 by the conventional method) (3.4 kb) was dissolved in 40 µl of Y-100 buffer, 4 units each of the restriction enzymes HindIII, BamHI and PstI were added, and the cleavage reaction was carried out at 37°C for 3 hours. From the reaction mixture, there was obtained about 0.5 µg of a DNA fragment of about 1.7 kb (PstI-BamHI fragment) containing the lipoprotein-derived terminator (ℓpp terminator) by the LGT method.

Separately, 3 µg of pKYP10 prepared by the method described in Japanese Patent Application (OPI) NO. 110600/83 was dissolved in 60 µl of Y-100 buffer, 6 units each of the restriction enzymes BanIII (Toyobo) and PstI were added, and the cleavage reaction was carried out at 37°C for 3 hours. From the reaction mixture, there was obtained about 0.5 µg of a DNA fragment of about 1.1 kb (BanIII-PstI fragment) containing the tryptophan promoter (Ptrp) by the LGT method.

On the other hand, in view of the necessity of substituting the N-terminal amino acid of mature hG-CSF, namely Thr, with Ser, Cys, Arg or Gly and providing the initiation codon (ATG) required for expression and also in view of adjusting the distance between the SD sequence and ATG downstream from Ptrp to an appropriate length of 6-18 bp, and for other reasons, the following DNA linker was synthesized: 
26 mer (4mix)
Ser
Cys
Arg
BanIII
Met Gly Pro Leu Gly
5'- CG ATAAGCTT ATG NGT CCA CTA GGC C -3'
3'- TATTCGAA TAC NCA GGT GAT -5'
20 mer (4mix)
 In the above formula, N is one of the bases G, A, T and C.

First, the 26-mer and 20-mer single-strand DNAs were synthesized by the ordinary phosphotriester method. The 26-mer and 20-mer (each 20 picomoles) were dissolved in 40 µl of T4 kinase buffer, 6 units of T4 polynucleotide kinase (Takara Shuzo) was added, and the phosphorylation reaction was carried out at 37°C for 60 minutes.

Then, 0.3 µg of the pCSF1-2-derived ApaI-BamHI fragment (about 1.5 kb), 0.2 µg of the pGEL1-derived PstI-BamHI fragment (about 1.7 kb) and 0.2 µg of the expression vector pKYP10-derived BanIII-PstI fragment (about 1.1 kb), each obtained in the above manner, were dissolved in a total of 30 µl of T4 ligase buffer, and about 1 picomole of the above DNA linker was added to the mixture solution. After further addition of 6 units of T4 DNA ligase to the solution, the ligation reaction was carried out at 4°C for 18 hours.

The recombinant plasmid-containing reaction mixture was used to transform E. coli C600SF8 (FERM BP-1070) [Cameron et al.: Proc. Natl. Acad. Sci. USA, 72, 3416 (1975)], and Ap^{r} colonies were obtained. From these transformants, there were separated and purified the plasmid DNAs by known methods. The structure of each of the plasmid DNAs was confirmed by cleavage with PstI, EcoRI and BanIII, followed by polyacrylamide gel electrophoresis. The plasmids obtained in this way are called pCfTL23, pCfTL38, pCfTL35 and pCfTL41, as shown in Fig. 3. The sequences in the vicinity of the N terminus of the hG-CSF derivative genes in said plasmids were confirmed by the dideoxy sequencing method using M13 phage to be as follows:

Met Gly Pro Leu Gly Pro Ala
pCfTL23 ATG GGT CCA CTA GGC CCT GCC

Met Ser Pro Leu Gly Pro Ala
pCfTL38 ATG AGT CCA CTA GGC CCT GCC

Met Cys Pro Leu Gly Pro Ala
pCfTL35 ATG TGT CCA CTA GGC CCT GCC

Met Arg Pro Leu Gly Pro Ala
pCfTL41 ATG CGT CCA CTA GGC CCT GCC

The substitution of the N-terminal Thr of mature hG-CSF was confirmed in the pCfTL23-encoded hG-CSF derivative, which is called hG-CSF[Gly¹]. Similarly, N-terminal amino acid substitution by Ser was confirmed in the pCfTL38-encoded hG-CSF derivative, which is called hG-CSF[Ser¹], substitution by Cys in the pCfTL35-encoded hG-CSF derivative, which is called hG-CSF[Cys¹], and substitution by Arg in the pCfTL41-encoded hG-CSF derivative, which is called hG-CSF[Arg¹].

### Example 4

### Construction of plasmids, pCfTM14, pCfTM17 and pCfTM113, which code for polypeptides resulting from substitution of the N-terminal and third amino acids of hG-CSF (cf. Fig. 4)

In 60 µl of Y-100 buffer, there was dissolved 3 µg of pCSF1-2 (4.5 kb) obtained by the procedure of Reference Example 1, 8 units each of ApaI and BamHI were added, and the cleavage reaction was carried out at 37°C for 3 hours. From this reaction mixture, there was obtained about 0.4 µg of a DNA fragment of about 1.5 kb (ApaI-BamHI fragment) containing most of the hG-CSF gene by the LGT method.

Separately, 2 µg of pGEL1 (3.4 kb) was dissolved in 40 µl of Y-100 buffer, 4 units each of the restriction enzymes HindIII, BamHI and PstI were added, and the cleavage reaction was carried out at 37°C for 3 hours. From this reaction mixture, there was obtained about 0.5 µg of a DNA fragment of about 1.7 kb (PstI-BamHI fragment) containing the lipoprotein terminator by the LGT method.

Further, separately, 3 µg of pKYP10 prepared by the procedure described in Japanese Patent Application (OPI) No. 110600/83 was dissolved in 60 µl of Y-100 buffer, 6 units each of the restriction enzymes BanIII and PstI were added, and the cleavage reaction was conducted at 37°C for 3 hours. From this reaction mixture, there was obtained, by the LGT method, about 0.5 µg of a Ptrp-containing DNA fragment of about 1.1 kb (BanIII-PstI fragment).

In view of the necessity of substituting the N-terminal amino acid Thr of mature hG-CSF with Ser and the third amino acid Leu of mature hG-CSF with one of Gly, Ser, Cys and Arg and providing the initiation codon (ATG) required for expression and also in view of the necessity of adjusting the distance between the SD sequence and ATG downstream from Ptrp and appropriate length of 6-18 bp and for other reasons, the following DNA linker was synthesized:
In the above formula, N is one of the bases G, A, T and C.

(4mix)
26-mer
Gly
Ser
Cys
Met Ser Pro Arg Gly
5' - CG ATA AGCTT ATG AGT CCA NGT GGC C - 3'
3' - TAT TCGAA TAC TCA GGT NCA - 5'
20 - mer (4mix)

First, the 26-mer and 20-mer single-stranded DNAs were synthesized by the ordinary phosphotriester method. The 26-mer and 20-mer (each 20 picomoles) were dissolved in 40 µl of T4 kinase buffer, 6 units of T4 polynucleotide kinase was added, and the phosphorylation reaction was carried out at 37°C for 60 minutes.

Then, 0.3 µg of the pCSF1-2-derived ApaI-BamHI fragment (about 1.5 kb), 0.2 µg of the pGEL1-derived PstI-BamHI fragment (about 1.7 kb) and 0.2 µg of the BanIII-PstI fragment (about 1.1 kb) of the expression vector pKYP10, each obtained in the above manner, were dissolved in 30 µl of T4 ligase buffer, and about 1 picomole of the above DNA linker was added to the mixture solution. After further addition of 6 units of T4 DNA ligase to the solution, the ligation reaction was carried out at 4°C for 18 hours.

The recombinant plasmid-containing reaction mixture was used to transform E. coli C600SF8 (FERM BP-1070) by the method of Cohen et al. and Ap^{r} colonies were obtained. The plasmid DNAs were separated and purified from these transformants by known methods. The structure of each of said plasmid DNAs was confirmed by cleavage with PstI, EcoRI and BanIII, followed by polyacrylamide gel electrophoresis. The plasmids obtained in the above manner are called pCfTM14, pCfTM17 and pCfTM113, as shown in Fig. 4. The sequences in the vicinity of the N terminus of the hG-CSF derivative-encoding genes were confirmed by the dideoxy sequencing method using M13 phage to be as follows:

Met Ser Pro Cys Gly Pro Ala
pCfTM14 - ATG AGT CCA TGT GGC CCT GCC

Met Ser Pro Arg Gly Pro Ala
pCfTM17 - ATG AGT CCA CGT GGC CCT GCC

Met Ser Pro Ser Gly Pro Ala
pCfTM113 - ATG AGT CCA AGT GGC CCT GCC

The substitution of the N-terminal Thr and third amino acid Leu of mature hG-CSF by Ser and Cys, respectively was confirmed in the pCfTM14-encoded derivative, which is called hG-CSF[Ser¹, Cys³]. Similarly, the substitution of the N-terminal Thr and third amino acid Leu by Ser and Arg, respectively was confirmed in the pCfTM17-encoded derivative, which is called hG-CSF[Ser¹, Arg³], and the substitution of the N-terminal Thr and third amino acid Leu by Ser and Ser, respectively in the pCfTM113-encoded derivative, which is called hG-CSF[Ser¹, Ser³].

### Example 5

### (1) Construction of the recombinant plasmid pCfWD1 (cf. Fig. 5)

In 50 µl of Y-100 buffer, there was dissolved 5 µg of pCfTA1 obtained by the procedure of Example 1, 10 units of the restriction enzyme StuI and 10 units of the restriction enzyme BanIII (Toyobo) were added, and the digestion reaction is carried out at 37°C for 1 hour. From the reaction mixture, there was obtained about 0.5 µg of an hG-CSF cDNA-containing DNA fragment of about 1.3 kb (BanIII-StuI fragment). Separately, 3 µg of pKYP26 produced by the procedure of Reference Example 2 was dissolved in 50 µl of Y-100 buffer, 6 units of BamHI was added, and the digestion reaction was carried out at 37°C for 1 hour.

To this was added an equal volume of phenol saturated with 10 mM Tris-HCI (pH 7.5) and 1 mM EDTA. After vigorous stirring, the aqueous layer was collected by low-speed centrifugation (3,300 rpm, 10 minutes; hereinafter, the same conditions were used). An equal volume of chloroform was added and, after vigorous stirring, the aqueous layer was collected by low-speed centrifugation. A 1/10 volume of 3 M sodium acetate was added, 2.5 volumes of ethanol was then added, and the mixture was allowed to stand at -20°C for 1 hour. The precipitate was collected by cold centrifugation (4°C, 11,000 rpm, 10 minutes). This precipitate was dissolved in 30 µl of Klenow buffer, dATP, dTTP, dCTP and dGTP were added each to a concentration of 100 µM, 2 units of DNA polymerase I Klenow fragment was added, and the reaction was carried out at 17°C for 15 minutes. The DNA polymerase I Klenow fragment was inactivated by treating at 68°C for 10 minutes, thereafter NaCI was added to a concentration of 100 mM, 5 units of the restriction enzyme PstI was added, and the digestion reaction was carried out at 37°C for 1 hour. From the reaction mixture, there was obtained, by the LGT method, about 0.6 µg of an ℓpp terminator-containing DNA fragment of about 1.8 kb [BamHI (blunt)-PstI fragment]. Separately, 4 µg of pGEL1 was dissolved in 40 µl of Y-100 buffer, 10 units each of the restriction enzymes BanIII (Toyobo) and PstI were added, and the digestion reaction was conducted at 37°C for 1 hour, and 0.4 µg of tryptophan promoter-containing DNA fragment of about 1 kb (BanIII-PstI fragment) was obtained from the reaction mixture by the LGT method.

About 0.2 µg of the pCfTA1-derived BanIII-StuI fragment (about 1.3 kb), about 0.1 µg of the pKYP26-derived BamHI(blunt)-PstI fragment (about 1.8 kb) and about 0.1 µg of the pGEL1-derived BanIII-PstI fragment (about 1 kb) were dissolved in 30 µl of T4 DNA ligase buffer, 4 units of T4 DNA ligase was added, and the ligation reaction was performed at 4°C for 18 hours.

The reaction mixture was used to transform E. coli HB101 and an Ap^{r} colony was obtained, and the plasmid DNA was recovered from this colony by the above-mentioned method of Birnboim et al. Thus was obtained pCfWD1 shown in Fig. 5.

### (2) Construction of pCfT95K19 (cf. Fig. 6)

In 50 µl of Y-100 buffer, there was dissolved 5 µg of the pCfTL38 obtained by the procedure of Example 3, 10 units each of the restriction enzymes HindIII and BglII were added, and the digestion reaction was carried out at 37°C for 1 hour. About 0.7 µg of a tryptophan promoter-containing DNA fragment of about 2.6 kb (HindIII-BglII fragment) was obtained from the reaction mixture by the LGT method. Separately, 100 µg of pCfTL38 was dissolved in 1.5 ml of Y-100 buffer, 80 units each of the restriction enzymes BamHI and HindIII were added, and the digestion reaction was conducted at 37°C for 6 hours. An hG-CSF cDNA-containing DNA fragment was recovered from the reaction mixture by the LGT method and purified using ELUTIP™ -d (Schleicher & Schuell). This DNA fragment was dissolved in a totoal volume of 90 µl of a solution containing 10 mM Tris-HCI (pH 7.5), 7 mM MgCl₂, 150 mM NaCl and 6 mM 2-mercaptoethanol (hereinafter referred to as "Y-150 buffer"), 3 units of the restriction enzyme DpnI (Boehringer Mannheim) was added, and the digestion reaction was carried out at 37°C for 15 minutes. About 1 µg of an hG-CSF cDNA-containing DNA fragment of about 300 bp (HindIII-DpnI fragment) was obtained from the reaction mixture by polyacrylamide gel electrophoresis.

Separately, 10 µg of pCfTB20 obtained by the procedure of Example 2 was dissolved in 100 µl of Y-100 buffer, 10 units of the restriction enzyme AvaI was added, and the digestion reaction was performed at 37°C for 1 hour. The DNA recovered from the digest by phenolchloroform extraction and ethanol precipitation was dissolved in 30 µl of Klenow buffer, 2 units of DNA polymerase I Klenow fragment was added, and the reaction was carried out at 17°C for 30 minutes. The DNA polymerase I Klenow fragment was inactivated by treating at 68°C for 10 minutes, NaCI was added to 100 mM, 10 units of the restriction enzyme BglII was added, and the digestion reaction was conducted at 37°C for 1 hour. About 0.3 µg of an ℓpp terminator portion-containing DNA fragment of about 480 bp [AvaI(blunt)-BglII fragment] was obtained from the reaction mixture by the LGT method.

In 30 µl of T4 DNA ligase buffer, there were dissolved about 0.1 µg of the pCfTL38-derived HindIII-BglII fragment (about 2.6 kb), about 0.2 µg of the pCfTL38-derived HindIII-DpnI fragment (about bp) and about 0.15 µg of the pCfTB20-derived AvaI(blunt)-BglII fragment (about 480 bp), each obtained in the above manner, and, after addition of 4 units of T4 DNA ligase, the ligation reaction was carried out at 4°C for 18 hours. The reaction mixture was used to transform E. coli HB101 and an Ap^{r} colony was obtained. From this colony, there was recovered the plasmid DNA by the above-mentioned method of Birnboim et al. Thus was obtained pCfT95K19 shown in Fig. 6.

### (3) Construction of pCfAA1 (cf. Fig. 6)

In 50 µl of Y-100 buffer was dissolved 5 µg of pCfT95K19 obtained as described in the preceding section. Thereto were added 7 units of the restriction enzyme BanIII (Toyobo) and 2 units of BglI (Nippon Gene), and the digestion reaction was conducted at 37°C for 1 hour. From the reaction mixture, there were obtained, by the LGT method, about 0.6 µg of tryptophan promoter portion-containing DNA fragment of about 1 kb (BanIII-BglI fragment) and about 1 µg of an ℓpp terminator portion-containing DNA fragment of about 1.8 kb (BglI-BglI fragment).

Separately, 15 µg of pCfT95K19 was dissolved in 150 µl of Y-100 buffer, 6 units of the restriction enzyme BglI (Nippon Gene) and 10 units of Sau3A were added, and the digestion reaction was carried out at 37°C for 1 hour. Polyacrylamide gel electrophoresis of the reaction mixture gave about 0.3 µg of an hG-CSF cDNA portion-containing DNA fragment of about 350 bp (BglI-Sau3A fragment).

Further, separately, the following DNA linker was synthesized:

BanIII
39 mer (2mix) XhoI Sau3A
Met Thr Pro Leu Gly Pro Asn Ser Ser
5'-CGATAAGCTT ATG ACA CCA CT^G_A GGC CCA AAC TCG AGT CT -3'
3'- TATTCGAA TAC TGT GGT GA^C_T CCG GGT TTG AGC TCA GACTAG-5'
41 mer (2mix)

First, the 39-mer and 41-mer single-stranded DNAs were synthesized by the ordinary phosphotriester method. The 39-mer and 41-mer (each 20 picomoles) were dissolved in a total volume of 40 µl of T4 DNA kinase buffer, 6 units of T4 polynucleotide kinase (Takara Shuzo) was added, and the phosphorylation reaction was carried out at 37°C for 60 minutes.

Then, 0.1 µg of the pCfT95K19-derived BanIII-BglI fragment (about 1 kb), 0.05 µg of the BglI-BglI fragment (about 1.8 kb) and 0.1 µg of the BglI-Sau3A fragment (about 350 bp), each obtained in the above manner, were dissolved in 25 µl of T4 DNA ligase buffer, followed by addition of about 2 picomoles of the above DNA linker. After further addition of 6 units of T4 DNA ligase, the ligation reaction was conducted at 4°C for 18 hours.

The reaction mixture was used to transform E. coli HB101, an Ap^{r} colony was obtained, and the plasmid DNA was recovered from this colony by the above-mentioned method of Birnboim et al. Thus was obtained pCfAA1 shown in Fig. 6. Determination of the base sequence of the linker portion of pCfAA1 by the above-mentioned dideoxy sequencing method revealed that the third base of the codon coding for the fourth amino acid Leu is A. In this pCfAA1, the DNA portion coding for the 14 amino acids from the 10th amino acid Pro to the 23rd amino acid Lys of hG-CSF is missing. Furthermore, such mutation has been introduced as to change the 6th amino acid of hG-CSF from Ala to Asn, and there is now a new XhoI site.

### (4) Construction of pCfAB5 (cf. Fig. 6)

In 30 µl of Y-100 buffer was dissolved 3 µg of pCfAA1 obtained as described in the previous section, 5 units of the restriction enzyme XhoI was added, and the digestion reaction was carried out at 37°C for 1 hour. After confirmation of complete XhoI cleavage by agarose gel electrophoresis, 1 unit of the restriction enzyme BglI (Nippon Gene) was added, and partial digestion was effected at 37°C for 25 minutes. From the reaction mixture, there was obtained, by the LGT method, about 1 µg of a tryptophan promoter portion-and ℓpp terminator portion-containing DNA fragment of about 3 kb (XhoI-BglI fragment). Separately, the following DNA linker was synthesized:

XhoI
Ser Ser Leu Pro Gln Ser Phe Leu
TCG AGT CTA CCA CAG AGC TTC CT 23 mer
CA GAT GGT GTC TCG AAG GAAAATTT 25 mer

Cys
Ser
Gly
Sau3A
Leu Lys Arg Leu Glu Gln Val Arg Lys
TTTA AAA NGC TTA GAG CAA GTG AGG AA 27 mer (4mix)
T NCG AAT CTC GTT CAC TCC TTCTAG 25 mer (4mix)

(N being G, A, T or C).

This linker DNA contains that DNA portion which codes for the 14 amino acids of hG-CSF from the 10th amino acid Pro to the 23rd amino acid Lys. Such portion is missing in the hG-CSF cDNA of pCfAA1.

First, the 27-mer, 25-mer (two kinds) and 23-mer single-stranded DNAs were synthesized by the ordinary phosphotriester method. The 27-mer and 25-mer DNAs complementary to each other and the 25-mer and 23-mer DNAs complementary to each other were dissolved in pairs, and each in an amount of 20 picomoles, in a total volume of 40 µl of T4 kinase buffer; 6 units of T4 polynucleotide kinase (Takara Shuzo) was added to each solution, and the phosphorylation reaction was performed at 37°C for 60 minutes.

Then, 0.1 µg of the pCfAA1-derived XhoI-BglI fragment (about 3 kb) obtained as described above and 0.1 µg of the pCfT95K19-derived BglI-Sau3A fragment (about 350 bp) obtained as described in the previous section were dissolved in 30 µl of T4 DNA ligase buffer, and 2 picomoles each of the above DNA linker portions were added to the mixture solution. Further, 6 units of T4 DNA ligase was added, and the ligation reaction was conducted at 4°C for 18 hours.

The reaction mixture was used to transform E. coli HB101 and Ap^{r} colonies were obtained. From these colonies, the plasmid DNAs were recovered by the above-mentioned method of Birnboim et al. There were thus obtained pCfAB5 and pCfAB14 shown in Fig. 6. Determination of the base sequence of the DNA linker moiety of pCfAB5 and of pCfAB14 by the above-mentioned dideoxy sequencing method revealed that the first base of the codon coding for the 17th amino acid is A in pCfAB5 and T in pCfAB14, hence said codon in for Ser (AGC) in the former and for Cys (TGC) in the latter, leading to substitution of Ser for the 17th amino acid Cys of mature hG-CSF in pCfAB5, but no substitution in pCfAB14.

### Example 6

### (1) Construction of pCfBA8 and pCfBA32 (cf. Fig. 7)

In 40 µl of Y-100 buffer were dissolved 3 µg of pCfAB5 obtained as described in the previous section, 5 units each of the restriction enzymes AvaI and BglII were added, and the digestion reaction was conducted at 37°C for 1 hour. From the reaction mixture, there was obtained, by the LGT method, about 1 µg of a tryptophan promoter portion- and an ℓpp terminator portion-containing DNA fragment of about 2.8 kb (AvaI-BglII fragment).

Separately, 6 µg of pCfWD1 obtained as described in section 1 was dissolved in 50 µl of Y-100 buffer, 5 units of the restriction enzyme BglII was added, and the digestion reaction was carried out at 37°C for 1 hour. Agarose gel electrophoresis confirmed that the cleavage with BglII was complete. Thereafter, 3 units of the restriction enzyme AvaI was added, and partial cleavage was effected at 37°C for 20 minutes. From the reaction mixture, there were obtained, by the LGT method, 0.4 µg of a DNA fragment (about 1.3 kb) containing most of the hG-CSF (BglII-AvaI fragmnent).

Then, 0.1 µg of the pCfAB5-derived AvaI-BglII fragment (about 2.8 kb) and 0.3 µg of the pCfWD1-derived BglII-AvaI fragment (about 1.3 kb), each obtained as described above, were dissolved in 25 µl of T4 DNA ligase buffer, and 3 units of T4 DNA ligase was added, and the ligation reaction was carried out at 4°C for 18 hours.

The reaction mixture was used to transform E. coli HB101 and an Ap^{r} colony was obtained. From this colony, the plasmid DNA was recovered by the above-mentioned method of Birnboim et al. Thus was obtained pCfBA8 shown in Fig. 7.

The amino acid sequence of the hG-CSF derivative encoded by pCfBA8 contains Asn in place of the 6th amino acid Ala of mature hG-CSF and Ser in place of the 17th amino acid Cys thereof. Hereinafter, this derivative is referred to as hG-CSF[NA8].

On the other hand, 3 µg of pCfAB14 obtained as described in the previous section were dissolved in 40 µl of Y-100 buffer, 5 units each of the restriction enzyme AvaI and BglII were added, and the digestion reaction was performed at 37°C for 1 hour. From the reaction mixture, there was obtained, by the LGT method, about 1 µg of a tryptophan promoter portion- and an ℓpp terminator portion-containing DNA fragment of about 2.8 kb (AvaI-BglII fragment).

Separately, 6 µg of pCfWD1 obtained as described in section 1 was dissolved in 50 µl of Y-100 buffer, 5 units of the restriction enzyme BglII were added, and the digestion reaction was carried out at 37°C of 1 hour. After confirmation of the completeness of the BglII cleavage by agarose gel electrophoresis, 3 units of the restriction enzyme AvaI were added, and partial cleavage was effected at 37°C for 20 minutes. From the reaction mixture, there were obtained, by the LGT method, 0.4 µg of a DNA fragment (about 1.3 kb) containing most of the hG-CSF cDNA (BglII-AvaI fragment).

Then, 0.1 µg of the pCfAB14-derived AvaI-BglII fragment (about 2.8 kb) and 0.3 µg of the pCfWD1-derived BglII-AvaI fragment (about 1.3 kb), each obtained as described above, were dissolved in 25 µl of T4 DNA ligase buffer, 3 units of T4 DNA ligase were added, and the ligation reaction was performed at 4°C for 18 hours.

The reaction mixture was used to transform E. coil HB101 and an Ap^{r} colony was obtained and, from this colony, the plasmid DNA was recovered by the above-mentioned method of Birnboim et al. Thus was obtained pCfBA32 shown in Fig. 7.

The amino acid sequence of the hG-CSF derivative encoded by pCfBA32 contains Asn in lieu of the 6th amino acid Ala of mature hG-CSF.

### (2) Construction of pCfBB101

In 50 µl of Y-100 buffer were dissolved 6 µg of pCfBA8 obtained as described in the previous section, 10 units of the restriction enzyme BanIII (Toyobo), 8 units of BglII and 8 units of XhoI were added, and the digestion reaction was conducted at 37°C for 1 hour. From the reaction mixture, there were obtained, by the LGT method, about 0.6 µg of an hG-CSF cDNA-containing DNA fragment of about 1.4 kb (XhoI-BglII fragment) and about 0.8 µg of a tryptophan promoter portion-containing DNA fragment of about 2.7 kb (BanIII-BglII fragment).

Separately, the following DNA linker was synthesized:

31 mer
XhoI
BanIII
Met Ala Pro Thr Arg Ser Ala
5'-CGATAAGCTT ATG GCA CCA ACA AGA AGC GCC -3'
3'- TATTCGAA TAC CGT GGT TGT TCT TCG CGG AGCT -5'
33 mer

First, the 31-mer and 33-mer single-stranded DNAs were synthesized by the ordinary phosphotriester method. The 31-mer and 33-mer (each 2 µg) were dissolved in a total of 40 µl of T4 kinase buffer, 30 units of T4 polynucleotide kinase (Takara Shuzo) were added, and the phosphorylation reaction was conducted at 37°C for 60 minutes.

Then, 0.1 µg of the pCfBA8-derived BanIII-BglII fragment (about 2.7 kb fragment) and 0.1 µg of the pCfBA8-derived XhoI-BglII fragment (about 1.4 kb fragment), each obtained as described above, were dissolved in 25 µl of T4 DNA ligase buffer, and about 2 picomoles of the above DNA linker were added to the mixture solution. After further addition of 6 units of T4 DNA ligase, the ligation reaction was carried out at 4°C for 18 hours.

The recombinant plasmid mixture thus obtained was used to transform E. coli HB101 and an Ap^{r} colony was obtained. From cultured cells derived from this colony, there was recovered the plasmid DNA. Thus was obtained pCfBB101 shown in Fig. 7. The amino acid sequence of the hG-CSF derivative encoded by pCfBB101 contains Ala, Thr, Arg, Ser and Ser in lieu of the first amino acid Thr, third amino acid Leu, fourth amino acid Gly, fifth amino acid Pro and 17th amino acid Cys of mature hG-CSF, respectively. Hereinafter, this derivative is referred to as hG-CSF[NB101].

### (3) Construction of pCfBC42B1, pCfBC45, pCfBC52, pCfBC59, pCfBC76, pCfBC77, pCfBC93, pCfBC95 and pCfBC97 (Cf. Fig. 8)

First, the following DNA linker was synthesized:

31 mer (128mix)                    XhoI

BanIII          Met-(16)-Pro-(8)-Arg Ser Ala

5'-CGATAAGCTT ATG   NNT CCA (G/A)NA AGA AGC GCC        -3'

  3'-TATTCGAA TAC   NNA GGT (C/T)NT TCT TCG CGG AGCT    -5'

                    33 mer (128mix)

   (N being one of G, A, T and C).

In this synthetic DNA linker, the three bases each represented by N are each independently one of G, A, T and C and one base is G or A (in the case of 31-mer) or C or T (in the case of 33-mer) and therefore this linker is obtained as a mixture of a total of 128 DNA linkers. As a result, the design of this linker is such that, in the N-terminal hG-CSF amino acid sequence encoded by this linker, 16 different amino acids are possible as the amino acid next to Met and 8 different amino acids are possible as the amino acid next to Pro, hence 128 amino acid sequences in total are possible.

First, the 31-mer and 33-mer single-stranded DNAs were synthesized by the ordinary phosphotriester method. The 31-mer and 33-mer (each 2 µg) were dissolved in a total volume of 40 µl of T4 kinase buffer, 30 units of T4 polynucleotide kinase (Takara Shuzo) were added, and the phosphorylation reaction was conducted at 37°C for 60 minutes.

Then, 0.1 µg of the pCfBA8-derived BanIII-BglII fragment (about 2.7 kb fragment) and 0.1 µg of the pCfBA8-derived XhoI-BglII fragment (about 1.4 kb fragment), each obtained in Example 6, were dissolved in 25 µl of T4 DNA ligase buffer, and about 2 picomoles of the above DNA linkers were added to the mixture solution. After further addition of 6 units of T4 DNA ligase, the ligation reaction was carried out at 4°C for 18 hours.

The recombinant plasmid mixture thus obtained was used to transform E. coli HB101 and Ap^{r} colonies were obtained. From cultured cells of these colonies, the plasmid DNAs were recovered. Thus were obtained pCfBC42B1, pCfBC45, pCfBC52, pCfBC59, pCfBC76, pCfBC77, pCfBC93, pCfBC95 and pCfBC97. Determination of the base sequence in each DNA linker moiety by the above-mentioned dideoxy sequencing method revealed that the base sequences on the N-terminal side of hG-CSF derivatives are as follows:

Met Thr Pro Glu Lys Ser Ala
pCfBC42B1 ATG ACT CCA GAA AAA AGC GCC

Met Val Pro Ile Arg Ser Ala
pCfBC45 ATG GTT CCA ATA AGA AGC GCC

Met Cys Pro Ile Arg Ser Ala
pCfBC52 ATG TGT CCA ATA AGA AGC GCC

Met Tyr Pro Ile Arg Ser Ala
pCfBC59 ATG TAT CCA ATA AGA AGC GCC

Met Arg Pro Thr Arg Ser Ala
pCfBC76 ATG CGT CCA ACA AGA AGC GCC

Met Thr Pro Thr Arg Ser Ala
pCfBC77 ATG ACT CCA ACA AGA AGC GCC

Met Asn Pro Glu Arg Ser Ala
pCfBC93 ATG AAT CCA GAA AGA AGC GCC

Met Ile Pro Thr Arg Ser Ala
pCfBC95 ATG ATT CCA ACA AGA AGC GCC

Met Ser Pro Thr Arg Ser Ala
pCfBC97 ATG AGT CCA ACA AGA AGC GCC

The substituent amino acid residues in the hG-CSF derivatives encoded by these plasmids as respectively compared with mature hG-CSF are as follows:

| Plasmid | Position of amino acid substitution (amino acid of hG-CSF) | | | | |
|---|---|---|---|---|---|
| | 1st (Thr) | 3rd (Leu) | 4th (Gly) | 5th (Pro) | 17th (Cys) |
| pCfBC42B1 | -* | Glu | Lys | Ser | Ser |
| pCfBC45 | Val | Ile | Arg | Ser | Ser |
| pCfBC52 | Cys | Ile | Arg | Ser | Ser |
| pCfBC59 | Tyr | Ile | Arg | Ser | Ser |
| pCfBC76 | Arg | Thr | Arg | Ser | Ser |
| pCfBC77 | -* | Thr | Arg | Ser | Ser |
| pCfBC93 | Asn | Glu | Arg | Ser | Ser |
| pCfBC95 | Ile | Thr | Arg | Ser | Ser |
| pCfBC97 | Ser | Thr | Arg | Ser | Ser |

| | | | | | |
|---|---|---|---|---|---|
| * No substitution | | | | | |

The hG-CSF derivatives encoded by pCfBC42B1, pCfBC45, pCfBC52, pCfBC59, pCfBC76, pCfBC77, pCfBC93, pCfBC95 and pCfBC97 are hereinafter referred to as hG-CSF[NC42B1], hG-CSF[NC45), hG-CSF[NC52], hG-CSF[NC59], hG-CSF[NC76], hG-CSF[NC77], hG-CSF[NC93], hG-CSF[NC95] and hG-CSF[NC97], respectively.

### (4) Construction of pCfBD28, pCfBD56 and pCfBD82

First, the following DNA linker was synthesized:

31 mer (256mix)          XhoI
BanIII          Met Ala Pro (4) (4) (4) (4)
5'-CGATAAGCTT ATG GCA CCA NCA NAT NGC GNC  -3'
3'-TATTCGAA TAC CGT GGT NGT NTA NCG CNG AGCT  -5'
33 mer (256mix)

(N being one of G, A, T and C)

In this DNA linker, the four bases represented by N are each independently G, A, T or C and, accordingly, the linker is obtained as a mixture of a total of 256 different DNA linkers. As a result, the design of this DNA linker is such that, in the N-terminal hG-CSF amino acid sequence encoded by the DNA linker, four amino acids are possible in each of the four positions in question, hence totally 256 different amino acid sequences are possible.

First the 31-mer and 33-mer single-stranded DNAs were synthesized by the ordinary phosphotriester method. In a total of 40 µl of T4 kinase buffer, there were dissolved 2 µg each of the 31-mer and 33-mer, 30 units of T4 polynucleotide kinase (Takara Shuzo) were added, and phosphorylation reaction was performed at 37°C for 60 minutes.

Then, 0.1 µg of the pCfBA8-derived BanIII-BglII fragment (about 2.7 kb fragment) and 0.1 µg of the pCfBA8-derived XhoI-BglII fragment (about 1.4 kb fragment), each obtained in Example 6, were dissolved in 25 µl of T4 DNA ligase buffer, and about 2 picomoles of the above DNA linkers were added to the mixture solution. After further addition of 6 units of T4 DNA ligase, the ligation reaction was conducted at 4°C for 18 hours.

The recombinant plasmid mixture obtained was used to transform E. coli HB101, and Ap^{r} colonies were obtained. From cultured cells of these colonies, the plasmids were respectively recovered. Thus were obtained pCfBD28, pCfBD56 and pCfBD82. Determination of the base sequence in the DNA linker moiety by the above-mentioned dideoxy sequencing method revealed that the base sequences on the N-terminal side of the hG-CSF derivatives are as follows:

Met Ala Pro Thr Tyr Arg Ala
pCfBD28 ATG GCA CCA ACA TAT CGC GCC

Met Ala Pro Ser Asn Ser Ala
pCfBD56 ATG GCA CCA TCA AAT AGC GCC

Met Ala Pro Pro Asn Arg Gly
pCfBD82 ATG GCA CCA CCA AAT CGC GGC

The replacing amino acid residues in the hG-CSF derivatives encoded by these plasmids as compared with mature hG-CSF are as follows;

| Position of amino acid substitution (amino acid of hG-CSF) | Plasmid | | |
|---|---|---|---|
| | pCfBD28 | pCfBD56 | pCfBD82 |
| 1st (Thr) | Ala | Ala | Ala |
| 3rd (Leu) | Thr | Ser | Pro |
| 4th (Gly) | Tyr | Asn | Asn |
| 5th (Pro) | Arg | Ser | Arg |
| 6th (Ala) | -* | -* | Gly |
| 17th (Cys) | Ser | Ser | Ser |

| | | | |
|---|---|---|---|
| * No substitution. | | | |

The hG-CSF derivatives encoded by pCfBD28, pCfBD56, and pCfBD82 are hereinafter referred to as hG-CSF[ND28], hG-CSF[ND56] and hG-CSF[ND82], respectively. An E. coli strain harboring pCfBD56, E. coli ECfBD56, and an E. coli strain harboring pCfBD28, E. coli ECfBD28, have been deposited at the Fermentation Research Institute under the deposit numbers FERM BP-1221 and FERM BP-1479, respectively in accordance with the Budapest treaty.

### (5) Construction of pCfTNS7 (cf Fig. 14)

The following DNA linker was synthesized.

18 mer

BanIII                    XhoI
                Met Pro Ala
5'-CGATAAGCT ATG CCA GCC    -3'
3'-  TATTCGA TAC GGT CGG AGCT  -5'

20 mer

According to the design of this DNA linker, the four amino acids from the 1st amino acid Thr to the 4th amino acid Gly of the N-terminal amino acid sequence of hG-CSF are missing in the N-terminal amino acid sequence encoded by the linker.

First, the 18-mer and 20-mer single-stranded DNAs were synthesized by the ordinary phosphotriester method. The 18-mer and 20-mer (each 2 µg) were dissolved in a total of 40 µl of T4 kinase buffer, 30 units of T4 polynucleotide kinase (Takara Shuzo) were added, and the phosphorylation reaction was performed at 37°C for 60 minutes.

Then, 0.1 µg of the pCfBA8-derived BanIII-BglII fragment (about 2.7 kb fragment) and 0.1 µg of the pCfBA8-derived XhoI-BglII fragment (about 1.4 kb), each obtained in Example 6, were dissolved in 25 µl of T4 DNA ligase buffer, about 2 picomoles of the above DNA linker were added to the mixture solution, and the ligation reaction was conducted at 4°C for 18 hours.

The recombinant plasmid mixture thus obtained was used to transform E. coli HB101, and an Ap^{r} colony was obtained. From cultured cells of this colony, there was recovered the plasmid. Thus was obtained pCfTNS7. In the hG-CSF derivative encoded by pCfTNS7, the 1st to 4th N-terminal amino acids of mature hG-CSF are missing and the 17th amino acid is Ser in place of Cys. The hG-CSF derivative encoded by pCfTNS7 is hereinafter referred to as hG-CSF[Δ1-4S].

### (6) Construction of pCfTAArg4S (cf. Fig. 14)

The following DNA linker was synthesized:

31 mer (4mix)

Arg
Gly          XhoI
BanIII          Lys
Met Thr Pro Leu Glu Pro Ala

                                                    AA
5'-CGATAAGCTT ATG ACA CCA CTA    A CCA GCC   -3'
                                                    GG

                                                 TT
3'-TATTCGAA TAC TGT GGT GAT    T GGT CGGAGCT   -5'
                                                 CC

33 mer (4mix)

In this DNA linker, two bases each independently is A or G, hence said linker is obtained as a mixture of a total of four DNA linkers. Accordingly, the design of this DNA linker is such that four amino acids are possible as the 4th amino acid in the N-terminal hG-CSF amino acid sequence encoded by said linker.

First, the 31-mer and 33-mer single-stranded DNAs were synthesized by the ordinary phosphotriester method. The 31-mer and 33-mer (each 2 µg) were dissolved in a total volume of 40 µl of T4 kinase buffer, 30 units of T4 polynucleotide kinase (Takara Shuzo) were added, and the phosphorylation reaction was carried out at 37°C for 60 minutes.

Then, 0.1 µg of the pCfBA8-derived BanIII-BglII fragment (about 2.7 kb fragment) and 0.1 µg of the pCfBA8-derived XhoI-BglII fragment (about 1.4 kb fragment), each obtained as described in section (1), were dissolved in 25 µl of T4 DNA ligase buffer, and about 2 picomoles of the above DNA linkers were added to the mixture solution. After further addition of 6 units of T4 DNA ligase, the ligation reaction was performed at 4°C for 18 hours.

The recombinant plasmid mixture thus obtained was used to transform E. coli HB101, and an Ap^{r} colony was obtained. From cultured cells of this colony, there was recovered the plasmid. Thus was obtained pCfTAArg4S. Determination of the base sequence of the DNA linker moiety by the above-mentioned dideoxy sequencing method revealed that the N-terminal base sequence of the hG-CSF derivative is as follows:

          Met Thr Pro Leu Arg Pro Ala
pCfTAArg4S   ATG ACA CCA CTA AGA CCA GCC

The hG-CSF derivative encoded by pCfTAArg4S is hereinafter referred to as hG-CSF[Arg⁴,Ser¹⁷].

### (7) Construction of pCfTN205 (cf. Fig. 15)

In 40 µl of K-150 buffer (same as Y-100 buffer except for replacement of 150 mM KCI for 100 mM NaCI), there was 3 µg of pCfTNS7 obtained in section 5, 5 units each of the restriction enzymes PvuI and XhoI, and the digestion reaction was conducted at 37°C for 1 hour. From the reaction mixture, there were obtained, by the LGT method, about 0.5 µg of a tryptophan promoter portion-containing DNA fragment of about 1.0 kb (PvuI-XhoI fragment).

Separately, 3 µg of pCfBA32 obtained in section 1 were dissolved in 40 µl of K-150 buffer, 5 units each of the restriction enzymes PvuI and XhoI were added, and the digestion reaction was carried out at 37°C for 1 hour. From the reaction mixture, there were obtained, by the LGT method, 2 µg of an about 3.0 kb DNA fragment (XhoI-PvuI fragment) containing most of the hG-CSF cDNA.

Then, 0.1 µg of the pCfTNS7-derived PvuI-XhoI fragment (about 1.0 kb) and 0.3 µg of the pCfBA32-derived XhoI-PvuI fragment (about 3.0 kb), each obtained in the above manner, were dissolved in 25 µl of T4 DNA ligase buffer, 3 units of T4 DNA ligase were added, and the ligation reaction was conducted at 4°C for 18 hours.

The reaction mixture was used to transform E. coli HB101, and Ap^{r} colony was obtained, and the plasmid DNA was recovered from this colony by the above-mentioned method of Birnboim et al. Thus was obtained pCfTN205 shown in Fig. 15.

In the amino acid sequence of the hG-CSF derivative encoded by pCfTN205, the 1st to 4th amino acids of mature hG-CSF are missing. Hereinafter, this derivative is referred to as hG-CSF[Δ1-4].

### (8) Construction of pCfTAArg4 (cf. Fig. 15)

In 40 µl of K-150 buffer, there were dissolved 3 µg of pCfTAArg4S obtained in section 6, 5 units each of the restriction enzymes PvuI and XhoI were added, and the digestion reaction was performed at 37°C for 1 hour. From the reaction mixture, there were obtained by the LGT method, about 0.5 µg of a tryptophan promoter portion-containing DNA fragment of about 1.0 kb (PvuI-XhoI fragment).

Separately, 3 µg of pCfBA32 obtained in section 1 were dissolved in 40 µl of K-150 buffer, 5 units each of the restriction enzymes PvuI and XhoI were added, and the digestion reaction was conducted at 37°C for 1 hour. From the reaction mixture, there were obtained, by the LGT method, 2 µg of an about 3.0 kb DNA fragment (XhoI-PvuI fragment) containing most of the hG-CSF cDNA.

Then, 0.1 µg of the pCfTAArg4S-derived PvuI-XhoI fragment (about 1.0 kb) and 0.3 µg of the pCfBA32-derived XhoI-PvuI fragment (about 3.0 kb), each obtained in the above manner, were dissolved in 25 µl of T4 DNA ligase buffer, 3 units of T4 DNA ligase were added, and the ligation reaction was carried out at 4°C for 18 hours.

The reaction mixture was used to transform E. coli HB101, Ap^{r} colony was obtained, and the plasmid DNA was recovered from this colony by the above-mentioned method of Birnboim et al. Thus was obtained pCfTAArg4 shown in Fig. 15.

The fourth amino acid in the amino acid sequence of the hG-CSF derivative encoded by pCfTAArg4 is Arg in lieu of Gly in mature hG-CSF. Hereinafter, this derivative is called hG-GSF[Arg⁴].

### (9) Construction of pCfTNS301 (cf. Fig. 16)

In 50 µl of Y-100 buffer, were dissolved 6 µg of pCfBA8 obtained in section 1, 10 units of the restriction enzyme HindIII and 8 units of BglII were added, and the digestion reaction was carried out at 37°C for 1 hour. From the reaction mixture, there were obtained, by the LGT method, about 0.6 µg of an hG-CSF cDNA-containing DNA fragment of about 1.4 kb (HindIII-BglII fragment).

Then, the following DNA linker was synthesized:

27 mer
BanIII                                    HindIII
              Met Ser Phe Leu Leu Lys
5'-CGATAAGCT ATG TCA TTT CTT TTA AAA        -3'
     3'
   TATTCGA TAC AGT AAA GAA AAT TTT TCGA     -5'

29 mer

The design of this DNA linker is such that the 11 amino acids from the first amino acid Thr to the 11th amino acid Gln of hG-CSF are missing in the N-terminal amino acid sequence encoded by said linker.

First, the 27-mer and 29-mer single-stranded DNAs were synthesized by the ordinary phosphotriester method. The 27-mer and 29-mer (each 2 µg) were dissolved in a total of 40 µl of T4 kinase buffer, 30 units of T4 polynucleotide kinase (Takara Shuzo) was added, and the phosphorylation reaction was conducted at 37°C for 60 minutes.

Then, 0.1 µg of the pCfBA8-derived BanIII-BglII fragment (about 2.7 kb fragment) and 0.1 µg of the pCfBA8-derived HindIII-BglII fragment (about 1.4 kb fragment), each obtained as mentioned hereinabove, were dissolved in 25 µl of T4 DNA ligase buffer, and about 2 picomoles of the above DNA linker were added to the mixture solution. After further addition of 6 units of T4 DNA ligase, the ligation reaction was carried out at 4°C for 18 hours.

The recombinant plasmid mixture thus obtained was used to transform E. coli HB101, and an Ap^{r} colony was obtained. From cultured cells of this colony, there was recovered the plasmid DNA. Thus was obtained pCfTNS301. In the hG-CSF derivative encoded by pCfTNS301 the 1st to 11th N-terminal amino acids of mature hG-CSF are missing and the 17th amino acid is Ser in place of Cys. The hG-CSF derivative encoded by pCfTNS301 is hereinafter called hG-CSF[Δ1-11S].

### (10) Construction of pCfTNS401 (cf. Fig. 16)

The following DNA linker was synthesized:

39 mer
BanIII                    Pro Gln Ser Phe Leu Leu    HindIII
          Met Ser Leu                                Lys
5'-CGATAAGCT ATG TCA CTA CCA CAA TCA TTT CTA TTA' AAA    3'
3'-  TATTCGA TAC AGT GAT GGT GTT AGT AAA GAT AAT TTTTCGA -5'
41 mer

The design of this DNA linker is such that the 7 amino acids from the first amino acid Thr to the 7th amino acid Ser of hG-CSF are missing in the N-terminal amino acid sequence encoded by the linker.

First, the 39-mer and 41-mer single-stranded DNAs were synthesized by the ordinary phosphotriester method. The 39-mer and 41-mer (each 2 µg) were dissolved in a total of 40 µl of T4 kinase buffer, 30 units of T4 polynucleotide kinase (Takara Shuzo) were added, and the phosphorylation reaction was performed at 37°C for 60 minutes.

Then, 0.1 µg of the pCfBA8-derived BanIII-BglII fragment (about 2.7 kb fragment) and 0.1 µg of the pCfBA8-derived HindIII-BglII fragment (about 1.4 kb fragment), each obtained as described above, were dissolved in 25 µl of T4 DNA ligase buffer, and about 2 picomoles of the above-mentioned DNA linker were added to this mixture solution. After further addition of 6 units of T4 DNA ligase, the ligation reaction was conducted at 4°C for 18 hours.

The recombinant plasmid mixture thus obtained was used to transform E. coli HB101, and an Ap^{r} colony was obtained. The plasmid DNA was recovered from this colony. Thus was obtained pCfTNS401. In the hG-CSF derivative encoded by pCfTNS401 the 1st to 7th N-terminal amino acids of mature hG-CSF are missing and the 17th amino acid is Ser in place of Cys. The hG-CSF derivative encoded by pCfTNS401 is hereinafter called hG-CSF[Δ1-7S].

### (11) Construction of pCfTNS501 (cf. Fig. 12)

In 40 µl of Y-100 buffer, there was dissolved pCfBA8-obtained in section 1, 10 units of the restriction enzyme XhoI were added, and the digestion reaction was carried out at 37°C for 1 hour. The DNA recovered by phenol-chloroform extraction and ethanol precipitation was dissolved in 30 µl of Klenow buffer, 2 units of DNA polymerase I Klenow fragment were added, and the reaction was carried out at 17°C for 30 minutes. The DNA polymerase I Klenow fragment was inactivated by 10-minute treatment at 68°C, KCI was added to a concentration of 150 nM, 8 units of the restriction enzyme PvuI were added, and the digestion reaction was conducted at 37°C for 1 hour. From the reaction mixture, there were obtained, by the LGT method, about 2 µg of an hG-CSF cDNA-containing DNA fragment of about 3 kb [XhoI(blunt)-PvuI].

Separately, 5 µg of the ATG vector pTrS20 (3.8 kb) obtained by the procedure of Reference Exlample 4 were dissolved in 50 µl of Y-0 buffer (Y-100 buffer minus 100 mM NaCI), 16 units of the restriction enzyme SacI were added, and the cleavage reaction was carried out at 37°C for 3 hours. The DNA recovered by phenol-chloroform extraction and ethanol precipitation was dissolved in 30 µl of Klenow buffer, 2 units of DNA polymerase I Klenow fragment were added, and the reaction was conducted at 17°C for 30 minutes. The DNA polymerase I klenow fragment was inactivated by treatment at 68°C for 10 minutes, KCI was added to a concentration of 150 mM, 8 units of the restriction enzyme PvuI were added, and the digestion reaction was performed at 37°C for 1 hour. From the reaction mixture, there were obtained, by the LGT method, about 0.5 µg of a Ptrp-containing DNA fragment of about 1 kb [SacI(blunt)-PvuI].

Then, 0.1 µg of the pCfBA8-derived XhoI(blunt)-PvuI fragment (about 3 kb) and 0.2 µg of the pTrS20-derived SacI(blunt)-PvuI fragment (about 1 kb) were dissolved in 25 µl of T4 DNA ligase buffer, 3 units of T4 DNA ligase were added, and the ligation reaction was carried out at 4°C for 18 hours.

The reaction mixture was used to transform E. coli HB101, an Ap^{r} colony was obtained, and the plasmid DNA was recovered by the above-mentioned method of Birnboim et al. Thus was obtained pCfTNS501 shown in Fig. 12.

In the hG-CSF derivative encoded by pCfTNS501, the 1st to 6th N-terminal amino acids of mature hG-CSF are missing and the 17th amino acid is Ser in place of Cys. The hG-CSF derivative encoded by pCfTNS501 is hereinafter called hG-CSF[Δ1-6S].

### Example 7

### (1) Construction of pCfCB101, pCfCC52, pCfCC59, pCfCD28 and pCfCD56 (cf. Fig. 8)

First, 3 µg of pBR322 [Bolivar et al.: Gene, 2, 95 (1977)] were dissolved in 40 µl of Y-100 buffer, 5 units of the restriction enzyme PstI were added, and the digestion reaction was conducted at 37°C for 1 hour. The DNA recovered by phenol-chloroform extraction and ethanol precipitation was dissolved in 20 µl of a solution containing 33 mM Tris-acetic acid (pH 7.9), 66 mM potassium acetate, 10 mM magnesium acetate, 5 mM dithiothreitol, and dATP, dCTP, dGTP and dTTP (each 0.4 mM) (hereinafter referred to as "T4 DNA polymerase buffer"), 1 unit of T4 DNA polymerase (Takara Shuzo) was added, and the reaction was carried at 37°C for 30 minutes. The DNA recovered by phenol-chloroform extraction and ethanol precipitation was dissolved in 20 µl of T4 DNA ligase buffer. To this were added about 8 picomoles of the BglII linker DNA [Takara Shuzo; d(pC-A-G-A-T-C-T-G)].After further addition of 6 units of T4 DNA ligase, the ligation reaction was performed at 4°C for 18 hours. The DNA recovered by phenol-chloroform extraction and ethanol precipitation was dissolved in 40 µl of Y-100 buffer, 10 units of the restriction enzyme EcoRI and 8 units of BglII were added, and the digestion reaction was conducted at 37°C for 1 hour. From the reaction mixture, there were obtained, by the LGT method, about 0.9 µg of a tetracycline-resistance gene portion-containing DNA fragment of about 3.6 kb (EcoRI-BglII fragment).

Separately, 3 µg of pCfBB101 obtained in Example 6-(2), pCfBC52 or pCfBC59 obtained in Example 6-(3) or pCfBD28 or pCfBD56 obtained in Example 6-(4) were dissolved in 10-fold concentrated Y-100 buffer, 5 units of the restriction enzyme EcoRI and 6 units of BglII were added, and the digestion reaction was performed at 37°C for 1 hour. From the reaction mixture, there were obtained, by the LGT method, about 0.4 µg of an hG-CSF cDNA portion-containing DNA fragment of about 1.8 kb (EcoRI-BglII fragment) in each case.

Five tubes each containing a solution of about 0.05 µg of the pBR322-derived EcoRI-BglII fragment (about 3.6 kb) obtained as described above in 20 µl of T4 DNA ligase buffer were prepared. To the tubes were added about 0.1 µg of the pCfBB101-, pCfBC52-, pCfBC59-, pCfBD28- or pCfBD56-derived EcoRI-BglII fragment (about 1.8 kb fragment) and, following further addition of 4 units of T4 DNA ligase, the ligation reaction was conducted at 4°C for 18 hours.

The recombinant plasmid mixtures obtained were used to transform E. coli HB101, and Tc^{r} colonies were obtained. From cultured cells of each of these colonies, the plasmid DNA was recovered. Thus, were obtained pCfCB101, pCfCC52, pCfCC59, pCfCD52 and pCfCD56, each shown in Fig. 8. The amino acid sequences of the hG-CSF derivatives encoded by these plasmids are identical with the amino acids of the hG-CSF derivatives encoded by pCfBB101, pCfBC52, pCfBC59, pCfBD28 and pCfBD56, respectively.

### Example 8

### Production and purification of hG-CSF derivatives

E. coli W3110 strA-derived transformants (called ECfTL23, ECfTL35, ECfTL38, ECfTL41, ECfTM14, ECfTM17, ECfTM113, ECfBB101, ECfBC42B1, ECfBC45, ECfBC52, ECfBC59, ECfBC76, ECfBC77, ECfBC93, ECfBC95, ECfBC97, ECfBD28, ECfBD56, ECfBD82, ECfTNS7, ECfTAArg4S, ECfTNS301, ECfTNS401, ECfTNS501, ECfBD28A17 and ECfBD28T17) harboring the recombinant plasmids (obtained in Examples 3, 4, 6 and 7) pCfTL23, pCfTL35, pCfTL38, pCfTL41, pCfTM14, pCfTM17, pCfTM113, pCfBB101, pCfBC42B1, pCfBC45, pCfBC52, pCfBC59, pCfBC76, pCfBC77, pCfBC93, pCfBC95, pCfBC97, pCfBD28, pCfBD56, pCfBD82, pCfTNS7, pCfAArg4S, pCfTNS301, pCfTN401, pCfTNS501, pCfBD28T17 and pCfBD28A17, respectively were each cultivated in LG medium (prepared by dissolving 10 g of Bactotryptone, 5 g of yeast extract, 5 g of NaCI and 1 g of glucose in 1 liter of water and adjusting the pH to 7.0 with NaOH) at 37°C for 18 hours. A 5-ml portion of the culture broth was inoculated into 100 ml of MCG medium (0.6% Na₂HPO₄, 0.3% KH₂PO₄, 0.5% NaCI, 0.5% casamino acids, 1 mM MgSO₄, 4 µg/ml vitamin B₁, pH 7.2) containing 25 µg/ml tryptophan and 50 µg/ml ampicillin. After incubation at 30°C for 4-8 hours, 10 µg/ml 3β-indoleacrylic acid (hereinafter IAA), a tryptophan promoter inducer, was added, and incubation was continued for further 2-12 hours. Cells were harvested by subjecting the culture broth to centrifugation at 8,000 rpm for 10 minutes, and washed with 30 mM NaCl-30 mM Tris-HCI buffer (pH 7.5). The washed cells were suspended in 30 ml of the above-mentioned buffer and then disrupted by ultra-sonication at 0°C (BRANSON SONIC POWER COMPANY'S SONIFIER CELL DISRUPTOR 200, Output Control 2, 10 minutes). Centrifugation at 9,000 rpm for 30 minutes gave a cell residue mass. Using the method of Marston et al. [F. A. O. Marston et al.: BIO/TECHNOLOGY, 2, 800 (1984)], the hG-CSF derivative was extracted from the cell residue mass, purified, solubilized and renatured. The protein quantity was determined by using Nippon Bio-Rad laboratories' protein assay kit (standard assay method) (M. M. Bradford: Anal. Biochem., 72, 248 (1976)].

The G-CSF activity was determined in the following manner. Bone marrow cells were aseptically collected from the femur of male C3H/He mice of 8-12 weeks of age (Shizuoka Laboratory Animal Center) and suspended in α-MEM supplemented with 10% fetal bovine serum (FBS). Nylon wool (Wako Pure Chemical Industries; Nylon Fiber 146-04231) (0.3 g) packed in a column was impregnated with 1.5 ml of this cell suspension (about 5 x 10⁷ cells), and the reaction was allowed to proceed in a 5% CO₂ incubator at 37°C for 90 minutes. Then, α-MEM warmed to 37°C in advance was passed through the column, and bone marrow cells unadsorbed on the nylon wool were obtained as an effluent fraction. These cells were washed once with α-MEM and adjusted to a predetermined concentration.

Then, the myelopoietic stem cell colony forming activity was determined by the method of Okabe et al. [Okabe, T. et al: Cancer Research, 44, 4503-4506 (1986)]. Thus, 0.2 ml of the marrow cells suspension prepared as above (2 x 10⁶ cells/ml) were admixed with a mixture of 0.2 ml of α-MEM, 0.4 ml of FBS and 0.2 ml of each 2-diluted sample. An equal volume (1.0 ml) of 0.6% agar (Difco, Agar purified #0560-01) warmed to 42°C was admixed with the mixture, and the whole mixture was distributed in 0.5-ml portions into a well of a 24-well plate Nunc's Multidish #143982) (5 x 10⁴ cells/dish, n =3). After incubation in a 5% CO₂ incubator at 37°C for 7 days, colonies each comprising not less than 40 cells were counted under a microscope (Olympus X40). After colony counting, cells were taken out onto a slide glass with care and fixed with an acetone-formalin mixed solution for 30 seconds. After esterase double stain of the cells by the method of Kubota et al. [Kubota, K. et al.: Exp. Hematology, 8, 339-344 (1980)], each colony was identified.

The potency of each sample was calculated on the basis of the result of counting for the 2-fold dilution in the colony formation assay, as follows. The activity which gives half of the maximum colony formation value for intact G-CSF used as the standard was defined as 50 units. The potency (in units) was calculated by multiplying by 20, inclusive of the dilution factor for each sample, for conversion to the activity per milliliter. The specific activity was expressed in terms of potency per unit weight (mg) of protein, hence in units/mg.

The potencies of intact G-CSF and G-CSF derivatives are shown in Table 3.

**Table 3**

| Strain | Plasmid borne | Plasmid-encoded product | Specific activity (units/mg) | Specific activity ratio |
|---|---|---|---|---|
| | | | | |
| ECfTA1 | pCfTA1 | G-CSF(intact) | 2.2×10⁸ | 1.0 |
| ECfTL38 | pCfTL38 | G-CSF(Ser¹) | 4.0×10⁸ | 1.8 |
| ECfTL41 | pCfTL41 | G-CSF(Arg¹) | 3.7×10⁸ | 1.7 |
| ECfTL23 | pCfTL23 | G-CSF(Gly¹) | 3.1×10⁸ | 1.4 |
| ECfTL35 | pCfTL35 | G-CSF(Cys¹) | 2.9×10⁸ | 1.3 |
| ECfBB101 | pCfBB101 | G-CSF(NB101) | 7.9×10⁸ | 3.6 |
| ECfBC42B1 | pCfBC42B1 | G-CSF(NC42B1) | 5.1×10⁸ | 2.3 |
| ECfBC45 | pCfBC45 | G-CSF(NC45) | 7.0×10⁸ | 3.2 |
| ECfBC52 | pCfBC52 | G-CSF(NC52) | 6.2×10⁸ | 2.8 |
| ECfBC59 | pCfBC59 | G-CSF(NC59) | 5.9×10⁸ | 2.7 |
| ECfBC76 | pCfBC76 | G-CSF(NC76) | 6.2×10⁸ | 2.8 |
| ECfBC77 | pCfBC77 | G-CSF(NC77) | 7.7×10⁸ | 3.5 |
| ECfBC93 | pCfBC93 | G-CSF(NC93) | 9.2×10⁸ | 4.2 |
| ECfBC95 | pCfBC95 | G-CSF(NC95) | 9.5×10⁸ | 4.3 |
| ECfBC97 | pCfBC97 | G-CSF(NC97) | 8.6×10⁸ | 3.9 |
| ECfBD28 | pCfBD28 | G-CSF(ND28) | 7.9×10⁸ | 3.6 |
| ECfBD56 | pCfBD56 | G-CSF(ND56) | 5.1×10⁸ | 2.3 |
| ECfBD82 | pCfBD82 | G-CSF(ND82) | 4.6×10⁸ | 2.1 |
| ECfTM14 | pCfTM14 | G-CSF(Ser¹,Cys³) | 3.1×10⁸ | 1.4 |
| ECfTM17 | pCfTM17 | G-CSF(Ser¹,Arg³) | 3.7×10⁸ | 1.7 |
| ECfTM113 | pCfTM113 | G-CSF(Ser¹,Ser³) | 2.9×10⁸ | 1.3 |
| ECfTNS7 | pCfTNS7 | G-CSF(Δ1-4S) | 7.7×10⁸ | 3.5 |
| ECfTAArg4S | pCfTAArg4S | G-CSF(Arg⁴,Ser¹⁷) | 5.7×10⁸ | 2.6 |
| ECfTNS301 | pCfTNS301 | G-CSF(Δ1-11S) | 3.1×10⁸ | 1.4 |
| ECfTNS401 | pCfTNS401 | G-CSF(Δ1-7S) | 5.5×10⁸ | 2.5 |
| ECfTNS501 | pCfTNS501 | G-CSF(Δ1-6S) | 4.4×10⁸ | 2.0 |
| ECfBD28A17 | pCfBD28A17 | G-CSF(ND28A17) | 6.8×10⁸ | 3.1 |
| ECfBD28T17 | pCfBD28T17 | G-CSF(ND28T17) | 5.9×10⁸ | 2.7 |
| ECfTN205 | pCfTN205 | G-CSF(Δ1-4) | 4.2×10⁸ | 1.9 |

### Example 9

### Measurement of activities of hG-CSF derivatives against human bone marrow cells

The marrow fluid was collected from the iliac bone of normal humans of 20-30 years age. An equal volume of α-MEM was added to and mixed with the marrow fluid. A 4-ml of the above bone marrow fluid was layered onto 3 ml of Ficoll-Paque solution (Pharmacia Fine Chemicals, specific gravity 1.077) and, after centrifugation at 400 x g for 30 minutes, the cells occurring in the intermediate layer were separated. The cells were washed twice with PBS (prepared by dissolving 8 g of NaCI, 0.2 g of KCI, 1.15 g of Na₂HPO₄ and 0.2 g of KH₂PO₄ in water to make 1 liter of solution) and then suspended in α-MEM supplemented with 10% of fetal bovine serum (FBS), and the cell concentration was adjusted to at most 2 x 10⁶ cells /ml. A 10-ml portion of the cell suspension was placed in a plastic dish (Falcon 3003) and incubated in a 5% CO₂ incubator at 37°C for 90 minutes. Cells unadsorbed in the plastic dish were recovered, washed once with α-MEM and, after adjustment of the concentration to a predetermined level, subjected to human myelopoietic stem cell growth promoting activity and colony formation tests. Thus, 10% FBS-supplemented α-MEM was distributed in 100-µl portions into wells of a 96-well flat microplate (NUNC, 167008). Then, samples of the hG-CSF and hG-CSF derivatives obtained by the method of Example 8 were added in 100-µl portions into wells of the first row. After thorough mixing, 100 µl of each mixture were transferred to a well of the second row for preparing a 2-fold dilution. Doubling dilution was continued in the same manner until the 12th row (n = 3). In a group, α-MEM alone was used as a negative control.

Then, 100 µl (5 x 10⁴ eukaryotic cells) of the bone marrow cell suspension prepared as described above were sowed into each well. Incubation was carried out in a 5% CO₂ incubator at 37°C for 3 days. During the 20-hour period preceding the last 18 hours, 10 µl of 6-³H-thymidine (Amersham Japan, code TRK61, 107 mci/mg) were added. Cells were recovered onto a glass filter using a cell harvester(Labo-Science), dried, and measured for the radioactivity taken up by the cells using a liquid scintillation counter (Packard, Tricarb 3320).

On the other hand, the human myelopoietic stem cell colony formation assay and colony identification were perfomed as described in Example 8.

For calculating the potency of each sample, the activity capable of causing formation of one colony was defined as 1 unit. Thus, the Half Max value (half of the maximum take-up value) was determined based on the dose-response curve showing linearity for the results of counting in the doubling dilution series, and the potency of each sample was calculated.

The specific activity was expressed in terms of potency per unit weight (mg) of protein, i.e., in units/mg,

The potencies of the intact hG-CSF and hG-CSF derivatives are shown in Table 4.

**Table 4**

| Strain | Plasmid borne | Plasmid-encoded product | Specific activity (units/mg) | Specific activity ratio |
|---|---|---|---|---|
| ECfTA1 | pCfTA1 | G-CSF(intact) | 2.8 x 10⁸ | 1.0 |
| ECfBC59 | pCfBC59 | G-CSF(NC59) | 7.7 x 10⁸ | 2.8 |
| ECfBC93 | pCfBC93 | G-CSF(NC93) | 7.0 x 10⁸ | 2.5 |
| ECfBC95 | pCfBC95 | G-CSF(NC95) | 9.5 x 10⁸ | 3.4 |
| ECfBD28 | pCfBD28 | G-CSF(ND28) | 10.4 x 10⁸ | 3.7 |
| ECfTAArg4 | pCfTAArg4 | G-CSR(Arg4) | 5.3 x 10⁸ | 1.9 |
| ECfTNS501 | pCfTNS501 | G-CSF(Δ1-6S) | 6.2 x 10⁸ | 2.2 |

### Example 10

### Production of hG-CSF derivative lacking the N-terminal 1st to 7th amino acids and having serine as the 17th amino acid (hereinafter referred to as M-7S)

To 50 ml of 10 mM Tris-HCI-100 mM NaCI solution (pH 8.0) containing the derivative (a) shown in Table 2 (132 µg/ml) as obtained by cultivating the E. coli strain (ECfBC59) carrying the recombinant plasmid pCfBC59 obtained in Example 6, followed by purification by the procedure of Example 8, there were added 0.7 µg of subtilisin BPN' (8.5 units/mg protein) (Sigma), and incubation was performed at 25°C for 40 hours. After 3-fold dilution with 10 mM Tris-HCI (pH 8.0), the incubation mixture was applied to a DEAE-Toyopearl 650M (Toyo Soda Manufacturing) column (1.7 cm x 4.4 cm) filled with 10 mM Tris-HCI (pH 8.0) at a flow rate of 10 ml/hour. Then, 20 ml of 10 mM Tris-HCI (pH 8.0) were passed through the column at a flow rate of 5 ml/hour. Thereafter, elution was conducted with a buffer system of 10 mM Tris-HCI (pH 8.0) showing a linear NaCI concentration gradient from 0 M to 0.4 M at the same flow rate (total eluent volume 50 ml). The M-7S derivative was eluted at NaCI concentrations of 100-150 mM (yield 0.7 mg, or 10%). The purity was not less than 90%.

### Example 11

### Production of M-7S

To 50 ml of a 10 mM Tris-HCI-100 mM NaCI solution (pH 8.0) containing the derivative (b) shown in Table 2 (132 µg/ml) as obtained by cultivatin the E. coli strain (ECfBC59) carrying the recombinant plasmid pCfBC59 obtained in Example 6, followed by purification by the procedure of Example 8, there were added 0.7 µg of subtilisin BPN' (8.5 units/mg protein) (Sigma), and incubation was performed at 25°C for 14 hours. After 3-fold dilution with 10 mM Tris-HCI (pH 8.0), the incubation mixture was applied to DEAE-Toyopearl 650M (Toyo Soda Manufacturing) column (1.7 cm x 4.4 cm) filled with 10 mM Tris-HCI (pH 8.0) at a flow rate of 10 ml/hour. Then, 20 ml of 10 mM Tris-HCI (pH 8.0) were passed through the column at a flow rate of 5 ml/hour. Thereafter, elution was conducted with a buffer system of 10 mM Tris-HCI (pH 8,0) showing a linear NaCI concentration gradient from 0 M to 0.4 M at the same flow rate (total eluent volume 50 ml). The M-7S derivative was eluted at NaCI concentrations of 100-150 mM (yield 4.2 mg, or 63%). The purity was not less than 90%.

### Example 12

### Production of M-7S

To 50 ml of a 10 mM Tris-HCI-100 mM NaCI solution (pH 8.0) containing the derivative (d) shown in Table 2 (132 µg/ml) as obtained by cultivating the E. coli strain (ECfTAArg4S) carrying the recombinant plasmid pCfTAArg4S obtained in Example 6, followed by purification by the procedure of Example 8, there were added 0.7 µg of subtilisin BPN' (8.5 units/mg protein) (Sigma), and incubation was performed at 25°C for 40 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of 10 mM Tris-HCI-0.5 M NaCI buffer on a linear pH gradient of from 8.0 to 6.0 at the same rate of flow. The M-7S derivative was eluted in the neighborhood of pH 7.0 (yield 0.6 mg, or 9%). The purity was 90%.

### Example 13

### Production of hG-CSF derivative lacking the N-terminal 1st to 6th amino acids and having serine as the 17th amino acid (hereinafter referred to as M-6S)

To 50 ml of a solution of the derivative (a) shown in Table 2 (132 µg/ml) in 10 mM Tris-HCI-100 mM NaCI (pH 8.0), there were added 0.7 µg of subtilisin BPN' (8.5 units/mg protein) (Sigma), and incubation was carried out at 25°C for 2 hours. After 3-fold dilution with 10 mM Tris-HCI (pH 8.0), the incubation mixture was applied to a DEAE-Toyopearl 650M (Toyo Soda Manufacturing) column (1.7 cm x 4.4 cm) filled with 10 mM Tris-HCI (pH 8.0) at a flow rate of 10 ml/hour. Then, 20 ml of 10 mM Tris-HCI (pH 8.0) were passed through the column at a flow rate of 5 ml/hour. Thereafter, elution was carried out with a total volume of 50 ml of a buffer system of 10 mM Tris-HCI (pH 8.0) showing a linear NaCI concentration gradient from 0 M to 0.4 M at the same rate of flow. The M-6S derivative was eluted at NaCI concentrations of 100-150 mM (yield 2.6 mg, or 40%). The purity was not less than 90%.

### Example 14

### Production of M-6S

To 50 ml of a 10 mM Tris-HCI-100 mM NaCI solution (pH 8.0) containing the derivative (a) shown in Table 2 (132 µg/ml), there were added 0.7 µg of Epolozyme (4120 units/mg protein) (Kyowa Hakko Kogyo), and incubation was carried out at 25°C for 20 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of 10 mM Tris-HCI-0.5 M NaCI buffer on a linear pH gradient of from 8.0 to 6.0 at the same rate of flow. The M-6S derivative was eluted in the neighborhood of pH 7.0 (yield 2.5 mg, or 38%). The purity was not less than 90%.

### Example 15

### Production of M-6S

To 50 ml of a 10 mM Tris-HCI-100 mM NaCI solution (pH 8.0) containing the derivative (b) shown in Table 2 (132 µg/ml), there were added 0.7 µg of subtilisin amylosacchalyticus, and incubation was carried out at 25°C for 20 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of 10 mM Tris-HCI (pH 8.0)-0.5 M NaCI buffer on a linear pH gradient of from 8.0 to 6.0 at the same rate of flow. The M-6S derivative was eluted in the neighborhood of pH 7.0 (yield 2.5 mg, or 38%). The purity was not less than 90%.

### Example 16

### Production of M-6S

To 50 ml of a 10 mM Tris-HCI-100 mM NaCI solution (pH 8.0) containing the derivative (d) shown in Table 2 (132 µg/ml), there were added 0.7 µg of subtilisin Carlsberg (0.034 unit/mg protein) (NOVO), and incubation was carried out at 25°C for 20 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0,5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of a buffer system of 10 mM Tris-HCl-0.5 M NaCl showing a linear pH gradient of from 8.0 to 6.0 at the same rate of flow. The M-6S derivative was eluted in the neighborhood of pH 7.0 (yield 3 mg, or 45%). The purity was not less than 90%.

### Example 17

### Production of M-6S

To 50 ml of a 10 mM Tris-HCI-100 mM NaCI solution (pH 8.0) containing the derivative (a) shown in Table 2 (132 µg/ml), there were added 0.7 µg of proteinase K (0.027 unit/mg protein) (Sigma), and incubation was carried out at 25°C for 40 hours. After 3-fold dilution with 10 mM Tris-HCI (pH 8.0), the incubation mixture was applied to a DEAE-Toyopearl 650M (Toyo Soda Manufacturing) column (1.7 cm x 4.4 cm) filled with 10 mM Tris-HCI (pH 8.0) at a flow rate of 10 ml/hour. Then, 20 ml of 10 mM Tris-HCI (pH 8.0) were passed through the column at a flow rate of 5 ml/hour. Thereafter, elution was carried out with a total volume of 50 ml of a buffer system of 10 mM Tris-HCI (pH 8.0) showing a linear NaCI concentration gradient of from 0 M to 0.4 M at the same rate of flow. The M-6S derivative was eluted at NaCI concentrations of 100-150 mM (yield 2.6 mg, or 39%). The purity was not less than 90%.

### Example 18

### Production of hG-CSF derivative lacking the N-terminal 1st to 5th amino acids and having serine as the 17th amino acid (hereinafter referred to as M-5S)

To 50 ml of a 10 mM Tris-HCI-100 mM NaCI solution (pH 8.0) containing the derivative (b) shown in Table 2 (132 µg/ml), there were added 0.5 µg of trypsin (267 units/mg protein) (Sigma), and incubation was carried out at 25°C for 10 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of a buffer system of 10 mM Tris-HCI (pH 8.0)-0.5 M NaCI showing a linear imidazole concentration gradient of from 0 M to 0.3 M at the same rate of flow. The M-5S derivative was eluted at 0.1 M imidazole (yield 2.7 mg, or 41%). The purity was not less than 90%.

### Example 19

### Production of hG-CSF derivative lacking the N-terminal 1st to 4th amino acids and having serine as the 17th amino acid (hereinafter referred to as M-4S)

To 50 ml of a 10 mM Tris-HCI-100 mM NaCI solution (pH 8.0) containing the derivative (d) shown in Table 2 (132 µg/ml), there were added 5 µg of trypsin (267 units/mg protein) (Sigma), and incubation was carried out at 25°C for 20 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total volume of 30 ml of a buffer system of 10 mM Tris-HCI (pH 8.0)-0.5 M NaCI showing a linear imidazole concentration gradient of from 0 M to 0.3 M at the same rate of flow. The M-4S derivative was eluted at 0.1 M imidazole (yield 2.7 mg, or 41%). The purity was not less than 90%.

### Example 20

### Production of M-4S

To 50 ml of a 10 mM Tris-HCI-100 mM NaCI solution (pH 8.0) containing the derivative (d) shown in Table 2 (132 µg/ml), there were added 5 µg of α-chymotrypsin (267 units/mg protein)·(Sigma), and incubation was carried out at 25°C for 20 hours. After adjustment of the NaCI concentration to 0.5 M, the incubation mixture was applied to a Zn chelate-Sepharose (Pharmacia Fine Chemicals) column (1.7 cm x 2.6 cm) filled with 10 mM Tris-HCI (pH 8.0)-0.5 M NaCI at a flow rate of 6 ml/hour. Then, 12 ml of 10 mM Tris-HCI (pH 8.0)-0.5 M NaCI were passed through the column at a flow rate of 3 ml/hour. Thereafter, elution was carried out with a total of 30 ml of a buffer system of 10 mM Tris-HCI (pH 8.0)-0.5 M NaCI showing a linear imidazole concentration gradient of from 0 M to 0.3 M at the same rate of flow. The M-4S derivative was eluted at 0.1 M imidazole (yield 2.3 mg, or 35%). The purity was not less than 90%.

### Example 21

As seen in Examples 10-20 there can be obtained those hG-CSF derivatives which have serine as a substitute for the 17th amino acid and are lacking in 4 (M-4S), 5 (M-5S), 6 (M-6S) and 7 (M-7S)N-terminal amino acids. The use of recombinant DNA technology generally results in addition of methionine to the N terminus, and this is one of the disadvantageous features of recombinant products. On the contrary, the use of the enzymatic cleavage technique according to the invention is advantageous since such products can be produced without addition of methionine to the N terminus.

The derivatives obtained in this manner were assayed for G-CSF activity for comparison. The results obtained are shown in Table 5.

**Table 5**

| Activity comparison among G-CSF derivatives formed by the enzymatic cleavage technique | |
|---|---|
| hG-CSF Derivative | Relative Activity (derivative/intact) |
| Intact | 1.0 |
| M-4S | 4.0 |
| M-5S | 3.5 |
| M-6S | 3.0 |
| M-7S | 3.3 |

From the results shown in Table 5, it was found that, in the above in vitro evaluation, the derivatives lacking in 4-7 N-terminal side amino acids have a 2- to 4-fold higher activity as compared with the intact hG-CSF.

Therefore, the derivatives lacking in N-terminal side amino acids that can be produced in accordance with the present invention have no methionine added to the N terminus and are 2- to 4-fold higher in activity than the intact product.

The following examples illustrate the acquisition of reactivity (susceptibility) to cleavage by hydrolytic enzymes as a result of mutation in the N-terminal portion.

### Test Example 1

### Comparison in reactivity with subtilisin Carlsberg between intact hG-CSF and N-terminal mutants of hG-CSF

The derivatives shown in Table 2 and intact hG-CSF were each incubated in the presence of 3.6 x 10⁻⁴ units of subtilisin Carlsberg (NOVO)/mg G-CSF at 25°C for 14 hours in the same manner as in Example 16. While the derivatives shown in Table 2 gave the M-6S derivative, the intact hG-CSF remained unreacted. Furthermore, even when this enzyme was used in a 100-fold increased amount (3.6 x 10⁻² units/mg G-CSF), the intact product failed to produce M-6S but preferentially underwent global decomposition reactions.

### Test Example 2

### Comparison in reactivity with trypsin between intact hG-CSF and N-terminal mutants of hG-CSF

The derivative (a) shown in Table 2 and intact hG-CSF were each incubated in the presence of 0.22 units of trypsin (Sigma)/mg G-CSF at 25°C for 20 hours in the same manner as in Example 19. While the derivative (a) shown in Table 2 gave the M-4S derivative, the intact hG-CSF remained unreacted.
Furthermore, even when the enzyme was in a 100-fold increased amount (22 units/mg G-CSF), the intact hG-CSF did not give M-4S but preferentially underwent global decomposition reactions.

### Test Example 3

### Heat stability of hG-CSF derivatives

A 20-µg portion of each of the various derivatives, shown in Table 6, of the invention was dissolved in 1 ml of phosphate-buffered physiological saline (PBS) (pH 7.2) or α-MEM supplemented with 10% fetal bovine serum (FBS). Incubation was carried out at 56°C, and samples were collected at timed intervals and assayed for CSF activity by colony formation testing using mouse bone marrow cells (the above-mentioned method of Okabe et al.).

Each sample was diluted by the doubling dilution technique from 40 ng/ml to give 10 dilution levels. For each level, activity assay was performed, and the residual activity was determined by comparing the activity at a certain concentration at which good dose-response with that before heating (0 minute).

The residual activity (corresponding to thermal stability) data obtained in PBS and in 10% FBS-supplemented α-MEM are shown in Table 6(A) and Table 6(B), respectively.

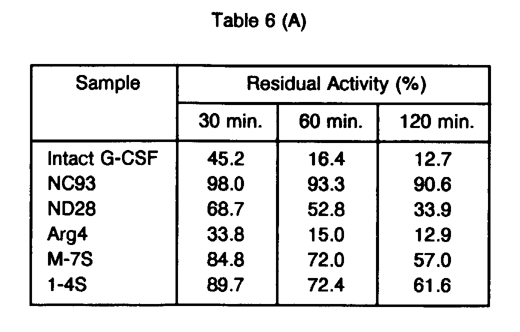

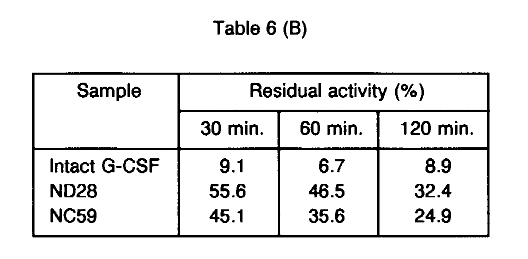

### Example 22

### Construction of pCfBD28A17 and pCfBD28T17 using site-directed mutagenesis (cf. Fig. 17)

### (a) Construction of single-stranded template DNA (single-stranded pt19BD28N)

In 50 µl of Y-100 buffer, there were dissolved 3 µg of pCfBD28 obtained by the procedure of Example 6-(4), 10 units each of the restriction enzymes BanIII (Toyobo) and PstI were added, and the cleavage reaction was carried out at 37°C for 2 hours. From the reaction mixture, there were obtained by the LGT method, about 0.1 µg of an about 210 bp DNA fragment (BanIII-PstI fragment) coding for the N-terminal portion of the hG-CSF derivative (ND28).

Separately, 1 µg of the M13 phage vector M13mp19RF DNA (Takara Shuzo) was dissolved in a total of 50 µl of Y-50 buffer, 10 units of the restriction enzyme AccI (Toyobo) were added, and the cleavage reaction was carried out at 37°C for 2 hours. Thereafter, NaCI was added to an NaCI concentration of 100 mM, 10 units of the restriction enzyme PstI were added, and the cleavage reaction was conducted at 37°C for 2 hours. From the reaction mixture, there was obtained, by the LGT method, about 0.8 µg of a DNA fragment of about 7.24 kb (AccI-PstI fragment).

In 50 µl of T4 DNA ligase buffer, there were dissolved 0.2 µg of the BanIII-PstI fragment (about 210 bp) and 0.05 µg of the AccI-PstI fragment (about 7.24 kb), each obtained as described above. T4 DNA ligase (10 units) was added to the mixture solution, and the ligation reaction was performed at 12°C for 16 hours.

Then, the above reaction mixture was used to transfect E. coli JM105 by a known method. Thus was obtained a recombinant phage. From cultured cells of an E. coli JM105-derived transformant infected with the recombinant phage, there was recovered the recombinant M13 phage RF DNA. The structure of this RF DNA (hereinafter referred to as pt19BD28N) was confirmed by cleavage with PstI, EcoRI, AvaI and XhoI followed by polyacrylamide gel electrophoresis. Then, the single-stranded pt19BD28N was recovered from the recombinant phage by a known method and used as an template.

### (b) Construction of gapped duplex DNA

In 30 µl of Y-100 buffer, there were dissolved 3 µg of the M13mp19 RF DNA (Takara Shuzo), 10 units each of the restriction enzymes EcoRI and HindIII were added, and the cleavage reaction was carried out at 37°C for 2 hours. From the reaction mixture, there were obtained, by the LGT method, about 2.5 µg of a DNA fragment of about 7.2 kb (EcoRI-HindIII fragment).

This M13mp19 RF DNA-derived EcoRI-HindIII fragment (about 7.2 kb) and 1 µg of the single-stranded template DNA pt19BD28N obtained as described in the preceding section were dissolved in 27 µl of Klenow buffer, and DNA denaturation was caused by boiling at 100°C for 6 minutes. Thereafter, the mixture was allowed to stand at 65°C for 10 minutes, at 37°C for 40 minutes, at 4°C for 40 minutes and in ice for 10 minutes to cause the annealing reaction to proceed, whereby a gapped duplex DNA was formed in which the G-CSF gene portion alone in the template was single-stranded. The thus-formed gapped duplex DNA was recovered by the LGT method.

### (c) Mutagenesis (construction of pt19BD28NA17 and pt19BD28NT17)

A single-stranded DNA (D-1) required for substituting Ala for the 17th amino acid (from the N terminus), namely Ser, of the hG-CSF derivative [ND28] obtained in Example 6 and a single-stranded DNA (D-2) required for substituting Thr for the Ser were synthesized by the ordinary phosphotriester method. The base sequences of D-1 (33-mer) and D-2 (33-mer) are shown below:

D-1

Ala
↑

12 13 14 15 16 17 18 19 20 21 22
17 Ser Phe Leu Leu Lys Ser Leu Glu Gln Val Arg
(Ser→Ala) 5'-AGC TTC CTT TTA AAG GCC TTA GAG CAA GTG AGG
StuI
-3' (33 mer)

D-2
Thr
↑
12 13 14 15 16 17 18 19 20 21 22
17 Ser Phe Leu Leu Lys Ser Leu Glu Gln Val Arg
(Ser→Thr) 5'-AGC TTC CTT TTA AAA ACT CTA GAG CAA GTG AGG
XbaI -3' (33 mer)

The designs of the above DNAs are such that mutagenesis using D-1 can cause formation of a new StuI site and mutagenesis using D-2 can give rise to a new XbaI site. Therefore, mutants can be identified by cleavage with these restriction enzymes.

D-1 and D-2 were each individually dissolved, in an amount of 1 µg, in 50 µl of T4 kinase buffer, 30 units of T4 polynucleotide kinase were added, and the phosphorylation reaction was carried out at 37°C for 60 minutes.

Then, 0.2 µg of the phosphorylated D-1 or D-2 and 0.1 µg of the gapped duplex DNA obtained as described in the opening section were dissolved in 34 µl of buffer containing 6.5 mM Tris-HCI (pH 7.5), 8 mM MgCl₂, 1 mM 2-mercaptoethanol and 100 mM NaCI, the solution was allowed to stand at 65°C for 60 minutes and then at room temperature for 30 minutes, whereby D-1 or D-2 was annealed with the gapped duplex DNA.

To the solution were added dATP, dTTP, dCTP and dGTP each to a concentration of 0.5 mM. Following further addition of 1.5 units of DNA polymerase I Klenow fragment and 10 units of T4 DNA ligase, the extension reaction was carried out at 4°C for 16 hours.

The reaction mixtures thus obtained were used to transfect E. coli JM105, and mutant phages were obtained. The RF DNAs were recovered from the mutant phage-infected E. coli JM105 transformants and identified by cleavage with AvaI, XhoI and StuI (when D-1 was used) or with XbaI (when D-2 was used), followed by polyacrylamide gel electrophoresis. The RF DNA with mutation introduced therein by means of D-1 is named pt19BD28NA17 and the RF DNA with mutation introduced therein by means of D-2 is named pt19BD28NT17. The base sequences of pt19BD28NA17 and pt19BD28NT17 in the vicinity of the StuI site and XbaI site, respectively, were confirmed by the dideoxy sequencing method using M13 phage to be as follows:

pt19BD28NA17
12 13 14 15 16 17 18 19 20 21 22
Ser Phe Leu Leu Lys Ala Leu Glu Gln Val Arg
AGC TTC CTT TTA AAG GCC TTA GAG CAA GTG AGG
StuI

pt19BD28NT17
12 13 14 15 16 17 18 19 20 21 22
Ser Phe Leu Leu Lys Thr Leu Glu Gln Val Arg
AGC TTC CTT TTA AAA ACT CTA GAG CAA GTG AGG
XbaI

### (d) Construction of pCfBD28A17 and pCfBD28T17

In 50 µl of Y-100 buffer, there were dissolved 3 µg of pt19BD28NA17 or pt19BD28NT17 obtained as described above, 10 units each of the restriction enzymes AvaI and XhoI were added, and the cleavage reaction was conducted at 37°C for 2 hours. From the reaction mixture, there were obtained, by the LGT method, 0.05 µg of an about 110 bp DNA fragment containing the site of mutation introduced as described in the preceding section (AvaI-XhoI fragment).

Separately, 2 µg of pCfBD28 obtained in Example 6-(4) was dissolved in 50 µl of Y-100 buffer, 10 units each of the restriction enzymes XhoI and BglII were added, and the cleavage reaction was performed at 37°C for 2 hours. From the reaction mixture, there was obtained, by the LGT method, about 1 µg of a tryptophan promoter portion-containing DNA fragment of about 2.74 kb (XhoI-BglII fragment).

Further, separately, 2 µg of pCfBD28 were dissolved in 50 µl of Y-100 buffer, 10 units of the restriction enzyme BglII were added, and the cleavage reaction was carried out at 37°C for 2 hours. After confirmation of the completeness of the BglII cleavage by agarose gel electrophoresis, 5 units of the restriction enzyme AvaI were added, and partial cleavage was effected at 37°C for 10 minutes. From the reaction mixture, there were obtained, by the LGT method, 0.4 µg of an about 1.29 kb DNA fragment (BglII-AvaI fragment) containing most of the mature hG-CSF cDNA with the ℓpp terminator portion.

Then, 0.1 µg of the pCfBD28-derived XhoI-BglII fragment (about 2.74 kb), 0.05 µg of the pCfBD28-derived BglII-AvaI fragment (about 1.29 kb) and 0.02 µg of the pt19BD28NA17- or pt19BD28NT17-derived AvaI-XhoI fragment (about 110 bp) were dissolved in 60 µl of T4 DNA ligase buffer, 10 units of T4 DNA ligase were added, and the ligation reaction was conducted at 12°C for 16 hours.

The reaction mixture thus obtained was used to transform E. coli HB101, and an Ap^{r} colony was obtained. The plasmid DNA was recovered from cultured cells of this colony. The plasmid constructed by using pt19BD28NA17 is named pCfBD28A17 and that constructed by using pt19BD28NT17 is named pCfBD28T17. The structure of pCfBD28A17 was confirmed by cleavage with AvaI, XhoI, BglII and StuI, followed by agarose gel electrophoresis. The structure of pCfBD28T17 was confirmed by cleavage with AvaI, XhoI, BglII and XbaI, followed by agarose gel electrophoresis.

The replacing amino acid residues in the hG-CSF derivatives encoded by these two plasmids as compared with mature hG-CSF are as follows:

| Position of amino acid substitution (amino acid of hG-CSF) | Plasmid | |
|---|---|---|
| | pCfBD28A17 | pCfBD28T17 |
| 1st (Thr) | Ala | Ala |
| 3rd (Leu) | Thr | Thr |
| 4th (Gly) | Tyr | Tyr |
| 5th (Pro) | Arg | Arg |
| 17th (Cys) | Ala | Thr |

The hG-CSF derivatives encoded by pCfBD28A17 and pCfBD28T17 are hereinafter called hG-CSF[ND28A17] and hG-CSF[ND28T17], respectively.

### Reference Example 1

### Isolation of the hG-CSF cDNA-carrying plasmid pCSF1-2

### (1) Preparation of poly(A) RNA from normal human peripheral blood macrophages

Macrophages, which are adherent cells, were isolated by culturing leukocytes obtained by centrifugation of normal human peripheral blood in a plastic bottle and removing nonadherent cells by washing. An RNA having poly(A) was prepared from the macrophages by the guanidine thiocyanate-lithium chloride method [Cathala et al.: DNA, 2, 329 (1983)], as follows.

Normal human peripheral blood (400 ml) was centrifuged on a Hitachi RPR10 rotor at 1,800 rpm for 20 minutes. The resultant blood cell precipitate was suspended in 50 ml of phosphate-buffered saline [8 g/liter NaCI, 0.2 g/liter KCI, 1.15 g/liter anhydrous Na₂HPO₄, 0.2 g/liter KH₂PO₄ (pH 7.2); hereinafter abbreviated as PBS]. A 25-ml portion of this suspension was layered on 25 ml of lymphocyte separation liquid (BIONETICS), and the whole was centrifuged on a Hitachi RPR10 rotor at 1,800 rpm for 30 minutes. Leukocytes in the middle layer were collected, washed with an equal volume of PBS (on a Hitachi RPR10 rotor at 1,500 rpm for 10 minutes), then suspended in 20 ml of RPMI 1640 medium (Nissui Seiyaku) containing 5% fetal bovine serum, and cultured using a tissue culture flask (Corning). After growing at 37°C for 1.5 hours, the culture supernatant was removed together with nonadherent cells. A fresh 20-ml portion of the same medium and E. coli-derived lipopolysaccharide (LPS) (in an amount to give a concentration of 0.3 mg/ml) were added, and cultivation was continued at 37°C for further 4 hours. Then, cells were harvested from the culture by centrifugation at 1,100 x g at 4°C for 10 minutes, washed with 80 ml of PBS and solubilized in 10 ml of a solution comprising 5 M guanidine thiocyanate, 10 mM EDTA, 50 mM Tris-HCI (pH 7) and 8% (v/v) 2-mercaptoethanol using a vortex mixer. This solubilization product was transferred to a centrifuge tube, 80 ml of 4 M LiCl were added, and the mixture was stirred, then allowed to stand at 4°C for 20 hours and centrifuged on a Hitachi RPR10 rotor at 9,000 rpm for 90 minutes. Thereafter, an RNA precipitate was recovered. The RNA precipitate was suspended in 50 ml of a solution comprising 4 M urea and 2 M lithium chloride, and the suspension was centrifuged on a Hitachi RPR10 rotor at 9,000 rpm for 60 minutes, and an RNA precipitate was again recovered.

The RNA precipitate was dissolved in 10 ml of a solution comprising 0.1% sodium lauryl sulfate, 1 mM EDTA and 10 mM Tris-HCI (pH 7.5) and the RNA was recovered by phenol-chloroform extraction and ethanol precipitation. The RNA obtained (about 0.8 mg) was dissolved in 1 ml of a solution comprising 10 mM Tris-HCI (pH 8.0) and 1 mM EDTA. After incubation at 65°C for 5 minutes, 0.1 ml of 5 M NaCI were added. The mixture was subjected to oligo(dT)-cellulose column (P-L Biochemicals) chromatography (column volume 0.5 ml). The adsorbed, poly(A)-containing mRNA was eluted with a solution comprising 10 mM Tris-HCI (pH 7.5) and 1 mM EDTA to give about 30 Ug of poly(A)-containing mRNA.

### (2) cDNA synthesis and insertion of the DNA into a vector

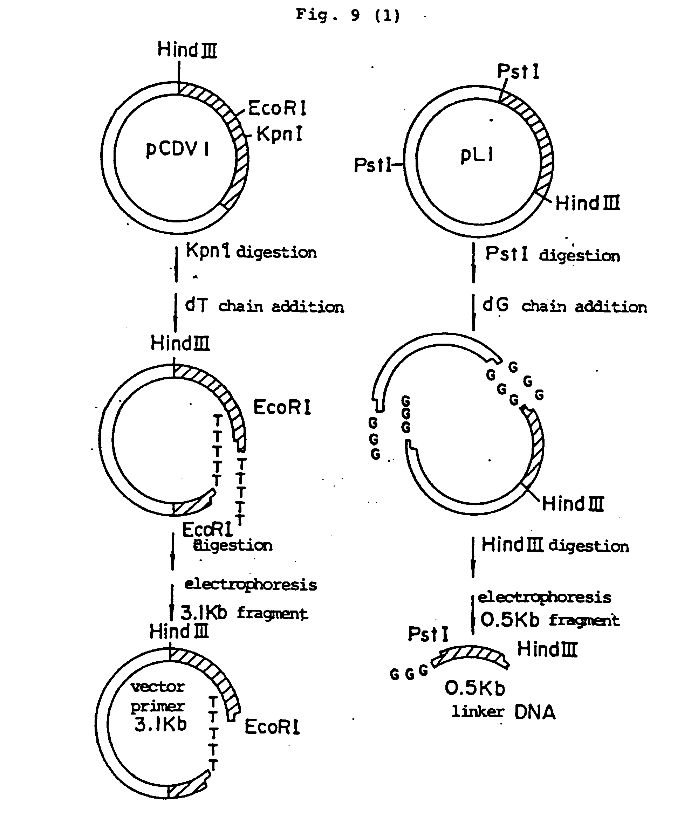

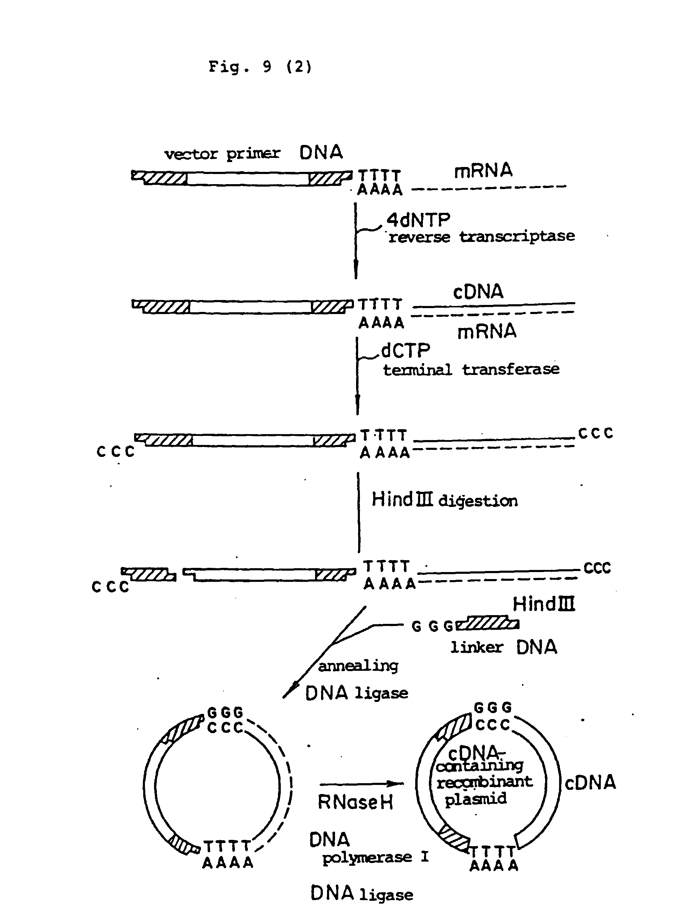

The Okayama-Berg method [Mol. Cell. Biol., 2, 161 (1982)] was used for cDNA synthesis and recombinant plasmid construction by insertion of the cDNA obtained. The processes therefor are outlined in Fig. 9.

To 300 µl of a solution comprising 10 mM Tris-HCI (pH 7.5), 6 mM MgCl₂ and 10 mM NaCI, there were added 400 µg of pCDV1 [Okayama & Berg: Mol. Cell. Biol., 3, 280 (1983)] and, after further addition of 500 units of KpnI, the reaction was carried out at 37°C for 6 hours, whereby the plasmid was cleaved at the KpnI site. The DNA was recovered by phenol-chloroform extraction and ethanol precipitation. About 200 µg of the KpnI-cleaved DNA was added to 200 µl of a solution prepared by adding dTTP in a concentration of 0.25 mM to a buffer (hereinafter abbreviated as TdT buffer) comprising 40 mM sodium cacodylate, 30 mM Tris-HCI (pH 6.8), 1 mM CaCl₂ and 0.1 mM dithiothreitol (hereinafter abbreviated as DTT) and, after further addition of 81 units of terminal deoxynucleotidyl transferase (hereinafter abbreviated as TdT) (P-L Biochemicals), the reaction was carried out at 37°C for 11 minutes, whereby a poly(dT) chain comprising about 67 dT residues was added to each KpnI cleavage site 3' end of pCDV1. About 100 µg of the poly(dT) chain-added pCDV1 DNA were recovered from the above reaction mixture by phenol-chloroform extraction and ethanol precipitation. The DNA was added to 150 µl of a buffer comprising 10 mM Tris-HCI (pH 7.5), 6 mM MgCl₂ and 100 mM NaCI, 360 units of EcoRI were further added, and the reaction was carried out at 37°C for 2 hours. The reaction mixture was treated by the LGT method, and a DNA fragment of about 3.1 kb was recovered. Thus were obtained about 60 µg of the poly(dT) chain-tailed pCDV1. The DNA was dissolved in 500 µl of a solution comprising 10 mM Tris-HCI (pH 8.0) and 1 mM EDTA, the solution was incubated at 65°C for 5 minutes and then cooled with ice, and 50 µl of 5 M NaCI were added. The mixture was subjected to oligo(dA)-cellulose column (Collaborative Research) chromatography. Molecules having a sufficient poly(dT) chain length were adsorbed on the column and they were eluted with a solution comprising 10 mM Tris-HCI (pH 8.0) and 1 mM EDTA. Thus were obtained 27 µg of the poly(dT) chain-tailed pCDV1 (hereinafter abbreviated as vector primer).

Then, a linker DNA was synthesized.

To 200 µl of a buffer comprising 10 mM Tris-HCI (pH 7.5), 6 mM MgCl₂ and 50 mM NaCI, there were added about 14 µg of pL1 [Okayama & Berg: Mol. Cell. Biol., 3, 280 (1983)], 50 units of PstI were further added, and the reaction was carried out at 37°C for 4 hours, whereby the pL1 DNA was cleaved at the PstI site. The reaction mixture was subjected to phenol-chloroform extraction, followed by ethanol precipitation, whereby about 13 µg of the PstI-cleaved pL1 DNA were recovered. The DNA (about 13 µg) was added to 50 µl of TdT buffer supplemented with dGTP in a final concentration of 0.25 mM, 54 units of TdT (P-L Biochemicals) were further added, and the mixture was incubated at 37°C for 13 minutes, whereby a (dG) chain containing about 14 dG residues was added to pL1 at each 3' end at the PstI cleavage site. The DNA was recovered by phenol-chloroform extraction followed by ethanol precipitation. The DNA was added to 100 µl of a buffer comprising 10 mM Tris-HCI (pH 7.5), 6 mM MgCl₂ and 60 mM NaCI, 80 units of HindIII were further added, and the mixture was incubated at 37°C for 3 hours, whereby the pL1 DNA was cleaved it the HindIII site. The reaction mixture was fractionated by agarose gel electrophoresis, and a DNA fragment of about 0.5 kb was recovered by the DEAE-paper method [Dretzen et al.: Anal. Biochem., 112, 295 (1981)]. Thus was obtained the oligo(dG) chain-tailed linker DNA (hereinafter referred to simply as linker DNA).

In 22.3 µl of a solution comprising 50 mM Tris-HCI (pH 8.3), 8 mM MgCl₂, 30 mM KCI, 0.3 mM DTT, 2 mM dNTP (dATP, dTTP, dGTP and dCTP) and 10 units of ribonuclease inhibitor (P-L Biochemicals), there were dissolved about 3 µg of the poly(A) RNA and about 1.4 µg of the vector primer, each prepared as described above, 10 units of reverse transcriptase (Seikagaku Kogyo) were added, and the mRNA was caused to synthesize a DNA complementary thereto by incubating the mixture at 41°C for 90 minutes. The reaction mixture was subjected to phenol-chloroform extraction and the vector primer DNA with the RNA-DNA double strand added thereto was recovered by ethanol precipitation. This DNA was dissolved in 20 µl of TdT buffer containing 66 µM dCTP and 0.2 µg of poly(A), 14 units of TdT (P-L Biochemicals) were added, and the mixture was incubated at 37°C for 2 minutes, whereby a (dC) chain containing 20 dC residues was added to the 3' end of the cDNA. The reaction mixture was extracted with phenol-chloroform, and the (dC) chain-tailed cDNA-vector primer DNA was recovered by ethanol precipitation. The DNA was dissolved in 400 µl of a solution comprising 10 mM Tris-HCI (pH 7.5), 6 mM MgCl₂ and 60 mM NaCI, 20 units of HindIII were added, and incubation was conducted at 37°C for 2 hours to cause cleavage at the HindIII site. Phenol-chloroform extraction of the reaction mixture and the subsequent ethanol precipitation gave 0.5 picomole of the (dC) chain-tailed cDNA-vector primer DNA. In 100 µl of a solution comprising 10 mM Tris-HCI (pH 7.5). 0.1 M NaCI and 1 mM EDTA, there were dissolved 0.2 picomole of the DNA and 0.4 picomole of the above-mentioned linker DNA, and incubation was carried out at 65°C, 42°C and 0°C for 10 minutes, 25 minutes and 30 minutes, respectively. A total volume of 1,000 µl of a reaction mixture was prepared which had the following composition: 20 mM Tris-HCI (pH 7.5), 4 mM MgCl₂, 10 mM (NH₄)₂SO₄, 0.1 M KCI and 0.1 mM β-NAD. To this reaction mixture were added 25 units of E. coli-derived DNA ligase (New England Bio-Labs), and incubation was carried out at 11°C for 18 hours. The reaction mixture was supplemented with 40 µM each dNTP and with β-NAD to give a final concentration of 0.15 mM, 10 units of E. coli DNA ligase, 20 units of E. coli DNA polymerase I (P-L Biochemicals) and 10 units of E. coli ribonuclease H (P-L Biochemicals) were added, and incubation was carried out at 12°C for 1 hour and then at 25°C for 1 hour. The above reaction procedure caused circularization of the cDNA-containing recombinant DNA and substitution of the RNA portion of the RNA-DNA double strand by the corresponding DNA. Thus was formed the recombinant plasmid in the completely double-stranded DNA form.

### (3) Selection of the hG-CSF cDNA-containing recombinant DNA

The recombinant plasmids obtained as described in (2) were used to transform E. coli C600SF8 by the method of Scott et al. [Katsuya Shigesada: Saibo Kogaku (Cell Technology), 2, 616 (1983)]. About 9,200 colonies obtained were fixed on a nitrocellulose filter. One strain capable of associating strongly at 60°C with a probe prepared by labeling, with ³²P, the 27-nucleotide synthetic DNA 5'-ACCCCCCTGGGCCCTGCCAGCTCCCTG-3' corresponding to the N-terminal 9 amino acids of the mature hG-CSF protein as isolated by Nagata et al [Nagata et al.: Nature, 319, 415 (1986)] was selected [the Grunstein-Hogness method; Proc. Natl. Acad. Sci. USA, 72, 3961(1975)]. The whole base sequence of the cDNA contained in the plasmid pCSF1-2 carried by this strain was determined by the dideoxy sequencing method using M13 phage (Table 1). As a result, it was found that the cDNA contained in pCSF1-2 codes for hG-CSF.

This bacterial strain has been deposited with the FRI under the designation E. coli ECSF1-2 (FERM BP-1220), as mentioned hereinabove.

### Reference Example 2

### Isolation and purification of the plasmid pKYP26

A pKYP26-bearing E. coli strain [E. coli IKYP26 (FERM BP-863)] was cultured in 10 ml of L medium (1% Bacto-tryptone, 0.5% yeast extract, 1% NaCI, pH 7.5) containing 50 µg/ml of ampicillin at 37°C for 18 hours. The whole culture was transferred to 1 liter of L medium containing 50 µg/ml of ampicillin, and cultured at 37°C. After 4 hours, chloramphenicol was added in a concentration of 170 µg/ml, and cultivation was continued at 37°C for additional 16 hours. Cells were harvested by centrifugation (5,000 rpm, 10 minutes), washed with 0.8% NaCI and suspended in 20 ml of 50 mM Tris-HCI (pH 8.0), and the suspension was cooled with ice. Lysozyme (10 mg/ml, 8 ml) was added and, after standing in ice for 10 minutes, 9.6 ml of 0.5 M EDTA were added. After standing in ice for 10 minutes, 2.3 ml of 2% Triton X-100 (Wako Pure Chemical Industries) were added, followed by further standing in ice for 1 hour. Ultracentrifugation at 50,000 x g at 4°C for 1 hour gave about 40 ml of a supernatant. Then, this supernatant was adjusted to pH 12.5 by addition of 3 M NaOH and stirred gently at room temperature for 10 minutes. The pH was brought back to 8.5 by addition of 2 M Tris-HCI (pH 7.5), followed by further stirring for 3 minutes. At this timepoint, the liquid volume was about 55 ml. A 1/9 volume of 5 M NaCI was added and then phenol extraction was carried out. A 1/250 volume of 5 mg/ml RNase A (Sigma) was added, the RNA degradation reaction was conducted at 37°C for 1 hour, a 1/5 volume of 5 M NaCI was then added, and a 1/3 volume of 30% PEG 6000 (Nakarai Chemicals) was added. The resultant mixture was allowed to stand at -20°C for 2 hours. The resultant precipitate was collected by centrifugation and dissolved in 2 ml of a solution comprising 10 mM Tris-HCI (pH 7.5) and 1 mM EDTA, sodium dodecyl sulfate (SDS) was added in a concentration of 0.5%, proteinase K (Sigma) was added in a concentration of 50 µg/ml, and the proteolytic reaction was carried out at 37°C for 1 hour. After three repetitions of phenol extraction, the DNA was recovered by chloroform extraction and ethanol precipitation, and dissolved in 1 ml of a solution comprising 10 mM Tris-HCI (pH 7.5) and 1 mM EDTA. In this way, 800 µg of pKYP26 could be obtained. The structure of pKYP26 was confirmed by cleavage with EcoRI, KpnI, BamHI, BglII and PstI, followed by agarose gel electrophoresis.

### Reference Example 3

### 1) Isolation of the human LT cDNA-carrying plasmid pLT1

### (1) Preparation of poly(A) RNA from Lukll cells

The guanidine thiocyanate-lithium chloride method [Cathala et al.: DNA, 2, 329 (1983)] was followed to prepare a poly(A)-carrying RNA from the human lymphoblastoid cell line LukII, as follows:

Human lymphoblastoid Lukll cells [Berish Y. Rubin et al.: Proc. Natl. Acad. Sci. USA, 82, 6637 (1985)] were sowed into 1 liter of RPMI 1640 medium (Nissui Seiyaku) containing 5% fetal bovine serum and 1 mM N-2-hydroxyethylpiperazine-N'-2-ethanesulfonic acid (HEPES) in a cell concentration of 8 x 10⁵ cells/ml and grown there. A spinner culture bottle was used for the culture. After cultivation at 37°C for 48 hours, cells were collected by centrifugation and transferred to a fresh 1-liter portion or RPMI 1640 medium containing 10 ng/ml phorbol myristate acetate (PMA), 5% fetal bovine serum and 1 mM HEPES, and cultivation was conducted at 37°C for further 48 hours. Then cells were harvested from a portion (250 ml) of this cell suspension by centrifugation at 1,100 x g at 4°C for 10 minutes, washed with 80 ml of phosphate buffer, and solubilized in 10 ml of a solution comprising 5 M guanidine thiocyanate, 10 mM EDTA, 50 mM Tris-HCI (pH 7) and 8% (v/v) 2-mercaptoethanol using a vortex mixer. The solubilization product was transferred to a centrifuge tube, 80 ml of 4 M LiCl were added, and the mixture was stirred and then allowed to stand at 4°C for 20 hours. After centrifugation on a Hitachi RPR10 rotor at 9,000 rpm for 90 minutes, an RNA precipitate was recovered. The RNA precipitate was suspended in 50 ml of a solution comprising 4 M urea and 2 M lithium chloride and, after centrifugation on a Hitachi RPR10 rotor at 9,000 rpm for 60 minutes, the RNA was again recovered as a precipitate. The RNA precipitate was dissolved in 10 ml of a solution comprising 0.1% sodium lauryl sulfate, 1 mM EDTA and 10 mM Tris-HCI (pH 7.5), and the RNA was recovered by phenol-chloroform extraction followed by ethanol precipitation. About 2.5 mg of the thus-obtained RNA was dissolved in 1 ml of a solution comprising 10 mM Tris-HCI (pH 8.0) and 1 mM EDTA. After incubation at 65°C for 5 minutes, 0.1 ml of 5 M NaCI were added. The mixture was subjected to oligo(dT)-cellulose column (P-L Biochemicals) chromatography (column volume 0.5 ml). The adsorbed, poly(A)-containing mRNA was eluted with a solution comprising 10 mM Tris-HCI (pH 7.5) and 1 mM EDTA. About 100 µg of the poly(A)-containing mRNA was obtained.

### (2) cDNA synthesis and insertion of the DNA into a vector

The Okayama-Berg method [Mol. Cell. Biol., 2, 161 (1982)] was followed for cDNA synthesis and recombinant plasmid construction by insertion of the cDNA obtained. The processes therefor are outlined in Fig. 9.

To 300 µl of a solution comprising 10 mM Tris-HCI (pH 7.5), 6 mM MgCl₂ and 10 mM NaCI, there were added 400 µg of pCDV1 [Okayama & Berg: Mol. Cell. Biol., 3, 280 (1983)] and, after further addition of 500 units of KpnI, the reaction was carried out at 37°C for 6 hours, whereby the plasmid was cleaved at the KpnI site. The DNA was recovered by phenol-chloroform extraction followed by ethanol precipitation. About 200 µg of the KpnI-cleaved DNA was added to 200 µl of a solution prepared by adding dTTP in a concentration of 0.25 mM to TdT buffer and, after further addition of 81 units of TdT [P-L Biochemicals), the reaction was carried out at 37°C for 11 minutes, whereby a poly(dT) chain (about 67 dT residues) was added to each 3' end of the KpnI cleavage site of pCDV1. About 100 µg of the poly(dT) chain-tailed pCDV1 DNA was recovered from the solution by ethanol precipitation following phenol-chloroform extraction. The DNA was added to 150 µl of a solution comprising 10 mM Tris-HCI (pH 7.5), 6 mM MgCl₂ and 100 mM NaCI and, after further addition of 360 units of EcoRI, the reaction was carried out at 37°C for 2 hours. The reaction mixture was treated by the LGT method, and a DNA fragment of about 3.1 kb was recovered. About 60 µg of the poly(dT) chain-tailed pCDV1 were thus obtained. The DNA was dissolved in 500 µl of a solution comprising 10 mM Tris-HCI (pH 8.0) and 1 mM EDTA, the solution was incubated at 65°C for 5 minutes and then cooled with ice, and 50 µl of 5 M NaCI were added. The mixture was subjected to oligo-(dA)-cellulose column (Collaborative Research) chromatography. Molecules having a sufficient poly(dT) chain length were adsorbed on the column and they were eluted with a solution comprising 10 mM Tris-HCI (pH 8.0) and 1 mM EDTA to give 27 µg of the poly(dT) chain-tailed pCDV1 (hereinafter referred to as vector primer).

Then, a linker DNA was prepared.

About 14 µg of pL1 [Okayama & Berg: Mol. Cell. Biol., 3, 280 (1983)] were added to 200 µl of a buffer comprising 10 mM Tris-HCI (pH 7.5), 6 mM MgCl₂ and 50 mM NaCI and, after further addition of 50 units of PstI, the reaction was carried out at 37°C for 4 hours for cleavage of the pL1 DNA at the PstI site. The reaction mixture was subjected to phenol-chloroform extraction and about 13 µg of the PstI-cleaved pL1 DNA were recovered by ethanol precipitation. About 13 µg of the DNA were added to 50 µl of TdT buffer containing dGTP in a final concentration of 0.25 mM and, after further addition of 54 units of TdT (P-L Biochemicals), incubation was carried out at 37°C for 13 minutes to cause addition of a (dG) chain (about 14 dG residues) to pL1 at each PstI cleavage site 3' end. After phenol-chloroform extraction, the DNA was recovered by ethanol precipitation. The DNA was added to 100 µl of a buffer comprising 10 mM Tris-HCI (pH 7.5), 6 mM MgCl₂ and 60 mM NaCI and, after further addition of 80 units of HindIII, incubation was carried out at 37°C for 3 hours to cause cleavage of the pL1 DNA at the HindIII site. The reaction mixture was fractionated by agarose gel electrophoresis, and a DNA fragment of about 0.5 kb was recovered by the DEAE-paper method [Dretzen et al.: Anal. Biochem., 112, 295 (1981)]. Thus was obtained the oligo(dG) chain-tailed linker DNA (hereinafter referred to simply as linker DNA).

About 2 µg of the poly(A) RNA and about 1.4 µg of the vector primer were dissolved in 22.3 µl of a solution comprising 50 mM Tris-HCI (pH 8.3), 8 mM MgCl₂, 30 mM KCI, 0.3 mM DTT, 2 mM dNTP (dATP, dTTP, dGTP and dCTP) and 10 units of ribonuclease inhibitor (P-L Biochemicals), 10 units of reverse transcriptase (Seikagaku Kogyo) were added, and incubation was carried out at 41°C for 90 minutes to cause the mRNA to synthesize a DNA complementary thereto. The reaction mixture was subjected to phenol-chloroform extraction followed by ethanol precipitation, whereby the vector primer DNA with the RNA-DNA double strand added thereto was recovered. The DNA was dissolved in 20 µl of TdT buffer containing 66 µM dCTP and 0.2 µg of poly(A), 14 units of TdT (P-L Biochemicals) were added, and incubation was performed at 37°C for 2 minutes to cause addition of a (dC) chain (20 dC residues) to the 3' end of the cDNA. The reaction mixture was subjected to phenol-chloroform extraction and then the (dC) chain-tailed cDNA-vector primer DNA was recovered by ethanol precipitation. The DNA was dissolved in 400 µl of a solution comprising 10 mM Tris-HCI (pH 7.5), 6 mM MgCl₂ and 60 mM NaCI, 20 units of HindIII were added, and incubation was carried out at 37°C for 2 hours for cleavage at the HindIII site. Phenol-chloroform extraction of the reaction mixture and ethanol precipitation gave 0.5 picomole of the (dC) chain-tailed cDNA-vector primer DNA. The DNA (0.2 picomole) and 0.4 picomole of the above-mentioned linker DNA were dissolved. in 100 µl of a solution comprising 10 mM Tris-HCI (pH 7.5), 0.1 M NaCI and 1 mM EDTA, and incubation was carried out at 65°C, 42°C and 0°C for 10 minutes, 25 minutes and 30 minutes, respectively, in that order. A total volume of 100 µl of a reaction mixture was prepared according to the following composition: 20 mM Tris-HCI (pH 7.5), 4 mM MgCl₂, 10 mM (NH₄)₂SO₄, 0.1 M KCI and 0.1 mM β-NAD. To this reaction mixture, there were added 25 units of E. coli DNA ligase (New England Bio-Labs), and incubation was performed at 11°C for 18 hours. The reaction mixture was supplemented with 40 µM each dNTP and with β-NAD in a final concentration of 0.15 mM and, after addition of 10 units of E. coli DNA ligase, 20 units of E. coli DNA polymerase I (P-L Biochemicals) and 10 units of E. coli ribonuclease H (P-L Biochemicals), incubation was carried out at 12°C for 1 hour and then at 25°C for 1 hour. The above reaction procedure caused circularization of the cDNA-containing recombinant DNA and substitution of the RNA portion of the RNA-DNA double strand by the corresponding DNA. Thus, the recombinant plasmid was formed in the form of a completely double-stranded DNA.

### (3) Selection of the human LT cDNA-containing recombinant DNA

The recombinant plasmids obtained as described in (2) were used to transform E. coli C600SF8[Cameron: Proc. Natl. Acad. Sci. USA, 72, 3416 (1975)] by the method of Scott et al. [Katsuya Shigesada: Saibo Kogaku (Cell Technology), 2, 616 (1983)]. About 30,000 colonies obtained were fixed on a nitrocellulose filter. One strain capable of strongly associating, at 52°C, with a probe prepared by labeling, with ³²P, the 17-nucleotide synthetic DNA 5'-GATCCCCGGCCTGCCTG-3' corresponding to the base sequence of part of the 5' untranslational region of the human LT cDNA isolated by Genentech [Patrick W. Gray et al.: Nature, 312, 721 (1984)] was selected [Grunstein-Hogness method: Proc. Natl. Acad. Sci. USA, 72, 3961 (1975)]. The whole base sequence of the cDNA of the plasmid pLT1 carried by this strain was determined by the dideoxy sequencing method using M13 phage. As a result, it was found that the pLT1 DNA codes for human LT.

### 2) Construction of the recombinant plasmid pLA1

In a total of 50 µl of a solution (hereinafter referred to as "Y-0 buffer") containing 10 mM Tris-HCI (pH 7.5), 7 mM MgCl₂ and 6 mM 2-mercaptoethanol, there were dissolved 5 µg of pLT1 (4.7 kb) obtained by the procedure described in the preceding section, 10 units of the restriction enzyme XhoII (Boehringer Mannheim) were added, and the cleavage reaction was conducted at 37°C for 2 hours. Then, NaCI was added in a final concentration of 150 mM, 10 units of the restriction enzyme NsiI (New England Bio-Labs) was added, and the cleavage reaction was carried out at 37°C for further 3 hours. From the reaction mixture, there was obtained, by the LGT method, about 0.3 µg of an about 750 bp DNA fragment (XhoII-NsiI fragment) containing most of the human LT DNA.

Separately, 20 µg of pLT1 was dissolved in 200 µl of Y-50 buffer, 40 units of the restriction enzyme HaeIII was added, and the cleavage reaction was performed at 37°C for 2 hours. Then, NaCI was added in a final concentration of 150 mM, 40 units of Nsil was added, and the cleavage reaction was carried out at 37°C for further 3 hours. Polyacrylamide gel electrophoresis of the reaction mixture gave about 40 ng of an about 50 bp DNA fragment (HaeIII-Nsil fragment) containing the N-terminal portion of human LT.

Further, separately, 3 µg of pGEL1 (3.4 kb) was dissolved in a total of 30 µl of Y-100 buffer, 6 units each of the restriction enzymes StuI and BglII were added, and the cleavage reaction was carried out at 37°C for 3 hours.

From the reaction mixture, there was obtained, by the LGT method, about 1.0 ug of an Ap^{r} genecontaining DNA fragment of about 2.3 kb (StuI-BglII fragment).

Then, 0.2 µg of the pLT1-derived XhoII-NsiI fragment (about 750 bp), 20 ng of the pLT1-derived HaeIII-Nsil fragment (about 50 bp) and 0.6 µg of the pGEL1-derived StuI-BglII fragment (about 2.3 kb) were dissolved in a total of 20 µl of T4 ligase buffer, 2 units of T4 DNA ligase (Takara Shuzo) was further added to this mixture solution, and the reaction was carried out at 4°C for 18 hours.

The recombinant plasmid DNA thus obtained was used to transform E. coli KM430 by the method of Cohen et al., and an Ap^{r} colony was obtained. The plasmid DNA was isolated and purified from this transformant by a known method, and the structure of the plasmid was analyzed by cleavage of said plasmid DNA with restriction enzymes such as StuI. As a result, it was confirmed that the desired plasmid had been obtained. This recombinant plasmid is named pLA1.

### 3) Construction of the LT expression plasmid pLSA1

An E. coli KM430 transformant harboring pLA1 (3.1 kb) obtained as described in the preceding section was cultured, and the pLA1 DNA was prepared from cultured cells thereof in the conventional manner. In 30 µl of Y-100 buffer, there were dissolved 3 µg of the pLA1 DNA obtained, 3 units each of StuI and BglII were added, and the cleavage reaction was conducted at 37°C for 3 hours. From the reaction mixture, there were obtained, by the LGT method, about 0.5 µg of an about 790 bp DNA fragment (StuI-BglII fragment) containing most of the human LT gene.

Separately, 3 µg of pKYP10 prepared by the method described in US Patent 4,686,191 were dissolved in 30 µl of Y-100 buffer, 6 units each of the restriction enzymes BanIII and PstI were added, and the cleavage reaction was carried out at 37°C for 3 hours. From the reaction mixture, there was obtained, by the LGT method, a tryptophan promoter (Ptrp)-containing DNA fragment of about 1.1 kb (BanIII-PstI fragment). Further, 2 µg of pGEL1 (3.4 kb) were dissolved in 20 µl of Y-100 buffer, 4 units each of the restriction enzymes HindIII, BamHI and PstI were added, and the cleavage reaction was carried out at 37°C for 3 hours. From the reaction mixture, there were obtained, by the LGT method, about 0.7 µg of a lipoprotein-derived terminator-containing DNA fragment of about 1.7 kb (PstI-BamHI fragment).

Separately, for such reasons as the necessity of providing the sequence from the N terminus of the mature human LT polypeptide, namely Leu (CTA) to the second base (GG) of the 5th amino acid Gly (GGC) as well as the initiation codon (ATG) required for expression and the necessity of adjusting the distance between the SD sequence downstream from Ptrp and ATG to an appropriate length of 6-18 bp, the following DNA linker was synthesized:

BanIII                27 mer                blunt-end
Met Leu Pro Gly Val
5'-CGATAAGCTT ATG CTA CCA GGA GTA GG    -3'
3'-   TATTCGAA TAC GAT GGT CCT CAT CC    -5'
25 mer

First, the 27-mer and 25-mer single-stranded DNAs were synthesized by the ordinary phosphotriester method. The 27-mer and 25-mer (each 20 picomoles) were dissolved in a total of 40 µl of T4 kinase buffer, 6 units of T4 polynucleotide kinase (Takara Shuzo) were added, and the phosphorylation reaction was carried out at 37°C for 60 minutes.

Then, 0.3 µg of the pLA1-derived StuI-BglII fragment (about 790 bp), 0.4 µg of the BanIII-PstI fragment (about 1.1 kb) of the expression vector pKYP10 and 0.6 µg of the pGEL1-derived PstI-BamHI fragment (about 1.7 kb), each obtained as described above, were dissolved in 25 µl of T4 ligase buffer, and about 1 picomole of the above DNA linker was added to this mixture solution. After further addition of 6 units of T4 DNA ligase to this mixture, the ligation reaction was conducted at 4°C for 18 hours.

The recombinant plasmid-containing reaction mixture was used to transform E. coli KM430, and an Ap^{r} colony was obtained. The plasmid DNA was recovered from cultured cells of this colony. The structure of the plasmid obtained was confirmed by cleavage with the restriction enzymes EcoRI, BanIII, PstI, HindIII and BglII followed by agarose gel electrophoresis. This plasmid is named pLSA1. The base sequence of pLSA1 in the vicinity of BanIII and HindIII was confirmed by the Maxam-Gilbert method [(A. M. Maxam et al.: Proc. Natl. Acad. Sci. USA, 74, 560 (1977)] to be as follows:

BanIII HindIII
Met Leu Pro Gly Val Gly Leu
CG ATA AGC TT ATG CTA CCA GGA GTA GGC CTC

### Reference Example 4

### Construction of the ATG vector pTrS20

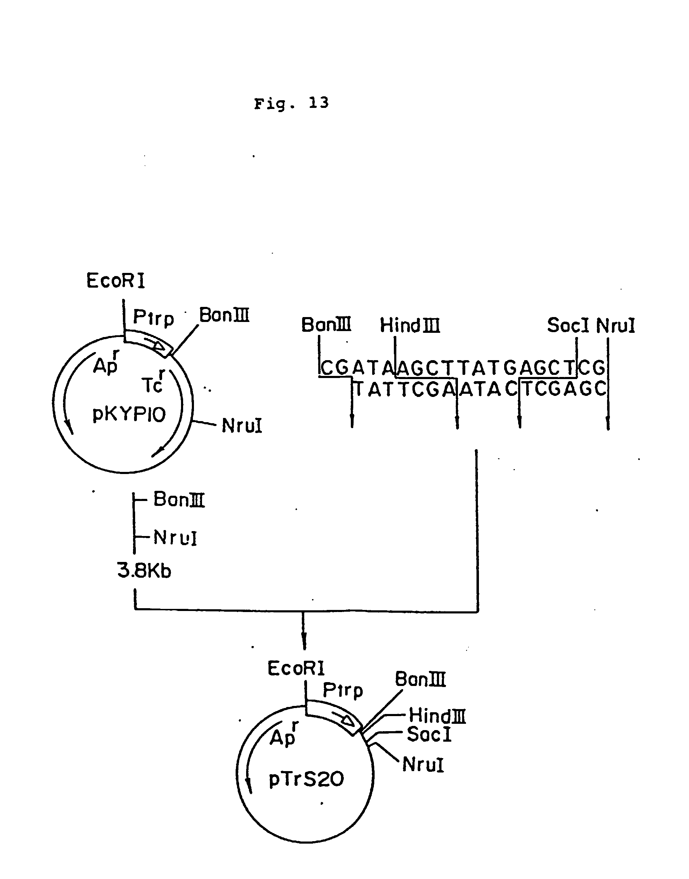

By following the procedure shown in Fig. 13, the ATG vector pTrS20 in which the distance between the SD sequence and the initiation codon ATG is 14 bases and which has an SacI site immediately behind the ATG codon was constructed.

First, 3 µg of pKYP10 prepared by the method described in US Patent 4,686,191 were dissolved in 30 µl of Y-100 buffer, 6 units each of the restriction enzymes BanIII and NruI (New England Bio-Labs) were added, and the cleavage reaction was conducted at 37°C for 3 hours. From the reaction mixture, there were obtained, by the LGT method, about 0.5 µg of a Ptrp-containing DNA fragment of about 3.8 kb (BanIII-NruI fragment).

Separately, the following DNA linker was synthesized by the phosphotriester method for providing the initiation codon ATG downstream from Ptrp:

BanIII HindIII SacI NruI
Met
5'- CGATAAGCTTATGAGCTCG -3' (19 mer)
3'- TATTCGAATACTCGAGC -5' (17 mer)

The 19-mer and 17-mer synthetic DNAs (each 10 picomoles) were dissolved in a total of 20 µl of a solution containing 50 mM Tris-HCI (pH 7.5), 10 mM MgCl₂, 5 mM dithiothreitol, 0.1 mM EDTA and 1 mM ATP, 3 units of T4 polynucleotide kinase (Takara Shuzo) were added, and the phosphorylation reaction was effected at 37°C for 60 minutes.

Then, 0.1 µg of the pKYP10-derived BanIII-NruI fragment (about 3.8 kb) obtained as described above and about 0.5 picomole of the above DNA linker were dissolved in 20 µl of T4 ligase buffer, 2 units of T4 DNA ligase were further added, and the ligation reaction was carried out at 4°C for 18 hours.

The recombinant plasmid mixture obtained was used to transform E. coli HB101 [Boliver et al.: Gene, 2, 75 (1977)], and an Ap^{r} colony was obtained. From cultured cells of this colony, there was recovered the plasmid DNA . The structure of the plasmid obtained was confirmed by cleavage with the restriction enzymes EcoRI, BanIII, HindIII, SacI and NruI, followed by agarose gel electrophoresis. This plasmid was named pTrS20 (Fig. 13). The base sequence of pTrS20 in the neighborhood of the BanIII and HindIII sites was confirmed by the dideoxy sequencing method using M13 phage to be as follows:

BanIII HindIII SacI NruI
SD Sequence Met
AAGG GTAT CGATA AGCTT ATG AGCT CG CGA

## Claims (Claims for the following Contracting State(s): BE, CH, DE, FR, GB, IT, LI, NL, SE)

1. A polypeptide having an amino acid sequence as derived from the amino acid sequence of the human granulocyte colony stimulating factor (hG-CSF) polypeptide by amino acid substitution,
characterized in that
at least one amino acid out of the 1st to 6th amino acids on the N terminus side and optionally the 17th amino acid thereof differs from the amino acid in the corresponding position of the hG-CSF polypeptide,
with the proviso that more than one amino acid of hG-CSF is substituted if the amino acid Thr⁺¹ of hG-CSF is replaced by Ala, Gly or Pro; and with the further proviso that the amino acids in position +1 and +2 are not simultaneously substituted.

2. A DNA coding for a polypeptide of Claim 1

3. A recombinant plasmid comprising a plasmid DNA and, as an insert therein, a DNA fragment coding for a polypeptide as defined in claim 1.

4. The recombinant plasmid according to Claim 3, wherein the plasmid DNA is a tryptophan promoter-containing plasmid DNA, with said DNA fragment inserted in said plasmid DNA at a site downstream from the tryptophan promoter.

5. The recombinant plasmid of claim 3 or 4 which is selected from the group consisting of pCfTL38, pCfTL41, pCfTL35 and pCfTAArg4 comprising an insert which codes for a hG-CSF polypeptide being modified as follows:
| Plasmid | Position of amino acid substitution (substituted amino acid of hG-CSF) | |
|---|---|---|
| | 1st (Thr) | 4th (Gly) |
| pCfTL38 | Ser | *) |
| pCfTL41 | Arg | |
| pCfTL35 | Cys | |
| pCfTAArg4 | | Arg |
| | | |
|---|---|---|
| *) No substitution | | |

6. The recombinant plasmid of claim 3 or 4 which is selected from the group consisting of pCfTM14, pCfTM17, pCfTM113 and pCfTAArg4S comprising an insert which codes for a hG-CSF polypeptide being modified as follows:
| Plasmid | Position of amino acid substitution (substituted amino acid of hG-CSF) | | | |
|---|---|---|---|---|
| | 1st (Thr) | 3rd(Leu) | 4th (Gly) | 17th (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) No substitution | | | | |

7. The recombinant plasmid of claim 3 or 4 which is selected from the group consisting of pCfBC42B1, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 and pCfBD28T17 comprising an insert which codes for a hG-CSF polypeptide being modified as follows:
| Plasmid | Position of amino acid substitution (amino acid of hG-CSF) | | | | | |
|---|---|---|---|---|---|---|
| | 1st (Thr) | 3rd (Leu) | 4th (Gly) | 5th (Pro) | 17th (Cys) | 6th (Ala) |
| pCfBC42B1 | -* | Glu | Lys | Ser | Ser | -* |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -* | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| -* No substitution | | | | | | |

8. A microorganism harboring a recombinant plasmid as defined in one of the Claims 5 to 7.

9. A method of producing a polypeptide of Claim 1, which method comprises (a) cultivating in a medium a microorganism harboring a recombinant plasmid coding for said polypeptide to thereby cause formation and accumulation of said polypeptide in the culture, and (b) recovering said polypeptide from said culture.

10. The method according to Claim 9, wherein said microorganism belongs to the species Escherichia coli.

11. The method according to Claim 9, wherein said recombinant plasmid is selected from the plasmids as defined in Claims 5 to 7.

12. A polypeptide derived from the hG-CSF polypeptide by deletion, wherein a peptide comprising the 1st to 4th, 1st to 5th, 1st to 6th, 1st to 7th or 1st to 11th amino acids on the N terminus portion of the hG-CSF polypeptide is missing, and optionally in addition to amino acid deletion, the 17th amino acid cystein (Cys⁺¹⁷) of the hG-CSF polypeptide has been replaced by serine (Ser), with the proviso that the 1st to 4th amino acis of hG-CSF are only then deleted if the Cys⁺¹⁷ of hG-CSF is replaced by Ser.

13. A DNA coding for a polypeptide of Claim 12.

14. A recombinant plasmid which comprises a plasmid DNA and, as an insert therein, a DNA fragment coding for a polypeptide as defined in Claim 12.

15. The recominant plasmid according to Claim 14 which comprises a plasmid DNA and, as an insert therein, a DNA fragment coding for a polypeptide as derived from the hG-CSF polypeptide by deletion of a peptide comprising the 1st to 11th amino acids on the N terminus portion thereof.

16. The recombinant plasmid according to Claim 14 or 15, wherein a tryptophan promoter-containing plasmid DNA is used as said plasmid DNA, with said DNA fragment inserted in the plasmid DNA at a site downstream from the tryptophan promoter.

17. The recombinant plasmid of claim 16 which is selected from the group consisting of pCfTNS7, pCfTNS301, pCfTNS401 and pCfTNS501 comprising an insert which codes for a hG-CSF polypeptide being modified as follows:
| Plasmid | type of sequence modification | |
|---|---|---|
| | Deletion¹⁾ | Substitution 17th (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| ¹⁾position of deleted amino acids of hG-CSF | | |
| ²⁾substituted amino acid hG-CSF | | |

18. A method of producing a polypeptide of Claim 12, which method comprises cultivating in a medium a microorganism harboring a recombinant plasmid comprising a plasmid DNA and, as an insert therein, a DNA fragment coding for said polypeptide to thereby cause formation and accumulation of said polypeptide in the culture, and recovering said polypeptide from said culture.

19. The method according to Claim 18 for the production of a polypeptide having an amino acid sequence as derived from the amino acid sequence of the hG-CSF polypeptide by substitution of the 17th amino acid (cysteine) by serine and deletion of 4-7 amino acids on the N terminus side thereof, which method comprises subjecting a polypeptide as derived from the hG-CSF polypeptide by substitution of the 17th amino acid (cysteine) by serine and of at least one amino acid out of the 1st to 6th amino acids on the N terminus side thereof by an amino acid different therefrom to the action of a protease in an aqueous medium to thereby cause formation of said novel polypeptide in the reaction mixture, and recovering said novel polypeptide from said reaction mixture.

20. The method of Claim 19, wherein said protease is selected from the group consisting of subtilisin A, subtilisin BPN', subtilisin Carlsberg, subtilisin novo, proteinase K, nagase, thermolysin, endoproteinase Arg-C, trypsin and α-chymotrypsin.

21. A pharmaceutical composition comprising an effective amount of at least one polypeptide as defined in claims 1 and 12, optionally in combination with a pharmaceutically acceptable carrier or adjuvant.

22. The use of a polypeptide according to claim 1 or 12 for preparing a pharmaceutical composition for the treatment of infectious diseases, for the treatment of leukemia and for increasing the number of neutrophiles.

## Claims (Claims for the following Contracting State(s): ES)

1. A method of producing a polypeptide having an amino acid sequence as derived from the amino acid sequence of the human granulocyte colony stimulating factor (hG-CSF) polypeptide by amino acid substitution, by which method a polypeptide is obtained, wherein
at least one amino acid out of the 1st to 6th amino acids on the N terminus side and optionally the 17th amino acid thereof differs from the amino acid in the corresponding position of the hG-CSF polypeptide,
with the proviso that more than one amino acid of hG-CSF is substituted if the amino acid Thr⁺¹ of hG-CSF is replaced by Ala, Gly or Pro; and with the further proviso that the amino acids in position +1 and +2 are not simultaneously substituted;
which method comprises (a) cultivating in a medium a microorganism harboring a recombinant plasmid coding for said polypeptide to thereby cause formation and accumulation of said polypeptide in the culture, and (b) recovering said polypeptide from said culture.

2. The method according to Claim 1, wherein said microorganism belongs to the species Escherichia coli.

3. The method according to any one of the preceding claims applying a recombinant plasmid comprising a plasmid DNA and, as an insert therein, a DNA fragment coding for a polypeptide as defined in claim 1.

4. The method according to Claim 3, wherein the plasmid DNA is a tryptophan promoter-containing plasmid DNA, with said DNA fragment inserted in said plasmid DNA at a site downstream from the tryptophan promoter.

5. The method according to Claim 3 or 4, wherein said recombinant plasmid is selected from the group consisting of pCfTL38, pCfTL41, pCfTL35 und pCfTAArg4 comprising an insert which codes for a hG-CSF polypeptide being modified as follows:
| Plasmid | Position of amino acid substitution (substituted amino acid of hG-CSF) | |
|---|---|---|
| | 1st (Thr) | 4th (Gly) |
| pCfTL38 | Ser | *) |
| pCfTL41 | Arg | |
| pCfTL35 | Cys | |
| pCfTAArg4 | | Arg |
| | | |
|---|---|---|
| *) No substitution | | |
or is selected from the group consisting of pCfTM14, pCfTM17, pCfTM113 und pCfTAArg4S comprising an insert which codes for a hG-CSF polypeptide being modified as follows:
| Plasmid | Position of amino acid substitution (substituted amino acid of hG-CSF) | | | |
|---|---|---|---|---|
| | 1st (Thr) | 3rd(Leu) | 4th (Gly) | 17th (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) No substitution | | | | |
or is selected from the group consisting of pCfBC42B1, pCfBC45, pCfBC52 pCfBC59, pCfBC76, pCfBC77, pCfBC93, pCfBC95, pCfBC97, pCfBD28, pCfBD56, pCfBD82, pCfBB101, pCfBD28A17 and pCfBD28T17 comprising an insert which codes for a hG-CSF polypeptide being modified as follows:
| Plasmid | Position of amino acid substitution (amino acid of hG-CSF) | | | | | |
|---|---|---|---|---|---|---|
| | 1st (Thr) | 3rd (Leu) | 4th (Gly) | 5th (Pro) | 17th (Cys) | 6th (Ala) |
| pCfBC42B1 | -* | Glu | Lys | Ser | Ser | -* |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -* | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| -* No substitution | | | | | | |

6. A method of producing a polypeptide derived from the hG-CSF polypeptide by deletion, wherein a peptide comprising the 1st to 4th, 1st to 5th, 1st to 6th, 1st to 7th or 1st to 11th amino acids on the N terminus portion of the hG-CSF polypeptide is missing, and optionally in addition to amino acid deletion, the 17th amino acid cystein (Cys⁺¹⁷) of the hG-CSF polypeptide has been replaced by serine (Ser), with the proviso that the 1st to 4th amino acis of hG-CSF are only then deleted if the Cys⁺¹⁷ of hG-CSF is replaced by Ser, which method comprises cultivating in a medium a microorganism harboring a recombinant plasmid comprising a plasmid DNA and, as an insert therein, a DNA fragment coding for said polypeptide to thereby cause formation and accumulation of said polypeptide in the culture, and recovering said polypeptide from said culture.

7. The method according to Claim 6 for the production of a polypeptide having an amino acid sequence as derived from the amino acid sequence of the hG-CSF polypeptide by substitution of the 17th amino acid (cysteine) by serine and deletion of 4-7 amino acids on the N terminus side thereof, which method comprises subjecting a polypeptide as derived from the hG-CSF polypeptide by substitution of the 17th amino acid (cysteine) by serine and of at least one amino acid out of the 1st to 6th amino acids on the N terminus side thereof by an amino acid different therefrom to the action of a protease in an aqueous medium to thereby cause formation of said novel polypeptide in the reaction mixture, and recovering said novel polypeptide from said reaction mixture.

8. The method of Claim 7, wherein said protease is selected from the group consisting of subtilisin A, subtilisin BPN', subtilisin Carlsberg, subtilisin novo, proteinase K, nagase, thermolysin, endoproteinase Arg-C, trypsin and α-chymotrypsin.

9. The method according to one of the Claims 6, 7, or 8, applying a recombinant plasmid which comprises a plasmid DNA and, as an insert therein, a DNA fragment coding for a polypeptide as defined in Claim 6.

10. The method of Claim 9, applying a recombinant plasmid which comprises a plasmid DNA and, as an insert therein, a DNA fragment coding for a polypeptide as derived from the hG-CSF polypeptide by deletion of a peptide comprising the 1st to 11th amino acids on the N terminus portion thereof.

11. The method of claim 9 or 10, applying a recombinant plasmid wherein a tryptophan promoter-containing plasmid DNA is used as said plasmid DNA, with said DNA fragment inserted in the plasmid DNA at a site downstream from the tryptophan promoter.

12. The method of Claim 11, applying a recombinant plasmid which is selected from the group consisting of pCfTNS7, pCfTNS301, pCfTNS401 and pCfTNS501 comprising an insert which codes for a hG-CSF polypeptide being modified as follows:
| Plasmid | type of sequence modification | |
|---|---|---|
| | Deletion¹⁾ | Substitution 17th (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| ¹⁾position of deleted amino acids of hG-CSF | | |
| ²⁾substituted amino acid hG-CSF | | |

13. A method of producing a DNA coding for a polypeptide as defined in Claim 1, which method comprises excising from a recombinant plasmid as defined in one of the claims 3 to 5 a DNA fragment encoding the polypeptide.

14. A method of producing a DNA coding for a polypeptide as defined in Claim 6, which method comprises excising from a recombinant plasmid as defined in one of the claims 9 to 12 a DNA fragment encoding the polypeptide.

15. A method of preparing a microorganism harboring a recombinant plasmid as defined in Claim 5, which method comprises transforming a suitable host with a recombinant plasmid as defined in Claim 5.

16. A method of preparing a recombinant plasmid as defined in one of the Claims 3 to 5, which method comprises inserting into a suitable plasmid a DNA fragment encoding a polypeptide as defined in Claim 1.

17. A method of preparing a recombinant plasmid as defined in one of the Claims 9 to 12, which method comprises inserting into a suitable plasmid a DNA fragment encoding a polypeptide as defined in Claims 6 or 7.

18. A process for preparing a pharmaceutical composition by providing an effective amount of at least one polypeptide as obtainable according to one of the claims 1 to 12, optionally in combination with a pharmaceutically acceptable carrier or adjuvant.

## Patentansprüche (Patentansprüche für folgende(n) Vertragsstaat(en): BE, CH, LI, DE, FR, GB, IT, NL, SE)

1. Polypeptid mit einer Aminosäuresequenz, die von der Aminosäuresequenz des humanen Granulozyten-Colony-Stimulating-Factor (hG-CSF) Polypeptids durch Aminosäuresubstitution abgeleitet ist, dadurch gekennzeichnet, daß wenigstens eine Aminosäure aus der 1. bis 6. Aminosäure des N-terminalen Endes und gegebenenfalls die 17. Aminosäure davon von der Aminosäure in der entsprechenden Position des hG-CSF-Polypeptids differiert, mit der Maßgabe, daß mehr als eine Aminosäure von hG-CSF substituiert ist, falls die Aminosäure Thr⁺¹ von hG-CSF ersetzt ist durch Ala, Gly oder Pro; und mit der weiteren Maßgabe, daß die Aminosäuren in Position +1 und +2 nicht gleichzeitig substituiert sind.

2. DNA, codierend für ein Polypeptid gemäß Anspruch 1.

3. Rekombinantes Plasmid, umfassend eine Plasmid-DNA und als Insert darin ein DNA-Fragment, das für ein Polypeptid gemäß Anspruch 1 codiert.

4. Rekombinantes Plasmid nach Anspruch 3, worin die Plasmid-DNA eine Tryptophan-Promotor enthaltende Plasmid-DNA ist, die das DNA-Fragment als Insert in der Plasmid-DNA stromabwärts vom Tryptophan-Promotor enthält.

5. Rekombinantes Plasmid nach Anspruch 3 oder 4, ausgewählt unter pCfTL38, pCfTL41, pCfTL35 und pCfTAArg4, umfassend ein Insert, welches für ein hG-CSF-Polypeptid codiert, das wie folgt modifiziert ist:
| Plasmid | Position der Aminosäure-Substitution (substituierte Aminosäure von hG-CSF) | |
|---|---|---|
| | 1. (Thr) | 4. (Gly) |
| pCfTL38 | Ser | *) |
| pCfTL41 | Arg | |
| pCfTL35 | Cys | |
| pCfTAArg4 | | Arg |
| | | |
|---|---|---|
| *) keine Substitution | | |

6. Rekombinantes Plasmid nach Anspruch 3 oder 4, ausgewählt unter pCfTM14, pCfTM17, pCfTM113 und pCfTAArg4S, umfassend ein Insert, welches für ein hG-CSF-Polypeptid codiert, das wie folgt modifiziert ist:
| Plasmid | Position der Aminosäure-Substitution (substituierte Aminosäure von hG-CSF) | | | |
|---|---|---|---|---|
| | 1. (Thr) | 3. (Leu) | 4. (Gly) | 17. (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) keine Substitution | | | | |

7. Rekombinantes Plasmid nach Anspruch 3 oder 4, ausgewählt unter pCfBC42Bl, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 und pCfBD28T17, umfassend ein Insert, welches für ein hG-CSF-Polypeptid codiert, das wie folgt modifiziert ist:
| Plasmid | Position der Aminosäure-Substitution (Aminosäure von hG-CSF) | | | | | |
|---|---|---|---|---|---|---|
| | 1. (Thr) | 3. (Leu) | 4. (Gly) | 5. (Pro) | 17. Cys) | 6. (Ala) |
| pCfBC42B1 | -*) | Glu | Lys | Ser | Ser | -*) |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -*) | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| *) keine Substitution | | | | | | |

8. Mikroorganismus, enthaltend ein rekombinantes Plasmid gemäß einem der Ansprüche 5 bis 7.

9. Verfahren zur Herstellung eines Polypeptids gemäß Anspruch 1, wobei man (a) in einem Medium einen Mikroorganismus kultiviert, der ein für das Polypeptid codierendes rekombinantes Plasmid enthält, wobei man die Bildung und Akkumulierung des Polypeptids in der Kultur bewirkt, und (b) man das Polypeptid aus der Kultur isoliert.

10. Verfahren nach Anspruch 9, worin der Mikroorganismus der Species Escherichia coli zuzuordnen ist.

11. Verfahren nach Anspruch 9, worin das rekombinante Plasmid ausgewählt ist unter den Plasmiden gemäß den Ansprüchen 5 bis 7.

12. Polypeptid, abgeleitet vom hG-CSF-Polypeptid durch Deletion, worin ein Peptid, umfassend die 1. bis 4., 1 bis 5., 1 bis 6., 1. bis 7. oder 1. bis 11. Aminosäure des N-terminalen Endes des hG-CSF-Polypeptids fehlt, und worin gegebenenfalls zusätzlich zur Aminosäure-Deletion die 17. Aminosäure, Cystein (Cys⁺¹⁷), des hG-CSF-Polypeptids durch Serin (Ser) ersetzt ist, mit der Maßgabe, daß die 1. bis 4. Aminosäure von hG-CSF nur dann deletiert ist, wenn Cys⁺¹⁷ von hG-SCF durch Ser ersetzt ist.

13. DNA, codierend für ein Polypeptid gemäß Anspruch 12.

14. Rekombinantes Plasmid, umfassend eine Plasmid-DNA und darin insertiert ein DNA-Fragment, codierend für ein Polypeptid gemäß Anspruch 12.

15. Rekombinantes Plasmid gemäß Anspruch 14, umfassend eine Plasmid-DNA und darin insertiert ein DNA-Fragment, welches für ein Polypeptid codiert, das vom hG-CSF-Polypeptid abgeleitet ist durch Deletion eines Peptids, umfassend die 1. bis 11. Aminosäure am N-terminalen Ende davon.

16. Rekombinantes Plasmid gemäß Anspruch 14 oder 15, worin eine Tryptophan-Promotor-enthaltende Plasmid-DNA als Plasmid-DNA verwendet wird, wobei das DNA-Fragment in der Plasmid-DNA stromabwärts vom Tryptophan-Promotor insertiert ist.

17. Rekombinantes Plasmid nach Anspruch 16, ausgewählt unter pCfTNS7, pCfTNS301, pCfTNS401 und pCfTNS501, umfassend ein Insert, welches für ein hG-CSF-Polypeptid codiert, das wie folgt modifiziert ist:
| Plasmid | Art der Sequenzmodifikation | |
|---|---|---|
| | Deletion¹⁾ | Substitution 17. (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| ¹⁾ Position der deletierten Aminosäuren von hG-CSF | | |
| ²⁾ substituierte Aminosäure von hG-CSF | | |

18. Verfahren zur Herstellung eines Polypeptids gemäß Anspruch 12, wobei man in einem Medium einen Mikroorgnismus kultiviert, der ein rekombinantes Plasmid enthält, das eine Plasmid-DNA umfaßt und darin insertiert ein DNA-Fragment, das für das Polypeptid codiert, wobei die Bildung und Akkumulierung des Polypeptids in der Kultur bewirkt wird und man das Polypeptid aus der Kultur isoliert.

19. Verfahren nach Anspruch 18 zur Herstellung eines Polypeptids mit einer Aminosäuresequenz, abgeleitet von der Aminosäuresequenz des hG-CSF-Polypeptids durch Substitution der 17. Aminosäure (Cystein) durch Serin und Deletion von 4-7 Aminosäuren am N-terminalen Ende davon, wobei man ein Polypeptid, abgeleitet vom hG-CSF-Polypeptid durch Substitution der 17. Aminosäure (Cystein) durch Serin und von wenigstens einer Aminosäure der 1. bis 6. Aminosäure des N-terminalen Endes davon durch eine davon verschiedene Aminosäure, der Wirkung einer Protease in einem wäßrigen Medium aussetzt, wodurch man die Bildung des neuen Polypeptids im Reaktionsgemisch bewirkt, und man das neue Polypeptid aus dem Reaktionsgemisch abtrennt.

20. Verfahren nach Anspruch 19, worin die Protease ausgewählt ist unter Subtilisin A, Subtilisin BPN', Subtilisin Carlsberg, Subtilisin Novo, Proteinase K, Nagase, Thermolysin, Endoproteinase Arg-C, Trypsin und α-Chymotrypsin.

21. Pharmazeutisches Mittel, umfassend eine wirksame Menge wenigstens eines Polypeptids gemäß Anspruch 1 oder 12, gegebenenfalls in Kombination mit einem pharmazeutisch verträglichen Träger oder Adjuvans.

22. Verwendung eines Polypeptids gemäß Anspruch 1 oder 12 zur Herstellung eines pharmazeutischen Mittels zur Behandlung infektiöser Erkrankungen, zur Behandlung von Leukämie und zur Erhöhung der Neutrophilen-Zahl.

## Patentansprüche (Patentansprüche für folgende(n) Vertragsstaat(en): ES)

1. Verfahren zur Herstellung eines Polypeptids mit einer Aminosäuresequenz, die von der Aminosäuresequenz des humanen Granulozyten-Colony-Stimulating- Factor (hG-CSF) Polypeptids durch Aminosäuresubstitution abgeleitet ist, wobei wenigstens eine Aminosäure aus der 1. bis 6. Aminosäure des N-terminalen Endes und gegebenenfalls die 17. Aminosäure davon von der Aminosäure in der entsprechenden Position des hG-CSF-Polypeptids differiert, mit der Maßgabe, daß mehr als eine Aminosäure von hG-CSF substituiert ist, falls die Aminosäure Thr⁺¹ von hG-CSF ersetzt ist durch Ala, Gly oder Pro; und mit der weiteren Maßgabe, daß die Aminosäuren in Position +1 und +2 nicht gleichzeitig substituiert sind,
wobei man
(a) in einem Medium einen Mikroorganismus kultiviert, der ein rekombinantes Plasmid enthält, das für das Polypeptid codiert, wodurch die Bildung und Akkumulierung des Polypeptids in der Kultur bewirkt wird, und
(b) man das Polypeptid aus der Kultur isoliert.

2. Verfahren nach Anspruch 1, worin der Mikroorganismus der Species Escherichia coli zuzuordnen ist.

3. Verfahren nach einem der vorhergehenden Ansprüche, wobei man ein rekombinantes Plasmid verwendet, das eine Plasmid-DNA umfaßt, und insertiert darin ein DNA-Fragment, welches für ein Polypeptid gemäß Anspruch 1 codiert.

4. Verfahren nach Anspruch 3, worin die Plasmid- DNA eine Tryptophan-Promotor enthaltende Plasmid-DNA ist, die das DNA-Fragment als Insert in der Plasmid-DNA stromabwärts vom Tryptophan-Promotor enthält.

5. Verfahren nach Anspruch 3 oder 4, worin das kombinante Plasmid ausgewählt ist unter pCfTL38, pCfTL41, pCfTL35 und pCfTAArg4, umfassend ein Insert, welches für ein hG-CSF-Polypeptid codiert, das wie folgt modifiziert ist:
| Plasmid | Position der Aminosäure-Substitution (substituierte Aminosäure von hG-CSF) | |
|---|---|---|
| | 1. (Thr) | 4. (Gly) |
| pCfTL38 | Ser | *) |
| pCfTL41 | Arg | |
| pCfTL35 | Cys | |
| pCfTAArg4 | | Arg |
| | | |
|---|---|---|
| *) keine Substitution | | |
oder ausgewählt ist unter pCfTM14, pCfTM17, pCfTM113 und pCfTAArg4S, umfassend ein Insert, welches für ein hG-CSF-Polypeptid codiert, das wie folgt modifiziert ist:
| Plasmid | Position der Aminosäure-Substitution (substituierte Aminosäure von hG-CSF) | | | |
|---|---|---|---|---|
| | 1. (Thr) | 3. (Leu) | 4. (Gly) | 17. (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) keine Substitution | | | | |
oder ausgewählt ist unter pCfBC42B1, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 und pCfBD28T17, umfassend ein Insert, welches für ein hG-CSF-Polypeptid codiert, das wie folgt modifiziert ist:
| Plasmid | Position der Aminosäure-Substitution (Aminosäure von hG-CSF) | | | | | |
|---|---|---|---|---|---|---|
| | 1. (Thr) | 3. (Leu) | 4. (Gly) | 5. (Pro) | 17. Cys) | 6. (Ala) |
| pCfBC42B1 | -*) | Glu | Lys | Ser | Ser | -*) |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -*) | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| *) keine Substitution | | | | | | |

6. Verfahren zur Herstellung eines Polypeptids, abgeleitet vom hG-CSF-Polypeptid durch Deletion, worin ein Peptid, umfassend die 1. bis 4., 1 bis 5., 1 bis 6., 1. bis 7. oder 1. bis 11. Aminosäure des N-terminalen Endes des hG-CSF-Polypeptids fehlt, und worin gegebenenfalls zusätzlich zur Aminosäure-Deletion die 17. Aminosäure, Cystein (Cys⁺¹⁷), des hG-CSF-Polypeptids durch Serin (Ser) ersetzt ist, mit der Maßgabe, daß die 1. bis 4. Aminosäure von HG-CSF nur dann deletiert ist, wenn Cys⁺¹⁷ von hG-SCF durch Ser ersetzt ist, wobei man in einem Medium einen Mikroorganismus kultiviert, der ein rekombinantes Plasmid enthält, das eine Plasmid-DNA umfaßt, und darin insertiert ein DNA-Fragment, welches für das Polypeptid codiert, wodurch die Bildung und Akkumulierung des Polypeptids in der Kultur bewirkt wird und man das Polypeptid aus der Kultur isoliert.

7. Verfahren nach Anspruch 6 zur Herstellung eines Polypeptids mit einer Aminosäuresequenz, abgeleitet von der Aminosäuresequenz des hG-CSF-Polypeptids durch Substitution der 17. Aminosäure (Cystein) durch Serin und Deletion von 4-7 Aminosäuren am N-terminalen Ende davon, wobei man ein Polypeptid, abgeleitet vom hG-CSF-Polypeptid durch Substitution der 17. Aminosäure (Cystein) durch Serin und von wenigstens einer Aminosäure der 1. bis 6. Aminosäure des N-terminalen Endes davon durch eine davon verschiedene Aminosäure, der Wirkung einer Protease in einem wäßrigen Medium aussetzt, wodurch man die Bildung des neuen Polypeptids im Reaktionsgemisch bewirkt, und man das neue Polypeptid aus dem Reaktionsgemisch abtrennt.

8. Verfahren nach Anspruch 7, worin die Protease ausgewählt ist unter Subtilisin A, Subtilisin BPN', Subtilisin Carlsberg, Subtilisin Novo, Proteinase K, Nagase, Thermolysin, Endoproteinase Arg-C, Trypsin und α-Chymotrypsin.

9. Verfahren nach Anspruch 6, 7 oder 8, wobei man ein rekombinantes Plasmid verwendet, welches eine Plasmid-DNA umfaßt und darin insertiert ein DNA-Fragment, das für ein Polypeptid gemäß Anspruch 6 codiert.

10. Verfahren nach Anspruch 9, wobei man ein rekombinantes Plasmid verwendet, umfassend eine Plasmid-DNA und darin insertiert ein DNA-Fragment, welches für ein Polypeptid codiert, das vom hG-CSF-Polypeptid abgeleitet ist durch Deletion eines Peptids, umfassend die 1. bis 11. Aminosäure am N-terminalen Ende davon.

11. Verfahren nach Anspruch 9 oder 10, wobei man ein rekombinantes Plasmid verwendet, worin eine Tryptophan-Promotor-enthaltende Plasmid-DNA als Plasmid-DNA verwendet wird, wobei das DNA-Fragment in der Plasmid-DNA stromabwärts vom Tryptophan-Promotor insertiert ist.

12. Verfahren nach Anspruch 11, wobei man ein rekombinantes Plasmid verwendet, welches ausgewählt ist unter pCfTNS7, pCfTNS301, pCfTNS401 und pCfTNS501, umfassend ein Insert, welches für ein hG-CSF-Polypeptid codiert, das wie folgt modifiziert ist:
| Plasmid | Art der Sequenzmodifikation | |
|---|---|---|
| | Deletion¹⁾ | Substitution 17. (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| ¹⁾ Position der deletierten Aminosäuren von hG-CSF | | |
| ²⁾ substituierte Aminosäure von hG-CSF | | |

13. Verfahren zur Herstellung einer DNA, codierend für ein Polypeptid gemäß Anspruch 1, wobei man aus einem gemäß einem der Ansprüche 3 bis 5 definierten rekombinanten Plasmid ein für das Polypeptid codierendes DNA-Fragment ausschneidet.

14. Verfahren zur Herstellung einer DNA, codierend für ein Polypeptid gemäß Anspruch 6, wobei man aus einem gemäß einem der Ansprüche 9 bis 12 definierten rekombinanten Plasmid ein für das Polypeptid codierendes DNA-Fragment ausschneidet.

15. Verfahren zur Herstellung eines Mikroorganismus, der ein rekombinantes Plasmid gemäß Anspruch 5 enthält, wobei man einen geeigneten Wirt mit einem rekombinanten Plasmid gemäß Anspruch 5 transformiert.

16. Verfahren zur Herstellung eines gemäß den Ansprüchen 3 bis 5 definierten rekombinanten Plasmids, wobei man in ein geeignetes Plasmid ein DNA-Fragment insertiert, das für ein Polypeptid gemäß Anspruch 1 codiert.

17. Verfahren zur Herstellung eines gemäß den Ansprüchen 9 bis 12 definierten rekombinanten Plasmids, wobei man in ein geeignetes Plasmid ein DNA-Fragment insertiert, das für ein Polypeptid gemäß Anspruch 6 oder 7 codiert.

18. Verfahren zur Herstellung eines pharmazeutischen Mittels, wobei man eine wirksame Menge wenigstens eines Polypeptids, erhältlich gemäß einem der Ansprüche 1 bis 12, gegebenenfalls in Kombination mit einem pharmazeutisch verträglichen Träger oder Adjuvans bereitstellt.

## Revendications (Revendications pour l'(les) Etat(s) contractant(s) suivant(s): BE, CH, LI, DE, FR, GB, IT, NL, SE)

1. Un polypeptide ayant une séquence d'acides aminés telle que dérivée de la séquence d'acides aminés du polypeptide (hG-CSF) facteur stimulant les colonies de granulocytes humains par substitution d'acides aminés,
caractérisé en ce que
au moins un acide aminé parmi les acides aminés du ler au 6ème du côté de l'extrémité N teminale et de façon optionnelle le 17ème acide aminé de celle-ci diffère de l'acide aminé dans la position correspondante du polypeptide hG-CSF,
avec la condition que plus d'un acide aminé de hG-CSF soit substitué si l'acide aminé Thr⁺¹ de hG-CSF est remplacé par Ala, Gly ou Pro, et avec l'autre condition que les acides aminés dans la position +1 et +2 ne sont pas substitués simultanement.

2. Un ADN codant pour un polypeptide de la Revendication 1.

3. Un plasmide recombinant comprenant un ADN plasmidique et, comme un insérat dans celui-ci, un fragment d'ADN codant pour un polypeptide tel que défini dans la Revendication 1.

4. Le plasmide recombinant selon la Revendication 3, dans lequel l'ADN plasmidique est un ADN plasmidique contenant le promoteur tryptophane, avec ledit fragment d'ADN inséré dans ledit ADN plasmidique à un site situé en aval du promoteur tryptophane.

5. Le plasmide recombinant de la Revendication 3 ou 4 qui est choisi dans le groupe constitué par pCfTL38, pCfTL41, pCfTL35 et pCfTAArg4 comprenant un insérat qui code pour un polypeptide hG-CSF étant modifié comme suit:
| Plasmide | Position de la substitution de l'acide aminé (acide aminé substitué de hG-CSF) | |
|---|---|---|
| | 1er (Thr) | 4ème (Gly) |
| pCfTL38 | Ser | *) |
| pCfTL41 | Arg | |
| pCfTL35 | Cys | |
| pCfTAArg4 | | Arg |
| | | |
|---|---|---|
| *) Aucune substitution | | |

6. Le plasmide recombinant de la Revendication 3 ou 4 qui est choisi dans le groupe comprenant pCfTM14, pCfTM17, pCfTM113 et pCfTAArg4S contenant un insérat qui code pour un polypeptide hG-CSF étant modifié comme suit:
| Plasmide | Position de la substitution de l'acide aminé (acide aminé substitué de hG-CSF) | | | |
|---|---|---|---|---|
| | 1er (Thr) | 3ème (Leu) | 4ème (Gly) | 17ème (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) Aucune substitution | | | | |

7. Le plasmide recombinant de la Revendication-3 ou 4 qui est choisi dans le groupe comprenant pCfBC42B1, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 et pCfBD28T17 contenant un insérat qui code pour un polypeptide hG-CSF étant modifié comme suit:
| Plasmide | Position de substitution de l'acide aminé (acide aminé de hG-CSF) | | | | | |
|---|---|---|---|---|---|---|
| | 1er (Thr) | 3ème (Leu) | 4ème (Gly) | 5ème (Pro) | 17ème (Cys) | 6ème (Ala) |
| pCfBC42B1 | -* | Glu | Lys | Ser | Ser | -* |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -* | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| -* Aucune substitution | | | | | | |

8. Un microorganisme habritant un plasmide recombinant comme défini dans l'une des Revendications de 5 à 7.

9. Une méthode de production d'un polypeptide de la Revendication 1, laquelle méthode comprend (a) la culture dans un milieu d'un microorganisme abritant un plasmide recombinant codant pour ledit polypeptide, pour, de cette façon, causer la formation et l'accumulation dudit polypeptide dans la culture, et (b) la récupération dudit polypeptide de ladite culture.

10. La méthode selon la Revendication 9, dans laquelle ledit microorganisme appartient à l'espèce Escherichia coli.

11. La méthode selon la Revendication 9, dans laquelle ledit plasmide recombinant est choisi parmi les plasmides comme définis dans les Revendications 5 à 7.

12. Un polypeptide dérivé du polypeptide hG-CSF par délétion, dans lequel un peptide comprenant les 1er au 4ème, les 1er au 5ème, les 1er au 6ème, les 1er au 7ème ou les 1er au 11ème acides aminés sur la partie N terminale du polypeptide hG-CSF, est manquant, et facultativement en plus de la délétion des acides aminés, le 17ème acide aminé cystéine (Cys⁺¹⁷) du polypeptide hG-CSF a été remplacé par une sérine (Ser), avec la condition que, les 1er au 4ème acides aminés de hG-CSF ne sont alors supprimés, seulement si la Cys⁺¹⁷ de hG-CSF est remplacée par Ser.

13. Un ADN codant pour un polypeptide de la Revendication 12.

14. Un plasmide recombinant qui comprend un ADN plasmidique et, en tant qu'insérat inclus, un fragment d'ADN codant pour un polypeptide comme défini dans la Revendication 12.

15. Le plasmide recombinant selon la Revendication 14 qui comprend un ADN plasmidique et, en tant qu'insérat inclus, un fragment d'ADN codant pour un polypeptide comme dérivé du polypeptide hG-CSF par délétion d'un peptide comprenant les 1er au 11ème acides aminés sur la partie N terminale de celui-ci.

16. Le plasmide recombinant selon la Revendication 14 ou 15, dans lequel un ADN plasmidique contenant le promoteur tryptophane est utilisé comme ledit ADN plasmidique, avec ledit fragment d'ADN inséré dans l'ADN plasmidique à un site situé en aval du promoteur tryptophane.

17. Le plasmide recombinant de la Revendication 16 qui est choisi dans le groupe comprenant pCfTNS7, pCfTNS301, pCfTNS401 et pCfTNS501 contenant un insérat qui code pour un polypeptide hG-CSF étant modifié comme suit:
| Plasmide | type de modification de la séquence | |
|---|---|---|
| | Délétion¹⁾ | Substitution 17ème (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| 1) position des acides aminés supprimés dans hG-CSF | | |
| 2) acide aminé substitué dans hG-CSF | | |

18. Une méthode de production d'un polypeptide de la Revendication 12, laquelle méthode comprend la culture dans un milieu, d'un microorganisme abritant un plasmide recombinant comprenant un ADN plasmidique et, comme insérat inclus, un fragment d'ADN codant pour ledit polypeptide pour causer de cette façon la formation et l'accumulation dudit polypeptide dans la culture, et la récupération dudit polypeptide à partir de ladite culture.

19. La méthode selon la Revendication 18 pour la production d'un polypeptide ayant une séquence d'acides aminés comme dérivée de la séquence d'acides aminés du polypeptide hG-CSF par substitution du 17ème acide aminé par de la sérine et la délétion de 4-7 acides aminés sur le côté N terminal de celui-ci, laquelle méthode comprend la soumission d'un polypeptide comme dérivé du polypeptide hG-CSF par substitution du 17ème acide aminé (cystéine) par de la sérine et d'au moins un acide aminé parmi les 1er au 6ème acides aminés sur le côté N terminal de celui-ci par un acide aminé différent de ceux-ci, à l'action d'une protéase dans un milieu aqueux pour, de cette façon, causer la formation dudit polypeptide nouveau dans le milieu de la réaction, et la récupération dudit polypeptide nouveau à partir dudit mélange de la réaction.

20. La méthode de la Revendication 19, dans laquelle ladite protéase est choisie dans le groupe comprenant la subtilisine A, la subtilisine BPN', la subtilisine Carlsberg, la subtilisine Novo, la protéinase K, la nagase, la thermolysine, l'endoprotéinase Arg-C, la trypsine et l'α-chymotrypsine.

21. Une composition pharmaceutique comprenant une quantité efficace d'au moins un polypeptide comme défini dans les Revendications 1 et 12, facultativement avec un support ou un adjuvant pharmaceutiquement acceptables.

22. L'utilisation d'un polypeptide selon la Revendication 1 ou 12 pour la préparation d'une composition pharmaceutique pour le traitement des maladies infectieuses, pour le traitement de la leucémie et pour augmenter le nombre des neutrophiles.

## Revendications (Revendications pour l'(les) Etat(s) contractant(s) suivant(s): ES)

1. Une méthode de production d'un polypeptide ayant une séquence d'acides aminés comme dérivée de la séquence des acides aminés du polypeptide (hG-CSF) facteur stimulant les colonies de granulocytes humains par substitution d'acides aminés, méthode par laquelle on obtient un polypeptide, dans lequel
au moins un acide aminé parmi les 1er au 6ème acides aminés sur le côté N terminal et facultativement le 17ème acide aminé de celui-ci diffère de l'acide aminé dans la position correspondante du polypeptide hG-CSF,
avec la condition que plus d'un acide aminé de hG-CSF soit substitué si l'acide aminé Thr⁺¹ de hG-CSF est remplacé par Ala, Gly ou Pro, et avec l'autre condition que les acides aminés dans la position +1 et +2 ne sont pas substitués simultanement,
laquelle méthode comprend (a) la culture dans un milieu d'un microorganisme abritant un plasmide recombinant codant pour ledit polypeptide pour de cette façon causer la formation et l'accumulation dudit polypeptide dans la culture, et (b) la récupération dudit polypeptide à partir de ladite culture.

2. La méthode selon la Revendication 1, dans laquelle ledit microorganisme appartient à l'espèce Escherichia coli.

3. La méthode selon l'une quelconque des Revendications précédentes s'appliquant à un plasmide recombinant comprenant un ADN plasmidique et, comme insérat inclus, un fragment d'ADN codant pour un polypeptide comme défini dans la Revendication 1.

4. La méthode selon la Revendication 3, dans laquelle l'ADN plasmidique est un ADN plasmidique contenant le promoteur tryptophane, avec ledit fragment d'ADN inséré dans ledit ADN plasmidique à un site situé en aval du promoteur tryptophane.

5. La méthode selon la Revendication 3 ou 4, dans laquelle ledit plasmide recombinant est choisi dans le groupe comprenant pCfTL38, pCfTL41, pCfTL35 et pCfTAArg4 contenant un insérat qui code pour un polypeptide hG-CSF étant modifié comme suit:
| Plasmide | Position de la substitution de l'acide aminé (acide aminé substitué de hG-CSF) | |
|---|---|---|
| | 1er (Thr) | 4ème (Gly) |
| pCfTL38 | Ser | *) |
| pCfTL41 | Arg | |
| pCfTL35 | Cys | |
| pCfTAArg4 | | Arg |
| | | |
|---|---|---|
| *) Aucune substitution | | |
ou est choisi dans le groupe comprenant pCfTM14, pCfTM17, pCfTM113 et pCfTAArg4S contenant un insérat qui code pour un polypeptide hG-CSF étant modifié comme suit:
| Plasmide | Position de la substitution de l'acide aminé (acide aminé substitué de hG-CSF) | | | |
|---|---|---|---|---|
| | 1er (Thr) | 3ème (Leu) | 4ème (Gly) | 17ème (Cys) |
| pCfTM14 | Ser | Cys | | *) |
| pCfTM17 | Ser | Arg | | |
| pCfTM113 | Ser | Ser | | |
| pCfTAArg4S | | | Arg | Ser |
| | | | | |
|---|---|---|---|---|
| *) Aucune substitution | | | | |
ou est choisi dans le groupe comprenant pCfBC42B1, pCfBC45, pCfBC52, pCfBC59, pCfBC76, pCfBC77, pCfBC93, pCfBC95, pCfBC97, pCfBD28, pCfBD56, pCfBD82, pCfBB101, pCfBD28A17 et pCfBD28T17 contenant un insérat qui code pour un polypeptide hG-CSF étant modifié comme suit:
| Plasmide | Position de substitution de l'acide aminé (acide aminé de hG-CSF) | | | | | |
|---|---|---|---|---|---|---|
| | 1er (Thr) | 3ème (Leu) | 4ème (Gly) | 5ème (Pro) | 17ème (Cys) | 6ème (Ala) |
| pCfBC42B1 | -* | Glu | Lys | Ser | Ser | -* |
| pCfBC45 | Val | Ile | Arg | Ser | Ser | - |
| pCfBC52 | Cys | Ile | Arg | Ser | Ser | - |
| pCfBC59 | Tyr | Ile | Arg | Ser | Ser | - |
| pCfBC76 | Arg | Thr | Arg | Ser | Ser | - |
| pCfBC77 | -* | Thr | Arg | Ser | Ser | - |
| pCfBC93 | Asn | Glu | Arg | Ser | Ser | - |
| pCfBC95 | Ile | Thr | Arg | Ser | Ser | - |
| pCfBC97 | Ser | Thr | Arg | Ser | Ser | - |
| pCfBD28 | Ala | Thr | Tyr | Arg | Ser | - |
| pCfBD56 | Ala | Ser | Asn | Ser | Ser | - |
| pCfBD82 | Ala | Pro | Asn | Arg | Ser | Gly |
| pCfBB101 | Ala | Thr | Arg | Ser | Ser | - |
| pCfBD28A17 | Ala | Thr | Tyr | Arg | Ala | - |
| pCfBD28T17 | Ala | Thr | Tyr | Arg | Thr | - |
| | | | | | | |
|---|---|---|---|---|---|---|
| -* Aucune substitution | | | | | | |

6. Une méthode de production d'un polypeptide dérivé du polypeptide hG-CSF par délétion, dans lequel un peptide comprenant les 1er au 4ème, 1er au 5ème, 1er au 6ème, 1er au 7ème ou 1er au 11ème acides aminés sur la partie N terminale du polypeptide hG-CSF est manquant, et facultativement en plus de la délétion des acides aminés, le 17ème acide aminé cystéine (Cys⁺¹⁷) du polypeptide hG-CSF a été remplacé par de la sérine (Ser), avec la condition que les 1er au 4ème acides aminés de hG-CSF ne soient alors supprimés seulement si la Cys⁺¹⁷ de hG-CSF est remplacée par Ser, laquelle méthode comprend la culture dans un milieu, d'un microorganisme abritant un plasmide recombinant contenant un ADN plasmidique et, comme insérat inclus, un fragment d'ADN codant pour ledit polypeptide, pour, de cette façon, causer la formation et l'accumulation dudit polypeptide dans la culture, et la récupération dudit polypeptide à partir de ladite culture.

7. La méthode selon la Revendication 6 pour la production d'un polypeptide ayant une séquence d'acides aminés telle que dérivée de la séquence des acides aminés du polypeptide hG-CSF par substitution du 17ème acide aminé (cystéine) par de la sérine et délétion de 4-7 acides aminés sur le côté N terminal de celui-ci, laquelle méthode comprend la soumission d'un polypeptide tel que dérivé du polypeptide hG-CSF par substitution du 17ème acide aminé (cystéine) par de la sérine et d'au moins un acide aminé parmi les 1er au 6ème acides aminés sur le côté N terminal de celui-ci par un acide aminé différent de ceux-ci, à l'action d'une protéase dans un milieu aqueux, pour, de cette façon, causer la formation dudit nouveau polypeptide dans le mélange de la réaction, et la récupération dudit nouveau polypeptide à partir dudit mélange de la réaction.

8. La méthode de la Revendication 7, dans laquelle ladite protéase est choisie dans le groupe comprenant la subtilisine A, la subtilisine BPN', la subtilisine Carlsberg, la subtilisine Novo, la protéinase K, la nagase, la thermolysine, l'endoprotéinase Arg-C, la trypsine et l'α-chymotrypsine.

9. La méthode selon l'une des Revendications 6, 7, ou 8, mettant en oeuvre un plasmide recombinant qui comprend un ADN plasmidique et, comme insérat inclus, un fragment d'ADN codant pour un polypeptide comme défini dans la Revendication 6.

10. La méthode de la Revendication 9, mettant en oeuvre un plasmide recombinant qui comprend un ADN plasmidique et, comme insérat inclus, un fragment d'ADN codant pour un polypeptide comme dérivé du polypeptide hG-CSF par délétion d'un peptide comprenant les 1er au 11ème acides aminés sur la partie N terminale de celui-ci.

11. La méthode de la Revendication 9 ou 10, mettant en oeuvre un plasmide recombinant dans lequel un ADN plasmidique contenant un promoteur tryptophane est utilisé comme ledit ADN plasmidique, avec ledit fragment d'ADN inséré dans l'ADN plasmidique à un site situé en aval du promoteur tryptophane.

12. La méthode de la Revendication 11, mettant en oeuvre un plasmide recombinant qui est choisi dans le groupe comprenant pCfTNS7, pCfTNS301, pCfTNS401 et pCfTNS501 contenant un insérat qui code pour un polypeptide hG-CSF étant modifié comme suit:
| Plasmide | type de modification de la séquence | |
|---|---|---|
| | Délétion¹⁾ | Substitution 17ème (Cys)²⁾ |
| pCfTNS7 | 1 - 4 | Ser |
| pCfTNS301 | 1 - 11 | Ser |
| pCfTNS401 | 1 - 7 | Ser |
| pCfTNS501 | 1 - 6 | Ser |
| | | |
|---|---|---|
| 1) position des acides aminés supprimés dans hG-CSF | | |
| 2) acide aminé substitué dans hG-CSF | | |

13. Une méthode de production d'un ADN codant pour un polypeptide comme défini dans la Revendication 1, laquelle méthode comprend l'excision à partir d'un plasmide recombinant comme défini dans l'une des Revendications 3 à 5, d'un fragment d'ADN codant pour le polypeptide.

14. Une méthode de production d'un ADN codant pour un polypeptide comme défini dans la Revendication 6, laquelle méthode comprend l'excision à partir d'un plasmide recombinant comme défini dans l'une des Revendications 9 à 12, d'un fragment d'ADN codant pour le polypeptide.

15. Une méthode de préparation d'un microorganisme abritant un plasmide recombinant comme défini dans la Revendication 5, laquelle méthode comprend la transformation d'un hôte convenable avec un plasmide recombinant comme défini dans la Revendication 5.

16. Une méthode de préparation d'un plasmide recombinant comme défini dans l'une des Revendications 3 à 5, laquelle méthode comprend l'insertion dans un plasmide convenable d'un fagment d'ADN codant pour un polypeptide comme défini dans la Revendication 1.

17. Une méthode de préparation d'un plasmide recombinant comme défini dans l'une des Revendications 9 à 12, laquelle méthode comprend l'insertion dans un plasmide convenable d'un fragment d'ADN codant pour un polypeptide comme défini dans les Revendications 6 ou 7.

18. Un procédé pour la préparation d'une composition pharmaceutique, en apportant une quantité efficace d'au moins un polypeptide comme ils peuvent être obtenus selon l'une des Revendications 1 à 12, facultativement en combinaison avec un support ou un adjuvant pharmaceutiquement acceptables.
